# EUROPEAN PATENT SPECIFICATION

(11) **EP 1 803 716 B1**
(45) Date of publication and mention of the grant of the patent: **25.07.2012**
(21) Application number: 06010861.0
(22) Date of filing: 26.05.2006
(51) Int. Cl.: C07D 311/58, C07D 407/06, C07D 413/14

(54) **A process for preparation of racemic nebivolol**
Ein Verfahren zur Herstellung von racemischem Nebivolol
Un procédé pour la préparation de nebivolol racémique

(30) Priority: 28.12.2005 US 319287; 04.01.2006 EP 06000149; 07.04.2006 US 790150 P
(43) Date of publication of application: 04.07.2007
(73) Proprietor: Acino Pharma AG, 4253 Liesberg (BL) (CH); University of Zürich, 8006 Zürich (CH)
(72) Inventor: Stutz, Alfred, 8037 Zürich (CH)
(74) Representative: Katzameyer, Michael

(56) References cited:
- EP-A- 0 145 067
- EP-A- 0 334 429
- WO-A-2004/041805
- CHANDRASEKHAR S ET AL: "Enantioselective Total Synthesis of the Antihypertensive Agent (S,R,R,R)-Nebivolol" TETRAHEDRON, ELSEVIER SCIENCE PUBLISHERS, AMSTERDAM, NL, vol. 56, no. 34, 18 August 2000 (2000-08-18), pages 6339-6344, XP004214993 ISSN: 0040-4020

## Description

### BACKGROUND OF THE INVENTION

### 1. FIELD OF INVENTION

This invention relates to a novel process for preparation of racemic Nebivolol, its enantiomeric compounds and to novel compounds made by the process.

### 2. DESCRIPTION OF RELATED ART

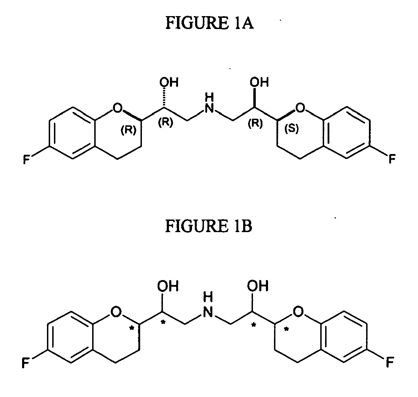

Nebivolol (see Figure 1A, showing d-Nebivolol, chemical name: [2R*[R*[R*(S*)]]]-α,α'-[iminobis(methylene)]bis[6-fluoro-3,4-dihydro-2H-1-benzopyran-2-methanol] or alternatively [2R*[R*[R*(S*)]]]-α,α'-[iminobis(methylene)]bis[6-fluoro-chroman-2-methanol] and Figure 1B showing a racemic Nebivolol, which is a mixture of 1- and d- Nebivolol) is known as an adrenergic beta-antagonist, an antihypertensive agent, a platelet aggregation inhibitor and a vasodilating agent.

Nebivolol is administered as tablets (e.g., a dosage of 5.45 mg Nebivolol hydrochloride is equivalent to 5 mg Nebivolol) which contain Nebivolol as a racemic mixture of enantiomers SRRR-Nebivolol (*dextro d*-Nebivolol) and RSSS-Nebivolol (*levo l*-Nebivolol).

Nebivolol contains four asymmetric centers, and therefore 16 stereoisomers are theoretically possible. However, because of the particular constitution of the structures and configurations of the stereoisomers (e.g., axial symmetry), only 10 stereoisomers (6 diastereomers: 4 *dl* forms and 2 meso forms) can be formed (Table 1).

A non-stereoselective preparation of these stereoisomers is generally described in U.S. Patent No. 4,654,362 to Van Lommen et al. (Janssen Pharmaceutica N. V.) (and its counter-part EP 0145067). A stereoselective synthesis of the isomer [2R, αS, 2'S, α'S]-α, α'-[iminobis(methylene)]bis[6-fluoro-3, 4-dihydro-2H-1-benzopyran-2-methanol] has been described in U.S. Patent No. 6,545,040 (Janssen PharmaceuticaN. V.) (and its counter-part EP 0334429).

A procedure for separating a diastereomeric mixture consisting of (±)-[2R*[1S*, 5S*(S*)]]+ [2R*[1S*[5R*(S*)]]]-α,α'-[iminobis(methylene)]bis[6-fluoro-3,4-dihydro-2H-1-benzopyran-2-methanol] by fractional crystallization of the corresponding hydrochloride salts was described in US 5,759,580 (Janssen Pharmaceutica N. V.) (and its counter-part EP 0744946). Nebivolol Hydrochloride could be obtained only in a very low yield of 6.6%.

A PCT patent application publication WO 2004/041805 (Egis Gyogyszergyar RT.) describes a new process for the preparation ofracemic [2S[2R*[R[R*]]]] and [2R[2S*[S[S*]]]]-(±)α,α'-[iminobis(methylene)]bis[6-fluoro-3,4-dihydro-2H-1-benzopyran-2-methanol] and its pure [2S[2R*[R[R*]]]]-, and [2R[2S*[S[S*]]]] enantiomers.

Alternative and enantioselective syntheses of *d*-Nebivolol were described in J. Am. Chem. Soc. 1998, 120, 8340-8347 and Tetrahedron 56, 2000, 6339-6344.

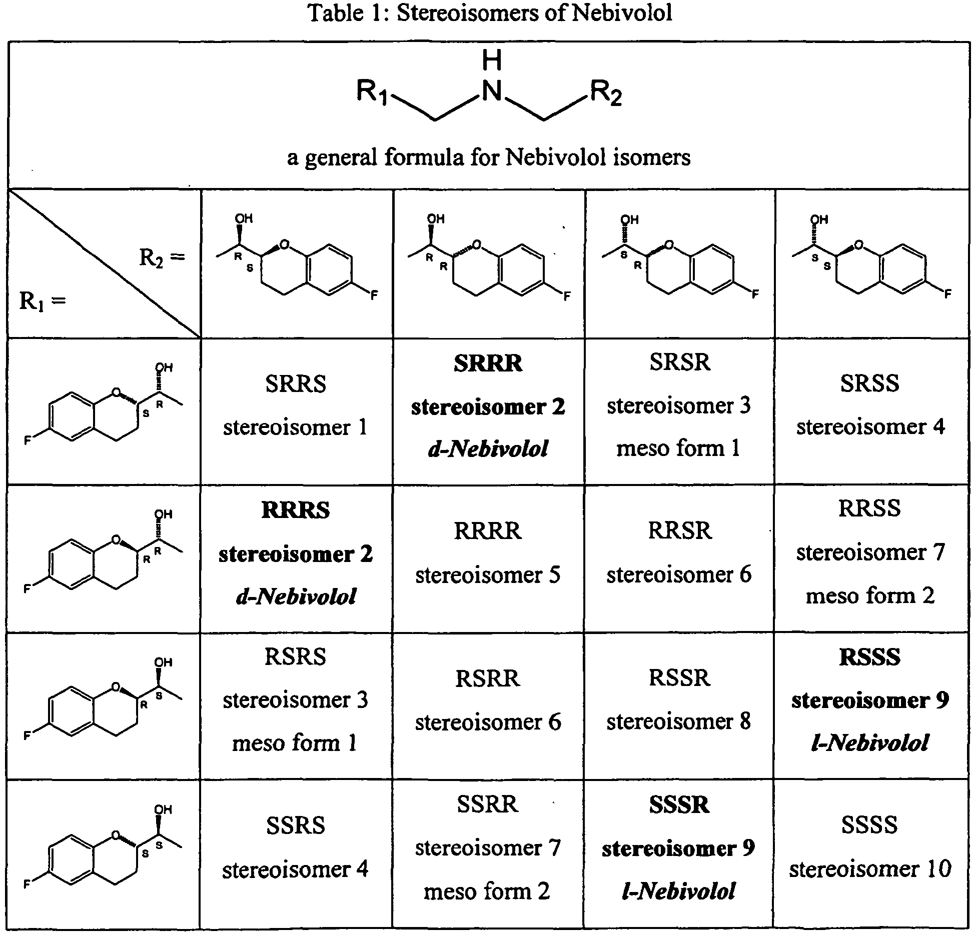

Methods of preparation of Nebivolol as described in the above mentioned references are summarized below.

a. U.S. Pat. No. 4,654,362 (and its counter part EP 0145067 U.S) (Janssen Pharmaceutica N. V.)

The synthetic route for the non-stereoselective preparation of Nebivolol is described, starting from 6-fluoro-4-oxo-4H-1-benzopyran-2-carboxylic acid **a1** (Scheme 1a):

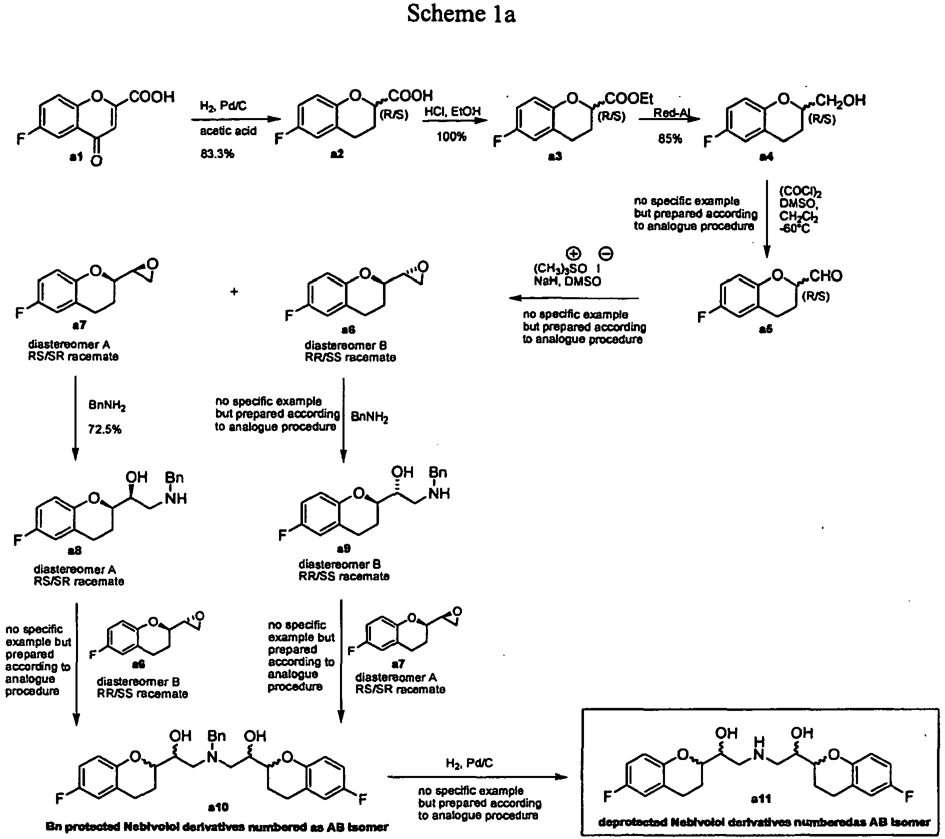

For the preparation of Nebivolol according to the Scheme 1a, U.S. Pat. No. 4,654,362 and its counter-part EP 0145067 contain detailed examples for the synthesis of components **a1**, **a2, a3, a4** and **a8** only. All other examples are analogue procedures that describe the preparation of related derivatives (e.g., derivatives without the aromatic fluoro substituent). The general strategy for the preparation ofNebivolol or its corresponding derivatives is based on the synthesis of the 2-oxiranyl-chromans **(a6** and **a7)** as key intermediates for the final coupling steps. Because they possess two asymmetric carbon atoms, these compounds may be formed from the racemic aldehydes **a5** as two diastereomeric racemates ("an A form" **a7** = RS/SR and "a B form"**a6** = SS/RR) which may be separated by chromatography. This reference does not provide descriptions of a workup procedure, crystallization and purification or separation of stereoisomers, yields etc. for the desired intermediates.

The racemates **a6** or **a7** can be transformed by reacting with benzylamine to the corresponding benzylated aminoalkohols **a8** and **a9.** A benzyl protected Nebivolol AB mixture **a10** may be prepared by reacting of the racemate **a8** (RS/SR) with the epoxide racemate **a6** (RR/SS) or by reacting of racemate **a9** (RR/SS) with the epoxide racemate **a7** (RS/SR). The protecting group may be removed in the final step by catalytic hydrogenation to give a Nebivolol AB mixture **a11.**

Scheme 1b shows further methods for the synthesis of the analogous 2-chromanylaldehydes (**a14**) and 2-oxiranyl-chromanes (**a16**) as key intermediates for the synthesis of Nebivolol derivatives having different substituents at the aromatic moiety.

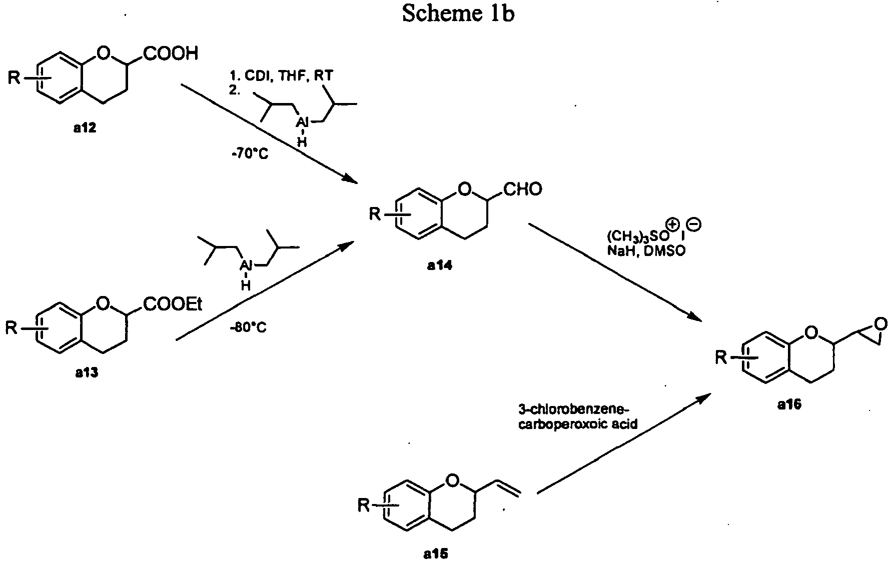

The aldehyde **a14** can be obtained by low temperature reduction of the imidazolide of **a12** or by the same reduction of the ester **a13.** The aldehyde **a14** is then converted into the 2-oxiranyl-chromans **a16** by reaction with sodium hydride and trimethyl sulfoxonium iodide in dimethyl sulfoxide in an analogous reaction as described above. Another possibility for the synthesis of 2-oxiranyl-chromans **a16** is the oxidation of 2-vinylchroman **a15** with 3-chlorobenzenecarboperoxide (the source of 2-vinylchroman **a15** is not described in these patents but according to EP 0334429 (see also below), compound **a14** can be converted into compound **a15** by a Wittig reaction).

Scheme 1c demonstrates that diastereomeric mixtures consisting of desired and undesired diastereomers (i.e., RSSS/SRRR and RSRR/SRSS) can be produced by the method shown in Scheme 1a.

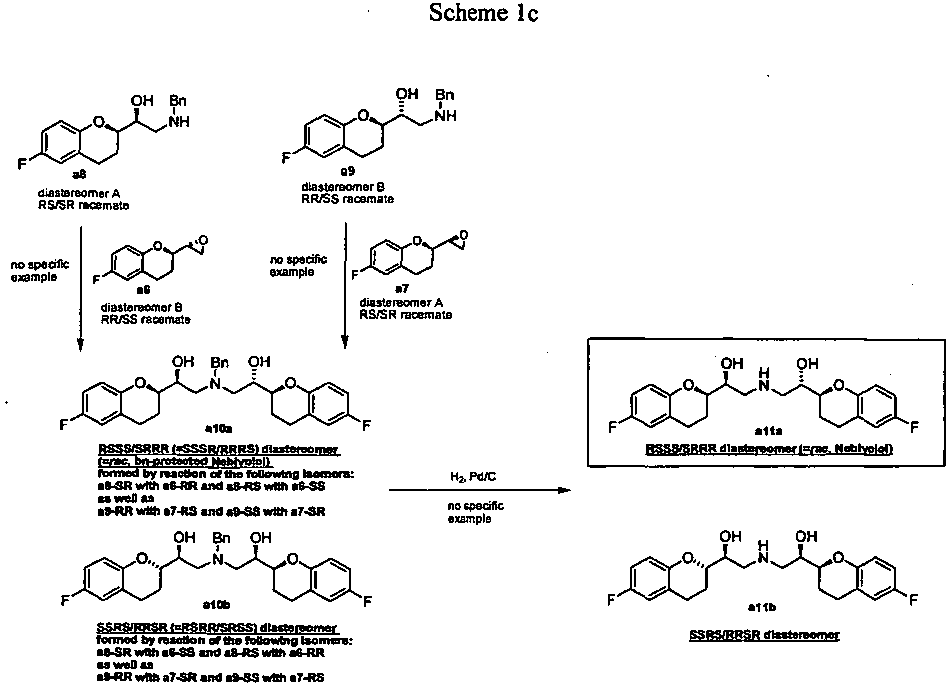

The strategy described in U.S. Pat. No. 4,654,362 and its counter-part EP 0145067 has the following disadvantages:
1. The synthesis of the aldehydes **a6** and **a14** requires very low temperatures and therefore requires special equipment, which makes the process more complicated and expensive;
2. The aldehyde **a5** is very unstable as stated in a PCT publication WO 2004/041805;
3. The synthesis of **a6/a7** from **a5** may be hazardous because it is known that the use of sodium hydride in solvents like DMSO, DMF, DMA and DMI can cause an exotherm and therefore, cause a runaway reaction (see UK Chemical Reaction Hazards Forum: "Sodium Hydride/DMF process stopped");
4. Compounds **a6** and **a7** have been characterized as oily substances (see PCT publication WO 2004/041805). Since the preparation according to the described procedure is likely to form a diastereomeric mixture of **a6** and **a7,** chromatographic purification may be required, which is not commercially viable;
5. Compounds **a10** and **a11** may be prepared by reaction of the racemic intermediate **a8** ("isomer A") with the racemate **a6** ("isomer B") or alternatively by reaction of the racemic intermediate **a9** ("isomer B") with the racemate **a7** ("isomer A") followed by deprotection. U.S. Pat. No. 4,654,362 and its counter-part EP 0145067 do not provide an explicit description as to whether the compounds **a10** and **a11** (characterized only as being the "AB" isomeric form) are single isomers or a mixture of isomers. No teaching for separation of such mixtures has been provided. It is obvious that such procedures may form diastereomeric mixtures consisting of the desired RSSS/SRRR diastereomer and the undesired RSRR/SRSS diastereomer (Scheme 1c; also compare Table 1 demonstrating combination of the different fragments to give all possible diastereomers). Moreover, it is known in prior art (see WO 2004/041805) that racemic Nebivolol prepared according to the process disclosed in U.S. Pat. No. 4,654,362 (and its counter-part EP 0145067) (Schemes 1a and 1c) and obtained as the diastereomeric racemate having the SRSS/RSRR configuration could not be successfully separated by fractional crystallization; and
6. The loss of expensive material via the formation of undesired Nebivolol isomers, especially during late process steps.

b. EP Patent Application Publication EP 0334429 and U.S. Patent No. 6,545,040 to Xhonneux et al. (Janssen Pharmaceutica N. V.)

Similar strategy for the synthesis of Nebivolol is described in EP 0334429 and U.S. Patent No. 6,545,040 but with the difference that *l*-Nebivolol is prepared by an enantioselective synthesis using the enantiopure fragments **b6** and **b11** (Scheme 2) as key intermediates.

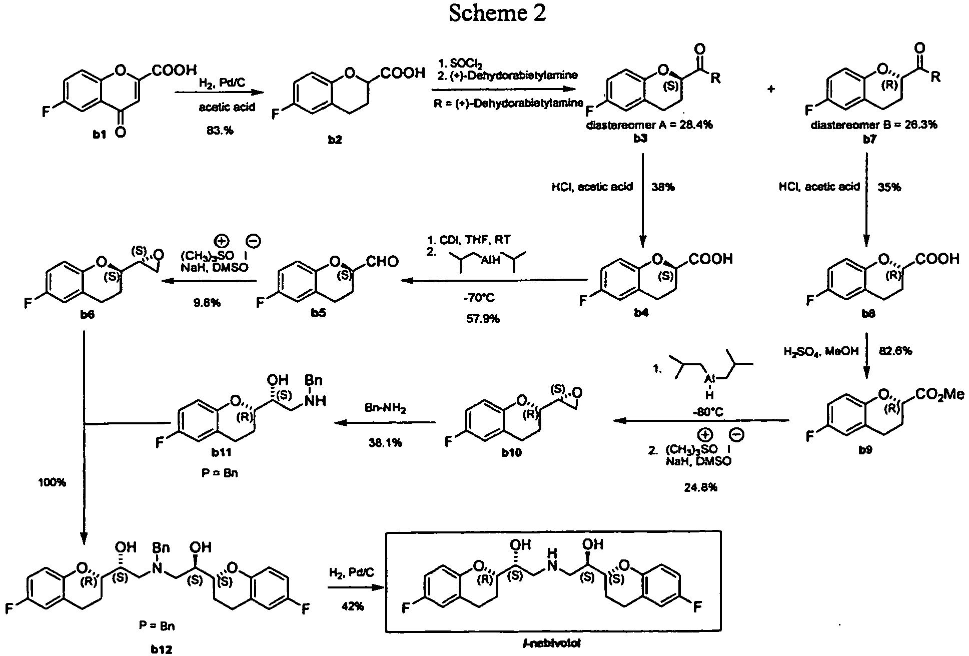

For this procedure, it was necessary to separate the racemic 6-fluoro-chroman-2-ylcarboxylic acid **b2** by formation of a diastereomeric amide **b3** with (+)-dehydroabietylamine followed by fractional crystallization of the diastereomers and hydrolysis of the amides. The next steps for the synthesis of the fragments **b6** and **b11** were done in convergent pathways using the (S)-form and (R)-form of the 6-fluoro-chroman-2-yl-carboxylic acids **b4** and **b8**. The (S)-6-fluoro-chroman-2-yl-carboxylic acid **b4** was first converted to the aldehyde **b5** according to the procedure, already mentioned in scheme 1b. The epoxide **b6** could be then obtained by reacting of **b5** with sodium hydride and trimethyl sulfoxonium iodide in dimethyl sulfoxide. On the second pathway the (R)-6-fluoro-chroman-2-yl-carboxylic acid **b8** was first esterified to **b9**. Epoxide **b10** was synthesized in a one-pot procedure by reduction of **b9** to the corresponding aldehyde followed by reaction with sodium hydride and trimethyl sulfoxonium iodide in dimethyl sulfoxide. The epoxide ring of **b10** was opened by substitution with benzylamine to give the second key fragment **b11,** which was then reacted with the epoxide **b6** to obtain benzyl-protected *l*-Nebivolol **b12**. Final deprotection by catalytical hydrogenation of **b12** gave *l-*Nebivolol.

The strategy described in EP 0334429 and U.S. Patent No. 6,545,040 has the following disadvantages:
1. The steps of preparing compounds **b5** from **b4** and **b10** from **b9** require very low temperatures for the diisobutylaluminum hydride (DIBAH) reduction, making the process more complicated and expensive due to the need for special refrigerating equipment;
2. The steps of preparing compounds **b6** from **b5** and **b10** from **b9** may have safety hazards because it is known that the use of sodium hydride in solvents like DMSO, DMF, DMA and DMI can lead to an exotherm and could cause a runaway reaction (see UK Chemical Reaction Hazards Forum: *"Sodium Hydride*/*DMF process stopped"*);
3. Compounds **b5**, **b6, b9** and **b10** are oily substances and therefore difficult to purify; in the likely case that compounds **b6** and **b10** are contaminated with undesired diastereomers, separation by column chromatography may be required, which is not a commercially viable procedure;
4. The low yields, especially those of steps of preparing compounds **b2-b3-b4, b2-b7-b8** and **b5-b6, b9-b10**, result in a very low overall yield (≤0.5%) for the synthesis of *l-*Nebivolol making this procedure uneconomical;
5. Since only *l*-Nebivolol is prepared and a racemic mixture is required for preparation of Nebivolol, additional steps are necessary to synthesize the corresponding d-form (i.e., *d*-Nebivolol); and
6. Upon reacting the intermediate b2, diastereomers b3 and b7 were formed which then had to be separated and treated separately to yield b6 and b 11, later combined to yield b 12, thus requiring multiple additional steps in the process.

c. PCT Patent Application Publication WO 2004/041805 to Trinka et al., (EGIS GYOGYSZERGYAR RT)

WO 2004/041805 describes the enantioselective synthesis of *d*- and *l*-Nebivolol (see Schemes 3a-c).

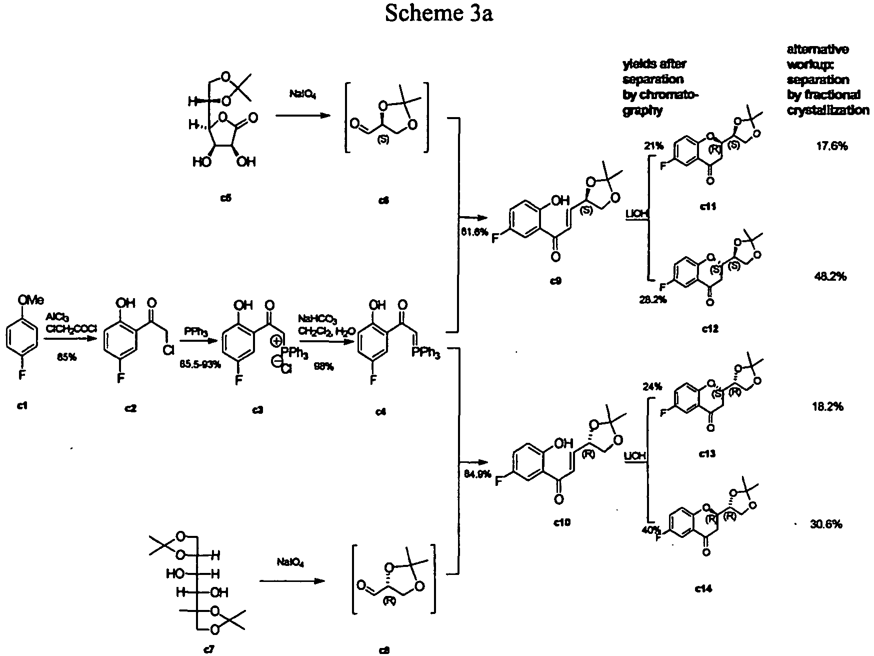

The strategy of this route is based on the synthesis and separation of isopropylidene protected (1',2'-dihydroxy-ethyl)-6-fluoro-chroman-4-one isomers **c11, c12**, **c13**, **c14** (Scheme 3a). These compounds were synthesized by starting with the Friedel-Crafts acylation of 4-fluoroanisole **c1** using chloroacetyl chloride to give the chloroacetyl compound **c2,** which was further transformed with triphenylphosphine followed by treatment with a weak base to form the stable phosphanylidene compound **c4.** The compound **c4** was then reacted separately with protected glycerinealdehydes **c6** (obtained from vitamin C) to give **c11** and **c12** or with **c8** (obtained from D-mannitol) to give **c13** and **c14** after the cycling.

Each of these isomers was further transformed in four pathways and in the same manner (Schemes **3b** and **3c),** whereby according to pathways 1 and 2, *l*-Nebivolol was prepared using c11 and c12 as starting compounds (Scheme 3b).

The enantiomeric *d*-Nebivolol was prepared in the analogous fashion, wherein the starting compounds were the S,R-isomer **c13** and R,R-isomer **c14** of isopropylidene protected (1',2'-dihydroxy-ethyl)-6-fluoro-chroman-4-one (pathways 3 and 4, Scheme 3c).

The typical reaction sequence for each pathway started with the deprotection of **c11** (pathway 1, Scheme 3b), **c12** (pathway 2, Scheme 3b), **c13** (pathway 3, Scheme 3c), **c14** (pathway 4, Scheme 3c) to obtain the respective diols **c15, c19**, c25, **c29.** Selective tosylation of the primary alcohol group gave the compounds **c16**, **c20, c26, c30** which could be transformed to the epoxides **c17**, **c21, c27, c31** by treatment with a base. After the conversion of these epoxides with benzylamine to **c18**, **c22, c28**, **c32** and substitution with the desired epoxides (**c18+c21, c22+c17, c28+c31, c32+c27**), the benzyl protected diketo compounds **c23** and **c33** were formed. Deprotection and reduction of the carbonyl groups could be carried out in a one pot reaction by catalytic hydrogenation to give either *l*-Nebivolol or *d*-Nebivolol.

Racemic Nebivolol was obtained by preparing a 1:1 mixture of the intermediates **c23** and **c33** before performing the last hydrogenation step (yield 52%).

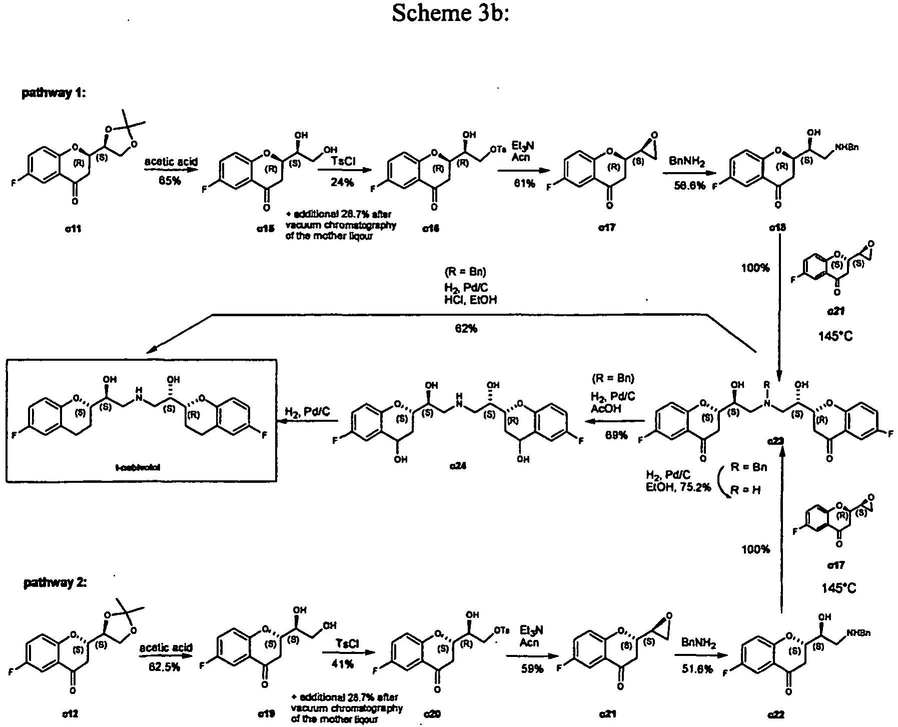

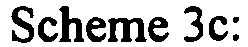

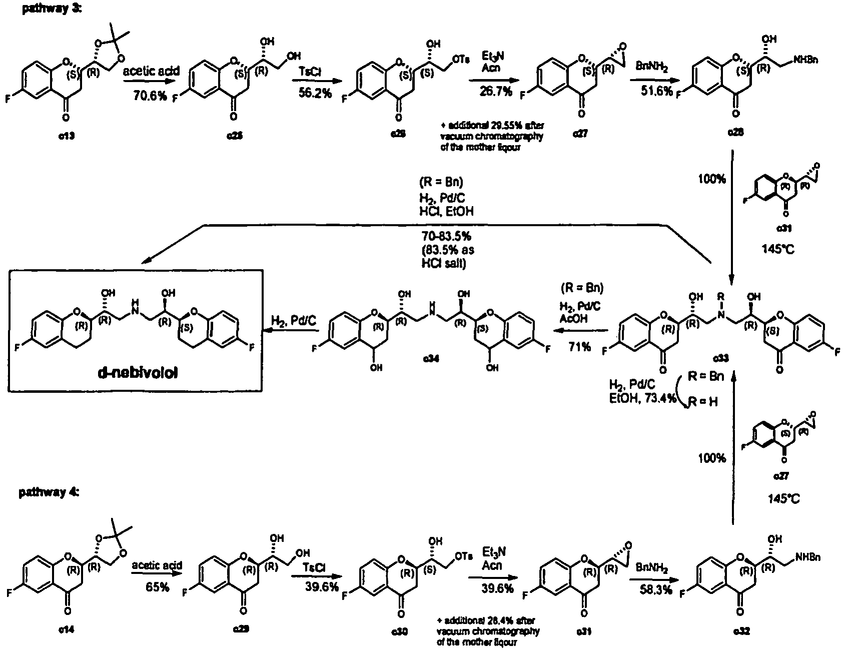

The strategy described in WO 2004/041805 has the following disadvantages:
1. Although the strategy is based on the use of all stereoisomers to synthesize either *l*-Nebivolol or *d*-Nebivolol, the main disadvantage is that up to 30 steps are necessary to get the racemic mixture by using all intermediates, which makes the production protracted and uneconomic; and
2. The steps of making compounds **c23** from **c18**, **c23** from **c22, c33** from **c28** and **c33** from **c32** are carried out without the use of a solvent at 145°C (presumably after melting of the reactant). Such a procedure is not feasible on large scale.

d. Johannes et al., J. Am. Chem. Soc. (1998), 120, 8340-8347

The Johannes et al. article describes an enantioselective preparation of *d*-Nebivolol (Scheme 4).

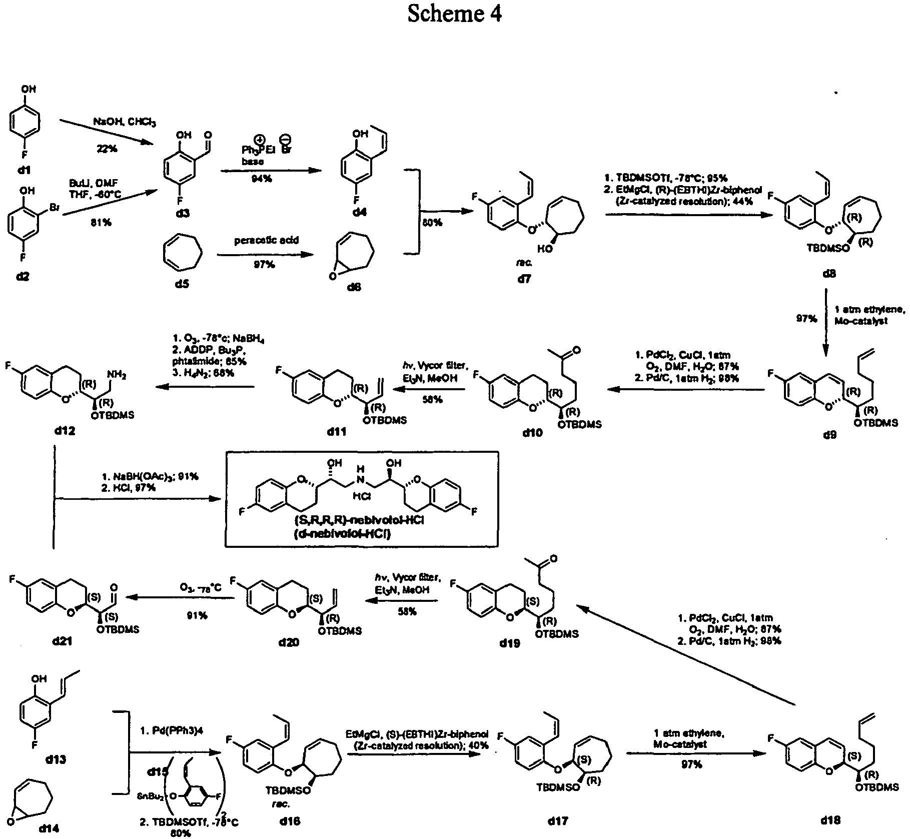

The strategy is based on the syntheses of the chiral chroman fragments **d12** (R, R-configuration) and **d21** (S, S-configuration) as key intermediates in convergent pathways which are finally coupled to give, after deprotection, *d*-Nebivolol. The essential step for the syntheses of these chiral chromans is the Zr-catalyzed kinetic resolution of the racemic intermediates **d7** and **d16.**

According to the first pathway, the starting material for the preparation of chromane fragment **d12** was the salicylic aldehyde **d3**, which was synthesized either by formylation of compound **d1** or by reaction of the lithiated compound **d2** at -60°C with DMF. The allylic cycloheptene epoxide which could be obtained by epoxidation of cycloheptadiene was then reacted with aldehyde **d4** to give the racemic compound **d7** by a regioselective and stereoselective nucleophilic opening of epoxide **d8**. Protection of the hydroxyl group of **d7** using TBSOT followed by treatment with 5 equiv EtMgCl and 10 mol% (R)-(EBTHI)Zr-binol delivered **d8** in 44% yield and >98%ee. The Mo-catalyzed metathesis reaction under an ethylene atmosphere, followed by Wacker oxidation of the terminal double bond and subsequent catalytic hydrogenation, gave **d10** in 83% overall yield. To synthesize **d11** from **d10,** a photochemical Norrish type II cleavage was necessary. The following three-step sequence of ozonolytic cleavage, Mitsunobu reaction using tributylphosphine and phthalide followed by hydrazinolysis to remove the phthalimidyl group gave intermediate **d12.** The second pathway started with the synthesis of cis configured racemate **d16,** which was then resolved in the presence of the Zirconium catalyst (S)-(EBTHI)Zr-biphenol. The compound **d17** was converted into compound **d18** by Mo-catalyzed metathesis reaction. Wacker oxidation of the terminal double bond and subsequent catalytic hydrogenation delivered intermediate **d19,** which was further converted by a photochemical Norrish type II cleavage and ozonolysis into the aldehyde **d21**. *D*-Nebivolol was then obtained by reductive amination of compounds **d12** and **d21** followed by removal of the silyl ether protection groups.

The strategy described in the Johannes et al. article has the following disadvantages:
1. Preparation of aldehyde **d3** occurs either in a low yield by formylation of **d1** using chloroform in the presence of a base or requires low temperature by lithiation and formylation of compound **d2**. Furthermore, handling of n-Buli during a scale-up process requires special precautions;
2. The steps of preparing compounds **d8** from **d7** and **d16** and**d17** from **d13/d14** also require low temperature (-78°C) for the silylation. Furthermore, a difficult resolution step using a special commercially unavailable Zr-catalyst is necessary;
3. The steps of preparing compounds **d10** to **d11** and **d19** to **d20** require special equipment for the photochemical reaction (Norrish type 2 cleavage);
4. The step of preparing compound **d12** from **d11** requires low temperature (-78°C) and special equipment for the ozonolysis; and
5. 16-20 steps are necessary for the synthesis of one Nebivolol enantiomer (d-form), but the racemic mixture is required; thus, additional steps are necessary to synthesize the corresponding 1-form (i.e., *l*-Nebivolol).

e. Chandrasekhar et al., Tetrahedron (2000), 56, 6339-6344

The Chandrasekhar et al. article describes another procedure for the enantioselective synthesis of *d*-Nebivolol (see Scheme 5).

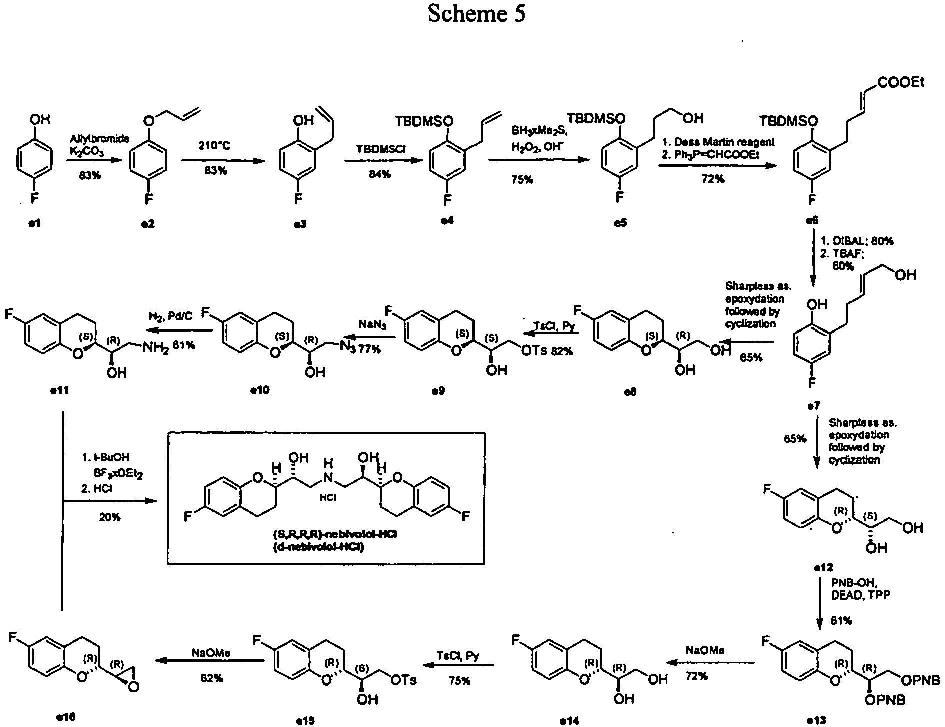

The basis for the enantioselective strategy is the asymmetric one-pot Sharpless epoxidation of allyl alcohol **e7** using (-)-DET and (+)-DET to provide both enantiomeric diols **e8** and **e12** after a cyclization step.

The starting compound was 4-fluoro-phenol **e1,** which was first converted into the allylether **e2.** Claisen rearrangement at 210°C followed by protection of the phenol group **(e3)** with TBDMS-Cl gave the intermediate **e4.** The primary alcohol **e5** was obtained by hydroboration and subsequent oxidative treatment using H₂O₂. This product was converted into the α,β-unsaturated ester **e6** by one pot oxidation with Dess-Martin periodane and Wittig olefination. Afterwards, the compound **e6** was reduced with DIBAL-H to the allyl alcohol **e7.** At this stage, the route was divided into two pathways each starting with the asymmetric Sharpless epoxidation and cyclization in one pot. On the first pathway, the diol **e8** could be obtained in 65% yield by using (-)-DET. Selective tosylation of the primary alcohol **e8** and substitution of **e9** with azide followed by catalytical reduction of **e10** gave the aminoalcohol **e11.** On the second pathway, the diol **e12** was synthesized in an almost similar manner as diol **e8** but with the exception that (+)-DET was used for the Sharples epoxidation to give the corresponding enantiomeric compound. Inversion at the C₂ carbon under Mitsunobu conditions with p-Nitrobenzoic acid gave the di-PNB protected compound **e13.** After removal of the protection groups, the diastereomeric diol **e14** could be obtained. Selective tosylation of **e14** and treatment of resulting **e15** with a base yielded epoxide **e16.** The synthesis of *d*-Nebivolol hydrochloride could be completed by coupling of aminoalcohol **e11** with epoxide **e16** followed by transformation to the hydrochloride salt.

The strategy described in the Chandrasekhar et al. article has the following disadvantages:
1. Step of making compound **e3** from **e2** requires high temperature for the Claisen rearrangement, which is not practicable a in scale-up procedure;
2. Up to 16 steps are necessary to synthesize only one Nebivolol enantiomer, but the racemic mixture is required;
3. The last coupling step yields *d*-Nebivolol in a low yield (20%);
4. The Asymmetric Sharpless epoxidation is known to give non-enantiopure products. Therefore contaminations with other stereoisomers are likely. As already mentioned in WO 2004/041805, the described method for the measurement of the optical purity is not sufficient to determine such possible contaminations.
5. Almost all intermediates were purified by column chromatography because most intermediates are oily compounds.

In summary, multiple steps (> 13 steps), the low yield, the usage of unusual catalyst, reaction conditions, special equipment and column chromatography for purification of the predominantly oily intermediates makes the available strategies and most of the steps too laborious and economically unsuitable for a commercial process.

Despite the above described efforts, there is a need for a new, efficient and commercially feasible process for the preparation of racemic Nebivolol having an improved overall yield.

### BRIEF SUMMARY OF THE INVENTION

The present invention provides new compounds and intermediates as well as processes that can be used directly for the selective synthesis ofNebivolol or racemic ([2S*[R*[R[R*]]]]-and ([2R*[S*[S[S*]]]]-(±)- alpha,alpha' -[imino-bis(methylene)]bis[6-fluoro-chroman-2-methanol] of the formula (1) 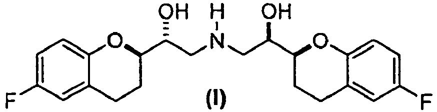 and its pure ([2S[2R*[R[R*]]]]- and ([2R*[S*[S[S*]]]]- enantiomeric compounds and pharmaceutically acceptable salts thereof.

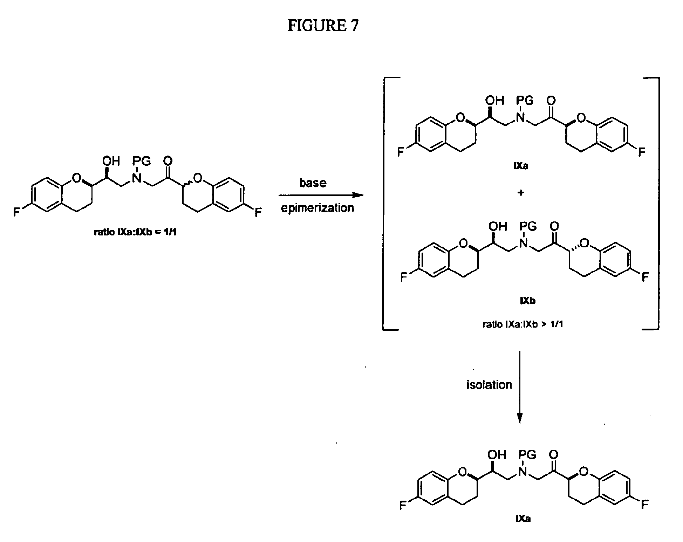

Accordingly, a process for preparing racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] and pharmaceutically acceptable salts thereof includes:
(a) providing a compound of formula (VIII) 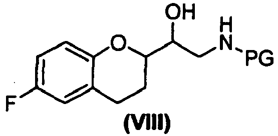 as a diastereomerically pure compound comprising at least 95% of RS/SR configuration or RR/SS configuration, wherein PG is hydrogen or an amine protecting group, wherein the amine protecting group is at least one of an allyl group or an aryl-C₁ alkyl group;
(b) providing a racemic compound of formula (V) 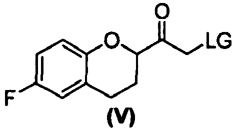 wherein LG is a member selected from the group consisting of chloro, bromo, iodo, alkylsulfonyloxy and arylsufonyloxy;
(c) N-alkylating the compound of formula (VIII) with the compound of formula (V), wherein said N-alkylating is carried out in an inert organic solvent in a presence of a base and optionally in the presence of a catalyst to give a compound of formula (IX) 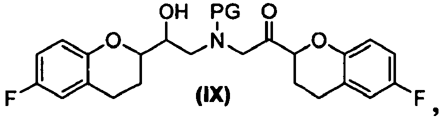 a compound of formula (IX') which is a cyclic semi-ketal form of the compound of formula (IX) 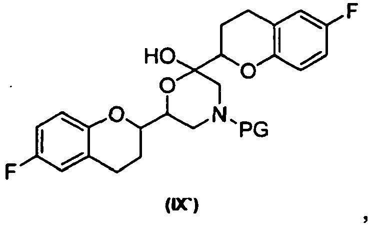 or a mixture thereof, wherein the compound of formula (IX) and the compound of formula (IX') are mixtures of diastereomers;
(d) separating diastereomers of the compound of formula (IX) or the compound of formula (IX') to obtain substantially pure diastereomers of formula (IX) or formula (IX') having at least 50% of a RSS/SRR or RRS/SSR configuration in a simultaneous epimerization-crystallization step, wherein the epimerization-crystallization step comprises:
   (1) epimerizing an RSR/SRS configuration of the compound of formula (IX) or (IX') to give a mixture of the RSS/SRR configuration and the RSR/SRS configuration of diastereomers of formula (IX) or formula (IX') or
      epimerizing an RRR/SSS configuration of the compound of formula (IX) or (IX') to give a mixture of the RRS/SSR configuration and the RRR/SSS configuration of diastereomers of formula (IX) or formula (IX'), provided that said epimerizing is conducted in a presence of a base and an organic solvent wherein the mixture is optionally cooled using a temperature gradient and wherein the RSS/SRR configuration or the RRS/SSR configuration in the mixture are obtained in at least two fold excess relative to the RSR/SRS configuration and the RRR/SSS configuration; and
   (2) crystallizing substantially pure diastereomers of formula (IX) or formula (IX') having the RSS/SRR configuration or the RRS/SSR configuration in at least two fold excess relative to the RSR/SRS configuration and the RRR/SSS configuration;
      separating the mixture by fractional crystallization optionally after salt formation or after derivatization to obtain substantially pure diastereomers of formula (IX) or formula (IX') having the RSS/SRR or RRS/SSR configuration;
(e) reducing substantially pure diastereomers of formula (IX) or formula (IX') having a RSS/SRR or RRS/SSR configuration to give a compound of formula (X) 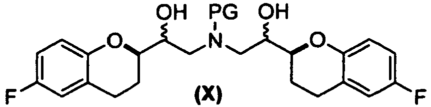 as a RSSS/SRRR diastereomeric mixture having a ratio of a RSSS/SRRR diastereomeric configuration to a SRSR or RRSS diastereomeric configuration, wherein said ratio is at least 1;
(f) deprotecting the compound of formula (X), provided that PG is not H and if PG is H then omitting said deprotection, to obtain a compound of formula (I) 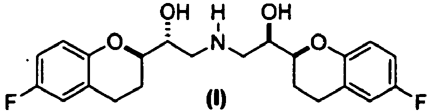 or pharmaceutically acceptable salts thereof; and
(g) removing a RSRS or RRSS diastereomeric configuration of the compound of formula (I) or pharmaceutically acceptable salts thereof if present by recrystallization or by a slurry to give racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] or pharmaceutically acceptable salts thereof. The preferred embodiment of the process is shown in Figures 7-9.

In a preferred embodiment, the protecting group is a benzyl group. In certain embodiments, the leaving group is a chloro or bromo group.

In certain embodiments, in step (c) the organic solvent is a polar aprotic solvent selected from the group consisting of DMF, DMA and NMP.

In certain embodiments, in step (c), the base is at least one of tertiary amines, alkali metal carbonate or alkali metal hydrogen carbonate. Preferably, the base is sodium hydrogen carbonate. Preferably, about 1.5 to about 2.5 equivalents of the base are used.

In certain embodiments, in step (c) the catalyst is at least one of alkali metal bromides, alkali metal iodides, tetraalkylammonium bromides or tetraalkylammonium iodides. Preferably, the catalyst is sodium bromide. In a preferred embodiment, about 0.05 to about 0.25 equivalents of the catalyst are used. In other preferred embodiments, 0.15 equivalents of the catalysts are used.

In certain embodiments, in step (c) said N-alkylation is carried out at a temperature between about room temperature and about 80°C.

In certain embodiments, in step (b) the compound of formula (V) is provided in an amount of about 1.0 to about 1.5 equivalents.

In certain embodiments, step (d) is carried out for the RSR/SRS configuration of the compound of formula (IX) or (IX'). Preferably, the RSS/SRR configuration in the mixture is obtained in at about nine fold excess of the RSR/SRS.

In certain embodiments, in step (d) the mixture is cooled using the temperature gradient from about 70°C to about 20°C. In a preferred embodiment, the temperature gradient is from 70°C to 40°C.

In certain embodiments, in step (d) the organic solvent is acetonitrile.

In certain embodiments, in step (d) said epimerizing is carried out in the presence of at least 0.1 equivalents of the base. In certain embodiments, said epimerizing is carried out in the presence of at least 0.25 equivalents of the base.

In certain embodiments, in step (d) the base is a member selected from the group consisting of an alkoxide, an amidine, a guanidine and a phosphazene. Preferably, the base is an amidine. In a preferred embodiment, the base is diazabicycloundecene.

In certain embodiments, in step (d) water if present cannot exceed 1.0%.

In certain embodiments, in step (d) water if present cannot exceed 0.1 %.

In certain embodiments, in step (e) said reducing is carried out for the RSS/SRR configuration the compound of formula (IX) or the compound of formula in a solvent with alkali borohydride, tetrabutylammonium borohydride, alkali-SELECTRIDE or zinc borohydride, optionally in a presence of a Lewis acid. Preferably, the Lewis acid is at least one of Ti(OAlkyl)4, ZnCl2 alkali halide or alkaline earth halide. In certain embodiments,the solvent is at least one of an ether, an alcohol or a halogenated hydrocarbon. In certain embodiments, said reducing is carried out at temperatures between about -20°C and about room temperature.

In certain embodiments, in step (f) said deprotecting is carried out by catalytic hydrogenation.

In certain embodiments, in step (g) said purifying the compound of formula (I) is done by a slurry of its hydrochloride salt in a solvent. Preferably, the slurry is carried out in methanol as the solvent.

Further provided is a racemic ([2S*[R*[R*[R*]]]]- and ([2R*[S*[S*[S*]]]]-(±)-alpha,alpha' -[imino-bis(methylene)]bis[6-fluoro-chroman-2-methanol] of the compound of the formula (I) prepared by the process described above.

Also provided is a process for preparing racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] and pharmaceutically acceptable salts thereof, the process comprising:
(a) providing a compound of formula (IX) 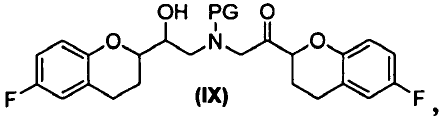 a compound of formula (IX') which is a cyclic semi-ketal form of the compound of formula (IX) 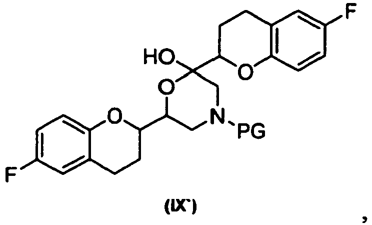 or a mixture thereof, wherein the compound of formula (IX) and the compound of formula (IX') are mixtures of diastereomers;
(b) separating diastereomers of the compound of formula (IX) or the compound of formula (IX') to obtain substantially pure diastereomers of formula (IX) or formula (IX') having at least 50% of a RSS/SRR or RRS/SSR configuration in a simultaneous epimerization-crystallization step, wherein the epimerization-crystallization step comprises:
   (1) epimerizing an RSR/SRS configuration of the compound of formula (IX) or (IX') to give a mixture of the RSS/SRR configuration and the RSR/SRS configuration of diastereomers of formula (IX) or formula (IX') or
      epimerizing an RRR/SSS configuration of the compound of formula (IX) or (IX') to give a mixture of the RRS/SSR configuration and the RRR/SSS configuration of diastereomers of formula (IX) or formula (IX'), provided that said epimerizing is conducted in a presence of a base and an organic solvent wherein the mixture is optionally cooled using a temperature gradient and wherein the RSS/SRR configuration or the RRS/SSR configuration in the mixture are obtained in at least two fold excess relative to the RSR/SRS configuration and the RRR/SSS configuration; and
   (2) crystallizing substantially pure diastereomers of formula (IX) or formula (IX') having the RSS/SRR configuration or the RRS/SSR configuration in at least two fold excess relative to the RSR/SRS configuration and the RRR/SSS configuration;
      separating the mixture by fractional crystallization optionally after salt formation or after derivatization to obtain substantially pure diastereomers of formula (IX) or formula (IX') having the RSS/SRR or RRS/SSR configuration;
(c) reducing substantially pure diastereomers of formula (IX) or formula (IX') having a RSS/SRR or RRS/SSR configuration to give a compound of formula (X) 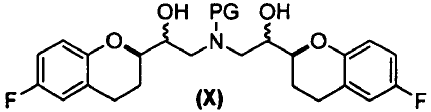 as a RSSS/SRRR diastereomeric mixture having a ratio of a RSSS/SRRR diastereomeric configuration to a SRSR or RRSS diastereomeric configuration, wherein said ratio is at least 1;
(d) deprotecting the compound of formula (X), provided that PG is not H and if PG is H then omitting said deprotection, to obtain a compound of formula (I) 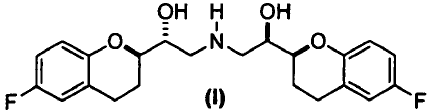 or pharmaceutically acceptable salts thereof; and
(e) removing a RSRS or RRSS diastereomeric configuration of the compound of formula (I) or pharmaceutically acceptable salts thereof if present by recrystallization or by a slurry to give racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] or pharmaceutically acceptable salts thereof.

Further provided is a process for preparing racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] and pharmaceutically acceptable salts thereof includes
(a) providing a compound of formula (VIII) 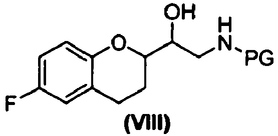 as a diastereomerically pure compound comprising at least 95% of RS/SR configuration or RR/SS configuration, wherein PG is hydrogen or an amine protecting group, wherein the amine protecting group is at least one of an allyl group or an aryl-C₁ alkyl group;
(b) providing a racemic compound of formula (V) 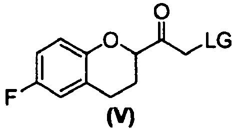 wherein LG is a member selected from the group consisting of chloro, bromo, iodo, alkylsulfonyloxy and arylsufonyloxy;
(c) N-alkylating the compound of formula (VIII) with the compound of formula (V), wherein said N-alkylating is carried out in an inert organic solvent in a presence of a base and optionally in the presence of a catalyst to give a compound of formula (IX) 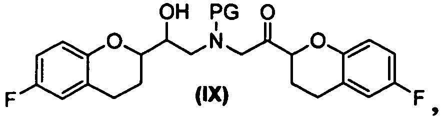 a compound of formula (IX') which is a cyclic semi-ketal form of the compound of formula (IX) 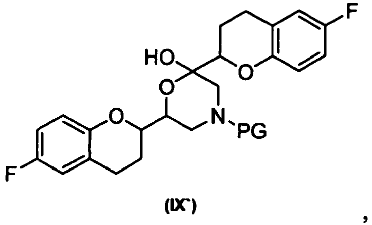 or a mixture thereof, wherein the compound of formula (IX) and the compound of formula (IX') are mixtures of diastereomers;
(d) separating diastereomers of the compound of formula (IX) or the compound of formula (IX') by fractional crystallization after salt formation or after derivatization to obtain PG is H then omitting said deprotecting, to obtain a compound of formula (I) substantially pure diastereomers of formula (IX) or formula (IX') having at least 50% of a RSS/SRR or RRS/SSR configuration;
(e) reducing substantially pure diastereomers of formula (IX) or formula (IX') having a RSS/SRR or RRS/SSR configuration to give a compound of formula (X) 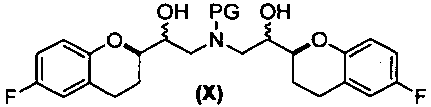 as a RSSS/SRRR diastereomeric mixture having a ratio of a RSSS/SRRR diastereomeric configuration to a SRSR or RRSS diastereomeric configuration, wherein said ratio is at least 1;
(f) deprotecting the compound of formula (X), provided that PG is not H and if PG is H then omitting said deprotecting, to obtain a compound of formula (I) or pharmaceutically acceptable salts thereof; and
(g) removing a RSRS or RRSS diastereomeric configuration of the compound of formula (I) or pharmaceutically acceptable salts thereof if present by recrystallization or by a slurry to give racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] or pharmaceutically acceptable salts thereof.

The preferred embodiment of the process is shown in Scheme 6a.

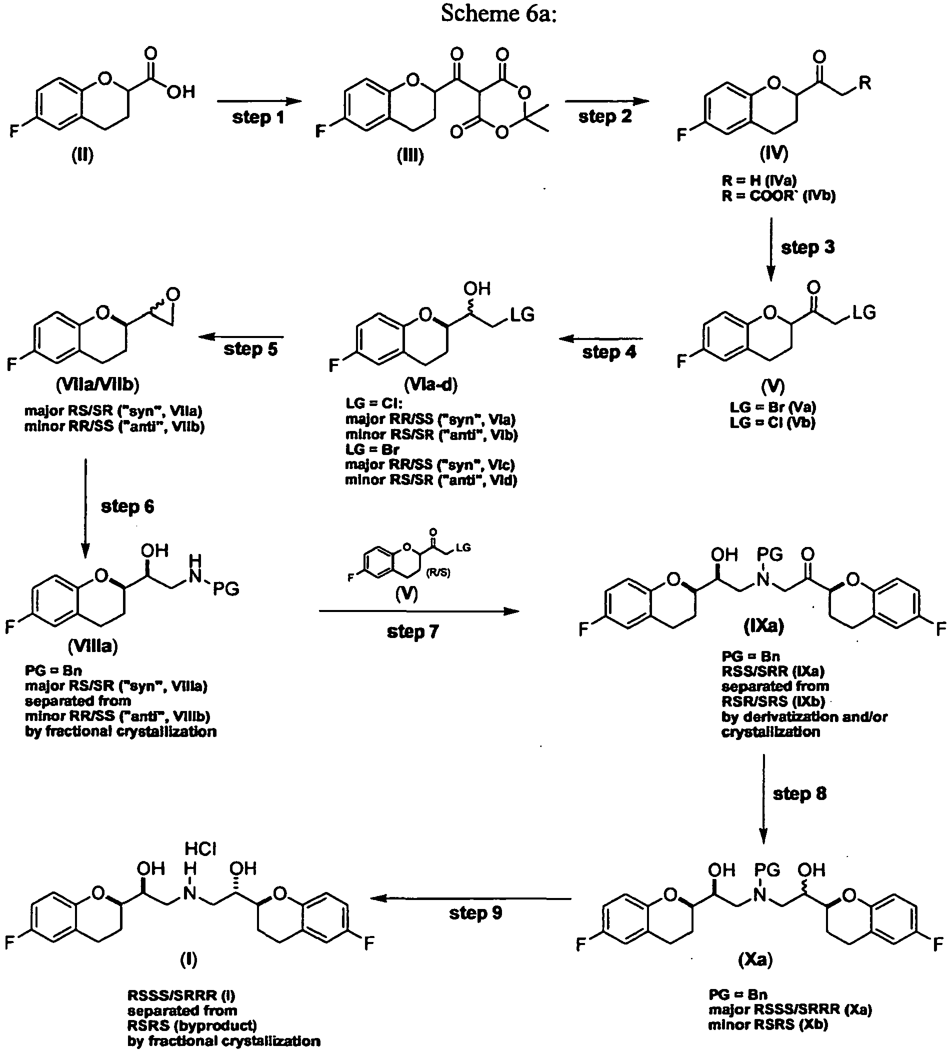

The starting compound is the 6-fluoro-chroman-2-carboxylic acid **II,** which is converted by appropriate transformations (steps 1, 2, and 3) into compound **V** bearing a suitable leaving group (LG). A selective reduction of compound **V**, followed by epoxide formation and substitution with a protected amine, gives compound **VIIIa** after fractional crystallization. In this case, the order of the transformations may be changed without the need for epoxide formation. Coupling of compound **VIIIa** with compound **V** gives a diastereomeric mixture of compounds **IXa** and **Xb,** whereupon compound **IXa** is selectively isolated and almost selectively reduced to a mixture of compounds **Xa** (major) and **Xb** (minor). This mixture is then deprotected, and after salt formation using HCl, racemic Nebivolol hydrochloride **I** is selectively crystallized. The overall yield is 8%; however, additional amounts of compound V used for step 7 were not taken into account.

In a preferred embodiment, the protecting group is a benzyl group. In certain embodiments, the leaving group is a chloro or bromo group.

In certain embodiments of the process, in step (b) the compound of formula (V) is provided in the amount of about 1.0 to about 1.5 equivalents.

In certain embodiments of the process, in step (c) the organic solvent is a polar aprotic solvent selected from the group consisting of DMF, DMA and NMP.

In certain embodiments of the process, in step (c) the base is at least one of tertiary amines, alkali metal carbonate or alkali metal hydrogen carbonate. Preferably, the base is sodium hydrogen carbonate. Preferably, about 1.5 to about 2.5 equivalents of the base are used.

In certain embodiments of the process, in step (c) catalyst is at least one of alkali metal bromides, alkali metal iodides, tetraalkylammonium bromides or tetraalkylammonium iodides. Preferably, the catalyst is sodium bromide. Preferably, about 0.1 to about 0.25 equivalents of the catalyst are used and most preferably, 0.15 equivalents of the catalysts are used.

In certain embodiments, in step (c) said N-alkylation is carried out at a temperature between about room temperature and about 80°C.

In certain embodiments, in step (d) the fractional crystallization is carried out in a solvent. Preferably, the solvent is acetonitrile. Preferably, a free amine is used for the fractional crystallization. In certain embodiments, about 0.4/n to about 0.6/n equivalents of the silylating reagent are used and n is an amount of transferred silyl groups per the silylating reagent. Preferably, the silylating reagent is at least one of trimethylsilyl chloride (TMSCl), HMDS or BSU.

In certain embodiments, in step (d) a silylation reagent is used for derivatization prior to the fractional crystallization from the solvent. Preferably, the derivatization is carried out in the presence of about 1.0 to about 2.0 equivalents of a base. Preferably, the base is imidazole.

In certain embodiments, in step (d) said separating the diastereomers of the compound of formula (IX) or the compound of formula (IX') is carried out in acetonitrile, MTBE, cyclohexane or mixtures thereof.

In certain embodiments, in step (e), said reducing is carried out for the RSS/SRR configuration the compound of formula (IX) or the compound of formula in a solvent with alkali borohydride, tetrabutylammonium borohydride, alkali-SELECTRIDE or zinc borohydride, optionally in a presence of a Lewis acid. In certain embodiments, the Lewis acid is at least one of Ti(OAlkyl)₄, ZnCl₂ alkali halide or alkaline earth halide. Preferably, the solvent is at least one of an ether, an alcohol or a halogenated hydrocarbon. In certain embodiments, said reducing is carried out at temperatures between about -20°C and about room temperature.

In certain embodiments, in step (f) said deprotecting is carried out by catalytic hydrogenation.

In certain embodiments, in step (g) said purifying the compound of formula (I) is done by a slurry of its hydrochloride salt in a solvent. Preferably, the slurry is carried out in methanol as the solvent.

In certain embodiments said providing the compound of formula (VIII) includes:
(i) reducing the racemic compound of formula (V) in a solvent and optionally in a presence of a Lewis acid, wherein LG is bromine or chlorine to give a diastereomeric mixture of compounds of formula (VI) 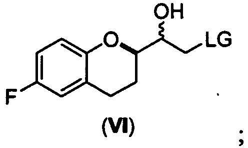
(ii) forming a mixture of diastereomers of a compound of formula (VII) 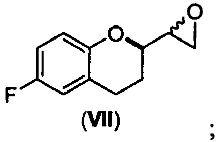
(iii) reacting diastereomers of the compound of formula (VII) with NH₂PG to give a mixture of diastereomers of the compound of formula (VIII) 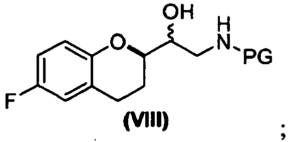 and
(iv) separating diastereomers of the compound of formula (VIII) from the mixture of diastereomers by the fractional crystallization optionally after formation of a salt. In a preferred embodiment, PG is a benzylic group.

In certain embodiments, at least one of the diastereomers of the compound of formula VIII having a RR/SS or RS/SR configuration is isolated.

In another aspect of the present invention, a recycling process of undesired diastereomers produced during the process is provided, which reduces the costs and makes the process of making Nebivolol more efficient. Specifically, during the selective preparations of compounds **VIIIa** and **Xa,** the undesired diastereomers are formed as minor products. Thus, recycling of the waste presents an economical and ecological advantage over prior methods of making Nebivolol.

Also, the alkylation of compound **VIIIa** with compound **V** forms a diastereomeric mixture (a 1/1 mixture of **IXa** and **IXb),** wherein the recycling step enables the transformation of the undesired form **IXb** either by selective cleavage into compound **VIIIa** or by a suitable epimerization step into a mixture of compounds **IXa** and **IXb** followed by selective isolation of desired diastereomer **IXa.**

In certain embodiments of the process, recycling the RR/SS configuration of the compound of formula (VIII) is conducted, wherein said recycling comprises:
providing the RR/SS configuration of the compound of formula (VIII) with a protective group; and
inversion of the alcohol configuration to provide the SR/RS configuration of formula VIII.

In certain embodiments, in step i) the reducing agent is selected from alkali borohydride, tetraalkylammonium borohydride, zinc borohydride, alkali triacetoxyborohydride, SUPERHYDRIDE, RED-AL, alkali-SELECTRID (alkali tri(sec-butyl) borohydride) or coordinated borohydrides. In certain embodiments, in step i) the reduction is carried out under Meerwein Pondorf Verley conditions. In certain embodiments, in step i) said reducing is carried out by catalytic hydrogenation. In certain embodiments, in step i) the Lewis acid is a member selected from the group consisting of alkali or alkaline earth chlorides, zinc chloride, titanium(IV) alkoxide, and aluminium trialkoxide. In certain embodiments, in step i) said reducing is carried out under conditions which give an RR/SS isomer of the compound of formula (VI) in excess. In certain embodiments, in step i) said reducing is carried out at a temperature between about-78°C and about room temperature. Preferably, said reducing is carried out at the temperature between-20°C and room temperature. In certain embodiments, in step i) the solvent is a member selected from the group consisting of alcohols, ethers, halogenated hydrocarbons and aromatic solvents.

In certain embodiments, in step ii) said forming the mixture of diastereomers of the compound of formula (VII) is carried out in a solvent and in a presence of a base. Preferably, the solvent is an alcohol and the base an alkali alkoholate. Preferably, 1.0 to 2.0 equivalents of the base are used.

In certain embodiments, in step ii) said forming of the mixture of diastereomers of the compound of formula (VII) is carried out at temperatures between 0°C and 40°C.

In certain embodiments, in step iv) the fractional crystallization is carried out in toluene, acetonitrile, a C₁-C₃-alcohol, an ether or mixtures thereof. Preferably, the C₁-C₃-alcohol is 2-propanol and the ether is at least one of diisopropylether or MTBE.

In certain embodiments, said providing the racemic compound of formula (V) comprises:
(1) transforming a compound of formula (II) 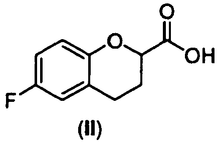 into an activated acid derivative;
(2) reacting the activated acid derivative with Meldrums acid in a presence of a base to give a compound of formula (III) 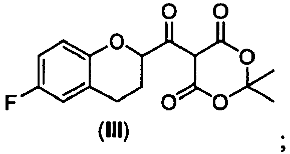
(3) converting the compound of formula (III) into a compound of formula (IV) 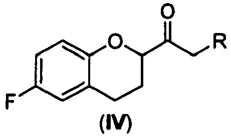 wherein R is hydrogen or COOR' and wherein R' is C₁-C₆ alkyl or aryl-C₁ alkyl; and
(4) halogenating the compound of formula (IV) and optionally conducting hydrolysis and decarboxylation to give the compound of formula (V).

In certain embodiments, in step (1) the carboxylic acid is transformed into a corresponding acid chloride.

In certain embodiments, in step (2) the base is a tertiary amine. In certain embodiments, 1 to 3 equivalents of Meldrums acids are used. In certain embodiments, in step (2), the reaction temperature is between about -10°C and about +30°C.

In certain embodiments, in step (3) the compound of formula (III) is hydrolyzed in a mixture of an organic acid and water to give a compound of formula (IV) wherein R is H. In a preferred embodiment, the organic acid is acetic acid and the hydrolysis is carried out at a reflux temperature. In certain embodiments, in step (3) the compound of formula (IV) having R as COOR' and R' as C₁-C₆ alkyl or aryl-C₁ alkyl is prepared by an alkoholysis of the compound of formula (III). Preferably, the alkoholysis is carried out with ethanol and *tert*-butanol. Preferably, the alkoholysis is carried out at temperatures between about 70°C and about 80°C.

In certain embodiments, in step (3) the solvent is at least one of alcohol or toluene.

In certain embodiments, in step (4) before the halogenation is carried out, the compound of formula (IV) wherein R is H is transformed to a corresponding silylenol ether having the terminal double bond by silylation. In certain embodiments, the silylation is done by a kinetically controlled deprotonation using LDA followed by silylation at about -78 °C to about - 40 °C. Preferably, the silylation is done at -78°C to -70°C. In a preferred embodiment, the silylating reagent is TMSC1.

In certain embodiments, in step (4) after the transformation to the silylenol ether, the halogenation is carried out by using a brominating reagent. Preferably, the bromination reagent is NBS.

In certain embodiments, in step (4) the compound of formula (IV) wherein R is COOR' is first halogenated and then transformed into the compound of formula (V) by ester hydrolysis followed by decarboxylation. Preferably, the halogenation is done in a presence of a catalyst. In certain embodiments, about 1.0 to about 1.5 equivalents of NBS, NCS or SO₂Cl₂ are used as halogenation reagents. In certain embodiments, about 0.2 equivalents to 0.4 equivalents of Mg(ClO₄)₂ are used as a catalyst.

In certain embodiments, in step (4) said halogenating is carried out at temperatures between 0°C and about room temperature.

In certain embodiments, in step (4) after said halogenating, the hydrolysis of the ester followed by decarboxylation is carried out in an aqueous organic acid solution. Preferably, the organic acid is at least one of trifluoro acetic acid, formic acid and acetic acid. Preferably, the hydrolysis and decarboxylation are carried out at temperatures between about 75°C and about 90°C.

In certain embodiments, the process further includes recycling an RSR/SRS or RRR/SSS configuration of the compound of formula (IX) or (IX'), wherein said recycling comprises:
epimerizing the RSR/SRS or RRR/SSS configuration of the compound of formula (IX) or (IX') to give a mixture of the RSS/SRR configuration containing the RSR/SRS configuration of diastereomers of formula (IX) or formula (IX') or RRS/SSR configuration containing the RRR/SSS configuration of diastereomers of formula (IX) or formula (IX') and
separating the mixture by fractional crystallization after salt formation or after derivatization to obtain substantially pure diastereomers of formula (IX) or formula (IX') having the RSS/SRR or RRS/SSR configuration.

In certain embodiments, the process further includes recycling an RSR/SRS or RRR/SSS configuration of the compound of formula (IX) or (IX') wherein said recycling comprises:
cleaving the RSR/SRS or RRR/SSS configuration of the compound of formula (IX) or (IX') to give a mixture comprising an RS/SR or RR/SS configuration of diastereomers of formula (VIII); and
separating the RS/SR or RR/SS configuration of diastereomers of formula (VIII).

Further provided is a compound of formula (III)

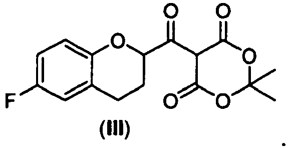

Further provided is a compound of formula (IV) 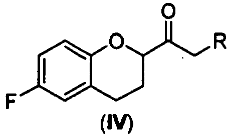 wherein R is hydrogen or COOR' and R' is C₁-C₆ alkyl or aryl-C₁ alkyl.

Further provided is a compound of formula (V) 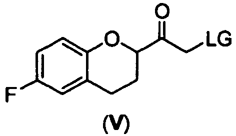 wherein LG is bromine or chlorine.

Further provided is a compound of formula (VI) 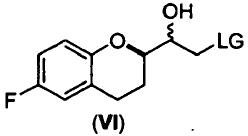 wherein LG is bromine or chlorine.

Further provided is a compound of formula (IX) 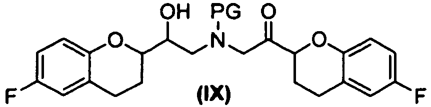 or its cyclic semi-katal form having the formula (IX') 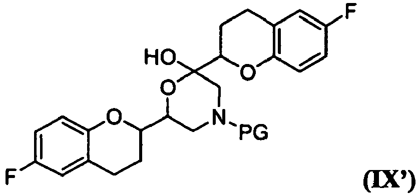 wherein PG is protecting group selected from hydrogen, allyl and aryl-C₁alkyl.

Further provided is a compound of formula (IX) having a RSS/SRR configuration 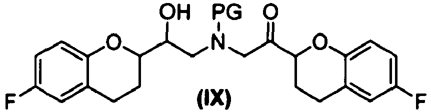 or its corresponding cyclic semi-ketal form (IX') 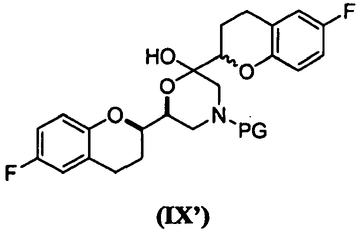 wherein PG is a benzylic group.

Also provided is a process for preparing racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] and pharmaceutically acceptable salts thereof, the process comprising: providing a compound of formula (IX); and reducing the compound of formula (IX) to obtain a compound of formula (X) having at most 50 % of a stereoisomer having a RSRS configuration. This process further comprises providing a compound of formula (VIII) and a compound of formula (V).

In accordance with the invention, enantiomerically pure Nebivolol (1-Nebivolol or d-Nebivolol) may also be obtained, e.g., after resolution of compound **II** (see Schemes 6b and 6c). Each of the enantiomers (S-**II** and R-**II)** may be transformed by the same methods as described for the corresponding racemic compound **II** (compare Scheme 6a) to give the first key intermediates S-**V** and R-**V**. L-Nebivolol-Hydrochloride (Scheme 6b) may be then prepared by the synthesis of R-**VIIIa** starting from R-**V,** followed by coupling with S-**V** (step 11), selective reduction (step 12), deprotection and salt formation (step 13). D-Nebivolol-Hydrochloride may be prepared in the same manner with the exception that, in contrast to the synthesis of 1-Nebivolol, the enantiomeric intermediate S-**VIIIa** will be prepared from S-**V** and then coupled with R-V (Scheme 6c, step 11). Selective reduction (step 12) followed by deprotection and salt formation will give d-Nebivolol-Hydrochloride.

It will be apparent to one skilled in the art that various changes and modifications of these routes may be used for enantioselective syntheses of l- or d-Nebivolol. Therefore, the use of intermediates for the enantioselective synthesis of l- or d-Nebivolol, prepared according to Schemes 6b and 6c, is not limited to the described routes. For example, the epoxides R-VIIa, R-VIIb, S-VIIa and S-VIIb (prepared from the key intermediates R-V or S-V) may also be used directly for the synthesis of each Nebivolol enantiomer, e.g., according to the route described in Scheme 2. Whether the epoxides R-VIIa, R-VIIb, S-VIIa and S-VIIb are prepared as major or minor compounds depends on the reducing agent.

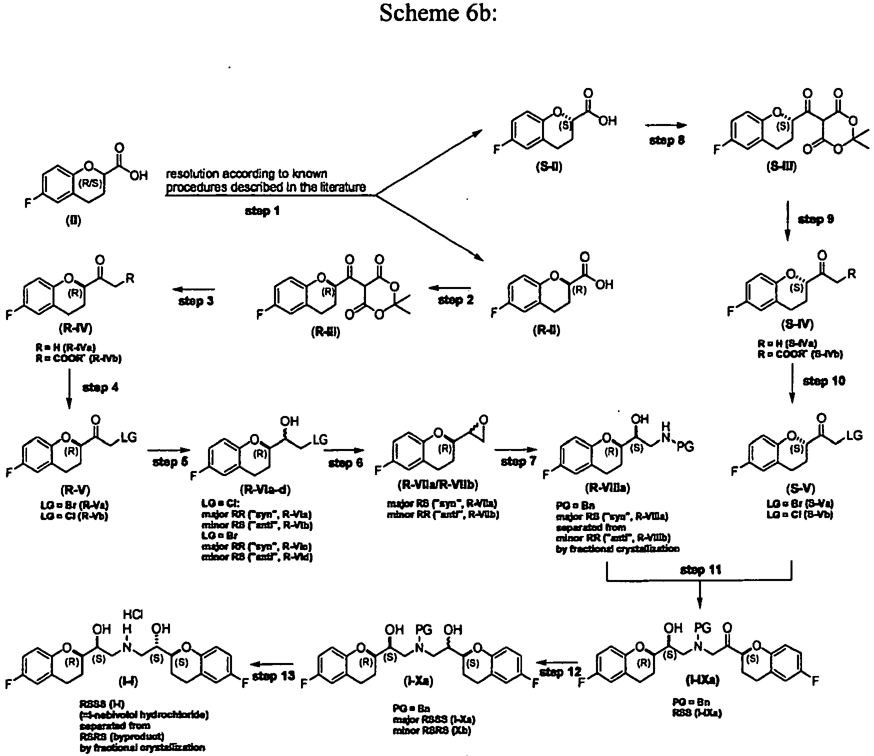

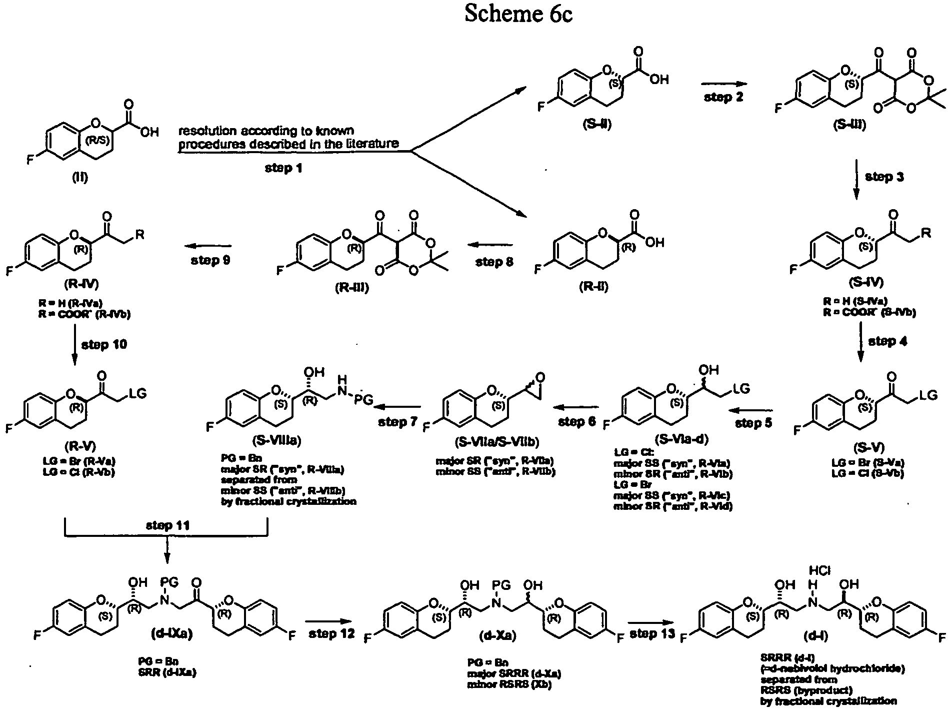

The process of preparing a compound of formula (I) as a racemic mixture or an enantomerically pure form and pharmaceutically acceptable salts thereof includes:
(a) resolving a compound of formula (II) 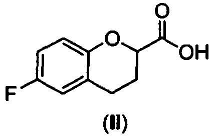 to obtain an S configuration and an R configuration of the compound of formula (II);
(b) converting the S configuration of the compound of formula (II) into an S configuration of a compound of formula (V) 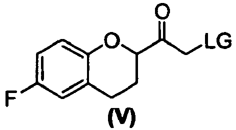 wherein LG is a member selected from the group consisting of chloro, bromo, iodo, alkylsulfonyloxy and arylsufonyloxy, via formation of an S configuration of a compound of formula (III) and an S configuration of a compound of formula (IV);
(c) converting the R configuration of the compound of formula (II) into an R configuration of the compound of formula (V) via formation of an R configuration of the compound of formula (III) and an R configuration of the compound of formula (IV);
(d) providing a compound of formula (VIII) 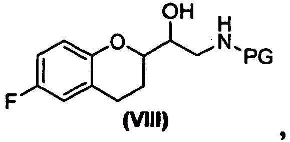 wherein PG is hydrogen or an amine protecting group, wherein the amine protecting group is at least one of an allyl group or an aryl-C₁ alkyl group and wherein the compound of formula (VIII) is enantiomeric compound having an RS or SR configuration;
(e) conducting N-alkylation of (i) the RS configuration of compound of formula (VIII) with the S configuration of compound of formula (V) or (ii) the SR configuration of compound of formula (VIII) with the R configuration of compound of formula (V), provided that said N-alkylation is carried out in an inert organic solvent in a presence of a base and optionally in the presence of a catalyst to give a RSS or SRR enantiomeric form of a compound of formula (IX) 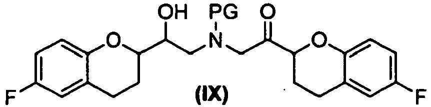 or a RSS or SRR enantiomeric form of a compound of formula (IX') which is a cyclic semi-ketal form of the compound of formula (IX) 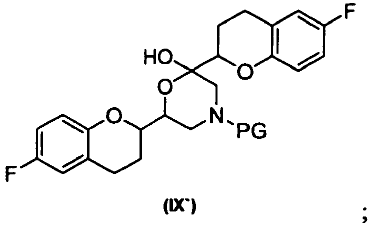
(f) reducing at least one of the RSS or SRR enantiomeric form of the compound of formula (IX) or formula (IX') to give at least one RSSS or SRRR enantomeric form of a compound of formula (X) 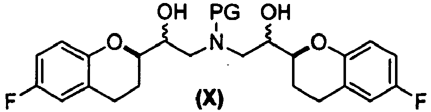
(g) deprotecting the at least one of the RSSS or SRRR enantomeric form of the compound of formula (X), provided that PG is not H and if PG is H then omitting said deprotecting, to obtain the compound of formula (I) or pharmaceutically acceptable salts thereof; and
(h) removing a RSRS diastereomeric configuration of the compound of formula (I) or pharmaceutically acceptable salts thereof if present as a byproduct by recrystallization or by a slurry to give at least one of ([2S*[R*[R*[R*]]]]- and ([2R*[S*[S*[S*]]]]- enantiomer of the compound of the formula (I) and pharmaceutically acceptable salts thereof; and
(i) optionally combining ([2S*[R*[R*[R*]]]]- and ([2R*[S*[S*[S*]]]]- enantiomer of the compound of the formula (I) and pharmaceutically acceptable salts thereof to form racemic ([2S*[R*[R*[R*]]]]- and ([2R*[S*[S*[S*]]]]-(±)- alpha,alpha' -[imino-bis(methylene)]bis[6-fluoro-chroman-2-methanol] of the compound of the formula (I) and pharmaceutically acceptable salts thereof.

Also provided is a process for preparing racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] and pharmaceutically acceptable salts thereof, the process comprising:
(a) providing a compound of formula (VIII) as a diastereomer having RR/SS configuration, wherein PG is hydrogen or an amine protecting group, wherein the amine protecting group is at least one of an allyl group or an aryl-C₁ alkyl group;
(b) providing a racemic compound of formula (V) wherein LG is a member selected from the group consisting of chloro, bromo, iodo, alkylsulfonyloxy and arylsufonyloxy;
(c) N-alkylating the compound of formula (VIII) with the compound of formula (V), wherein said N-alkylating is carried out in an inert organic solvent in a presence of a base and optionally in the presence of a catalyst to give a compound of formula (IX), a compound of formula (IX') which is a cyclic semi-ketal form of the compound of formula (IX), or a mixture thereof, wherein the compound of formula (IX) and the compound of formula (IX') are mixtures of diastereomers having a RRR/SSS and RRS/SSR configuration;
(d) separating diastereomers of the compound of formula (IX) or the compound of formula (IX') by fractional crystallization after salt formation or after derivatization to obtain substantially pure diastereomers of formula (IX) or formula (IX') having at least 50% of the RRR/SSS or RRS/SSR configuration;
(e) reducing the substantially pure diastereomers of formula (IX) or formula (IX') having a RRS/SSR configuration to give a compound of formula (X) as a RSSS/SRRR diastereomeric mixture having a ratio of a RSSS/SRRR diastereomeric configuration to a SRSR or RRSS diastereomeric configuration, wherein said ratio is at least 1;
(f) deprotecting the compound of formula (X), provided that PG is not H, to obtain a compound of formula (I) or pharmaceutically acceptable salts thereof; and
(g) removing a RSRS diastereomeric configuration of the compound of formula (I) or pharmaceutically acceptable salts thereof if present by recrystallization or by a slurry to give racemic [2S[2R*[R[R*]]]] and [2R[2S*[S[S*]]]]-(±)α,α'-[iminobis(methylene)]bis[6-fluoro-3,4-dihydro-2H-1-benzopyran-2-methanol] or pharmaceutically acceptable salts thereof.

In certain embodiments, the process further comprises cleaving the RRR/SSS configuration of the compound of formula (IX) or the compound of formula (IX') to give the compound of formula (VIII) as the diastereomer having RR/SS configuration.

In one variant, the process further comprises epimerizing the RRR/SSS configuration of the compound of formula (IX) or the compound of formula (IX') to give said mixtures of diastereomers having the RRR/SSS and RRS/SSR configuration of the compound of formula (IX) or the compound of formula (IX'). In yet another variant of the above variant, the process further comprises cleaving the RRR/SSS configuration of the compound of formula (IX) or the compound of formula (IX') to give the compound of formula (VIII) as the diastereomer having RR/SS configuration.

Also provided is a process of making a compound of formula (VIII) 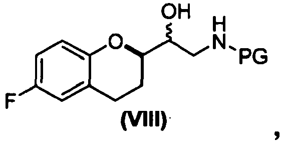 the process includes
(i) providing a racemic compound of formula (V) 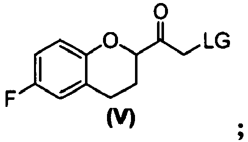
(i) reducing the racemic compound of formula (V) in a solvent and optionally in a presence of a Lewis acid, wherein LG is bromine or chlorine to give a diastereomeric mixture of a compound of formula (VI) 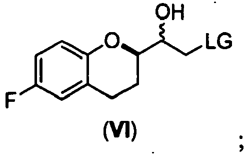
(ii) forming a mixture of diastereomers of a compound of formula (VII) 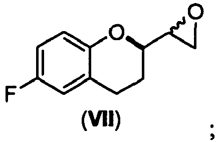
(iii) reacting diastereomers of the compound of formula (VII) with NH₂PG to give the compound of formula (VIII) as a mixture of diastereomers; and
(iv) optionally separating diastereomers of the compound of formula (VIII) from the mixture of diastereomers by the fractional crystallization.

Novel compounds discovered by the inventors include the following 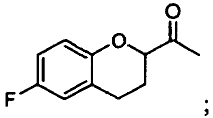 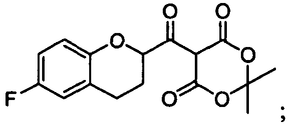 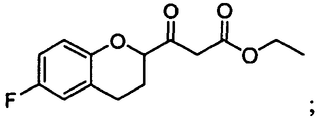 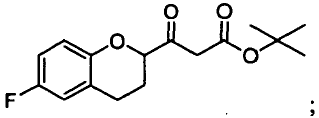 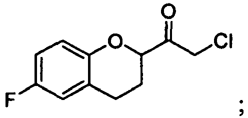 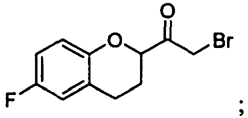 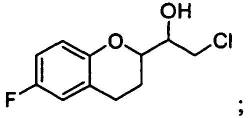 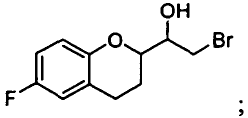 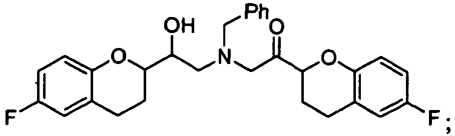 and

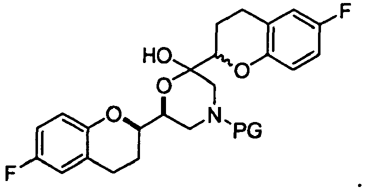

### BRIEF DESCRIPTION OF SEVERAL VIEWS OF THE DRAWINGS

The invention will be described in conjunction with the following drawings in which like reference numerals designate like elements and wherein:
Figure 1A depicts a structural formula of d-Nebivolol, Figure 1B depicts a structural formula of racemic Nebivolol.
Figure 2 is an atom-numbering schematic representation of the molecule of compound VIIIa (50% probability ellipsoids; H-atoms given arbitrary displacement parameters for clarity).
Figure 3 is a ¹³C-NMR graph of compound IXa in a cyclic semi-ketal form.
Figure 4 is a ¹³C-NMR graph of compound IXb in a cyclic semi-ketal form.

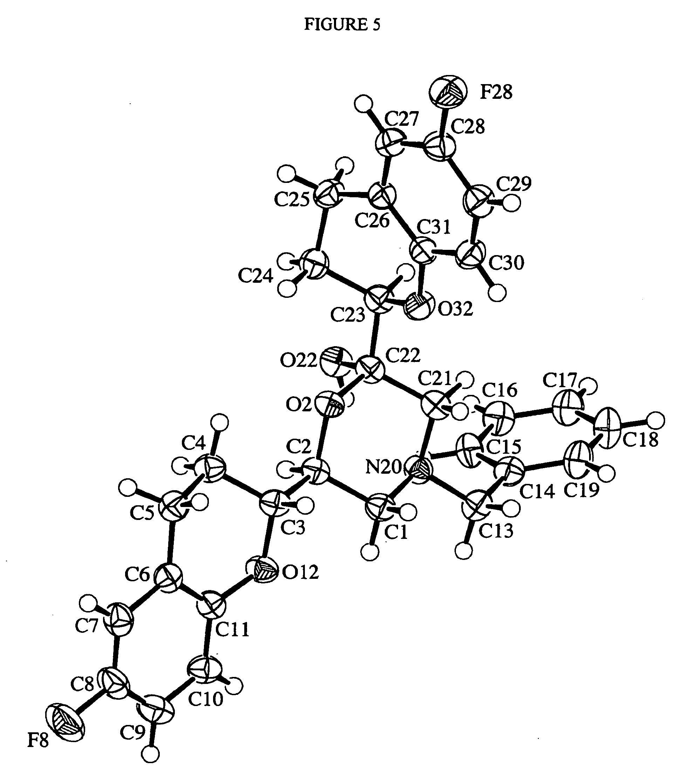

Figure 5 is an atom-numbering schematic representation of the molecule of compound IXb in a cyclic semi-ketal form.
Figure 6 is a scheme demonstrating a process of making racemic Nebivolol and its pharmaceutically acceptable salts.
Figure 7 is a scheme demonstrating the simultaneous epimerization-crystallization procedure of the invention.
Figure 8 is a scheme demonstrating the preferred example of the simultaneous epimerization-crystallization procedure of the invention, wherein the protective group is a benzyl group.
Figure 9 is a scheme demonstrating a process of making racemic Nebivolol and its pharmaceutically acceptable salts wherein step 7b depicts the simultaneous epimerization-crystallization step of the invention.

### DETAILED DESCRIPTION OF THE INVENTION

The present invention provides new compounds and methods for the synthesis of a racemic Nebivolol and its pharmaceutically acceptable salts as well as an enantiomerically pure Nebivolol and its pharmaceutically acceptable salts. This invention was driven by a desire to create a more efficient process having fewer reaction steps by avoiding steps of separating enantiomers prior to making a racemic mixture. One example of separating enantiomers is described in WO 2004/041805 (Scheme 3b). Since all enantiomers were separated during early stages of the process, up to 30 steps in four convergent pathways were necessary to produce racemic Nebivolol. Thus, the process of making racemic Nebivolol based on such strategy is complicated and inefficient. This invention offers a solution by enabling selective preparation of the intermediates as depicted in Scheme 6a, wherein each of the intermediates is obtained as a racemic mixture without prior resolution of enantiomers which are formed during the process.

Further, this invention offers possibility of preparing of enantiomerically pure Nebivolol, e.g., after resolution of selected racemic compounds, e.g., compound **II** (Schemes 6b and 6c).

This invention also provides a method of preparation of racemic Nebivolol, in which the formation of undesired diastereomers (e.g., SRSS/RSRR) in the final steps is reduced to a minimum by facilitating the purification and increasing the efficiency. Surprisingly, inventors have discovered that effective preparation ofNebivolol can advantageously utilize differences in solubility of other diastereomeric Nebivolol compounds as their HCl salts. Specifically, inventors observed that the racemic Nebivolol meso form (as HCl salt) having the configuration RSRS has an increased solubility as compared to the solubility of Nebivolol hydrochloride, whereas the second meso form having the configuration RRSS has a comparable solubility to that of Nebivolol hydrochloride. Solubility of the HCL-salts in MeOH: Nebivolol equals 1.5%, RRSS-meso form equals 1.0%, and RSRS-meso form is above 15%. In a preferred embodiment of this invention, a selective preparation of racemic Nebivolol is provided which may contain only a selected diastereomer (e.g., RSRS-meso form) as a possible impurity, which can be easily removed by a simple recrystallization due to the higher solubility. Therefore, a difficult and low yielding purification of the final product is avoided by this synthetic strategy because the formation of poorly soluble Nebivolol diastereomers (SRRS/RSSR, RRSS, SRSS/RSRR and RRRR/SSSS) is prevented.

Another aspect of the present invention is to provide a diastereoselective synthesis of the intermediate **VIIIa** containing the preferred syn-configuration. This compound is a useful intermediate in the above described synthetic strategy for the selective preparation of racemic Nebivolol, since it can only form as a contaminant the diastereomer having the RSRS configuration which can be easily removed in the final steps (Scheme 6a, steps 8 and 9).

Yet another aspect of the present invention is to provide an efficient method for selective reduction of compound **IXa** wherein the formation of the undesired RSRS isomer is reduced to a minimum.

Inventors have discovered that the efficiency of the process is further increased by providing recycling methods for the re-use of undesired diastereomers, which may be produced during the syntheses of the intermediates.

The method of preparing racemic Nebivolol (as shown in Scheme 6a) will now be described in detail.

The starting material for the present process is compound **II,** a racemic acid, which can be prepared by different routes according to the methods disclosed in U.S. Patent No. 5,171,865 (see also counterpart EP 0331078) and U.S. Patent No. 4,985,574 (see also counterpart EP 0264586).

Step 1 involves preparation of (±)-5-[6-fluorochroman-2-carbonyl]-2, 2-dimethyl[1, 3]dioxane-4, 6-dione (compound **III**) from compound **II** as shown in Scheme 7.

Initially, the racemic acid **II** is transformed in an activated acid derivative which is then reacted with Meldrum's acid in an organic solvent and in the presence of a base to give the corresponding acylated Meldrumate **III** as a novel compound and a useful intermediate in the synthesis of Nebivolol. The acylation of Meldrum's acid can be carried out in the manner similar to a conventional procedure, e.g., as disclosed in J. Org. Chem. 43(10), 1978, 2087.

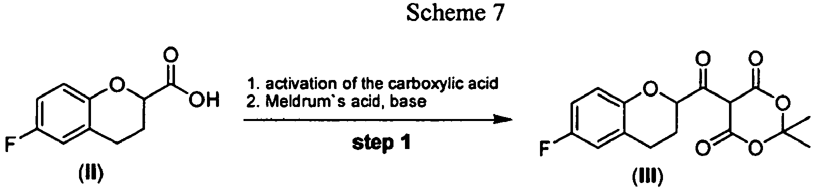

The carboxylic group can be activated in a conventional manner, e.g., as a carboxylic acide halide by using PHal₅, PHal₃, SOHal₂, (COHal)₂, as a carboxylic acid anhydride, as an activated ester, etc. Activation as an acid halide is preferred, and the chloride is the most preferred acid halide, which is prepared with 1 to 5 equivalents SOCl₂, preferably with 1-3 equivalents, in the presence of catalytical amounts of DMF. This reaction can be carried out without the use of any solvent or in a solvent such as, for example, benzene, alkyl or halogen substituted benzene, halogenated hydrocarbons, etc. Alkyl or halogen substituted benzene are preferred solvents and toluene is the most preferred solvent.

The reaction temperature can have a range from room temperature to the boiling point of the solvent. In a preferred embodiment, the reaction temperature ranges from 60°C to 90°C in toluene as the solvent. The acyl chloride can be obtained in almost quantitative yield by evaporation of the solvent together with the excess of chlorination reagent.

The acylation of Meldrum's acid can be done in the same solvent as used for the activation of the carboxylic acid. Halogenated hydrocarbons are the preferred solvents and methylene chloride is most preferred. Typically, the Meldrum's acid is used in molar proportions of 1 to 3 moles per mole of compound **II,** preferably in 1 to 1.5 moles per mole of compound **II.** The reaction is carried out in the presence of an organic or inorganic base, preferably in the presence of an organic base such as, for example, tertiary amine and most preferably in the presence of pyridine. In certain embodiments, 1 to 5 equivalents, preferably 1.5 to 3 equivalents of pyridine are used as the base. The reaction temperature may range between about -10°C (but not below the melting point of the pure solvent, e.g., benzene) and about + 30°C, preferably between 0°C and room temperature. The reaction carried out in this temperature range (0°C-RT) will be typically completed within 2 hours.

At the end of the reaction, the mixture is hydrolyzed and extracted with water or a diluted aqueous solution of an inorganic acid, preferably a 5% to 10% aqueous hydrochloride solution.

After separation of the layers, the organic solvent is evaporated and the residue may be used directly for the next step or purified by a recrystallization or by slurry in an organic solvent. Preferably, the residue is purified by slurry in an ether and most preferably in methyl tertiarybutyl ether (MTBE) or in diisopropyl ether.

If the preparation of enantiomerically pure Nebivolol is desired, it can be achieved by resolution of compound **II** using e.g. (+)-dehydroabietylamine as described in U.S. Patent No. 6,545,040.

Step 2 involves preparation of compound **IV**, Scheme 6a, wherein R is H, yielding (±)-1-(6-fluoro-chroman-2-yl)-ethanone (compound **IVa**, Scheme 8, Route A), or COOR', wherein R' is alkyl or substituted alkyl, yielding (±)-3-(6-fluorochroman-2-yl)-3-oxo-propionic acid alkyl ester (compound **IVb**, Scheme 9, Route B).

Acylated Meldrum's acids are suitable intermediates for the preparation of the corresponding methyl ketone after hydrolysis and decarboxylation, or for the preparation of the corresponding beta-keto acids after alcoholysis and decarboxylation. The reactions may be carried out in the manner similar to a conventional procedure, e.g. as disclosed in J. Org. Chem. 43(10), 1978, 2087 and Synth. Commun., 10, 1980, 221.

### Route A:

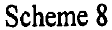

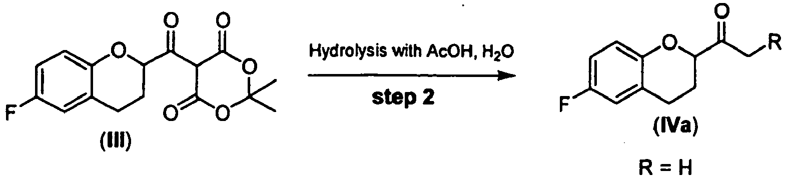

The hydrolysis and decarboxylation of compound **III** to yield compound **IVa** (Scheme 8) as a novel compound and useful intermediate for the synthesis of Nebivolol can be carried out in an aqueous acid solution at reflux temperature. A mineral acid or an organic acid may be used, wherein acetic acid is preferred. Water may be used in excess; equal volume amounts of water and acetic acid are preferred. The compound can be used directly as a crude product or may be further purified by column chromatography.

### Route B:

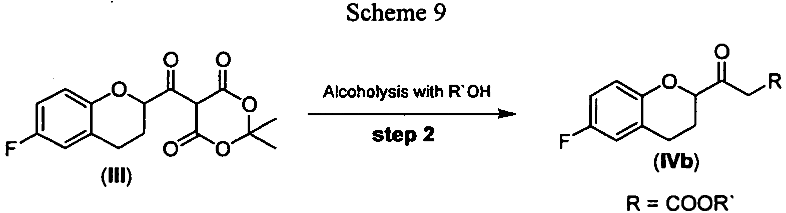

Reaction of compound **III** with alcohols gives the corresponding beta-keto ester **IVb** as a novel compound and useful intermediate for the synthesis ofNebivolol, wherein R' is alkyl or substituted alkyl. This alcoholysis can be carried out with primary, secondary or tertiary alcohols, preferably with primary and tertiary alcohols and most preferably with ethanol or *tert-*butanol. As a solvent, the alcohol itself or an inert aromatic solvent can be used. Ethanol is the preferred solvent for the synthesis of the corresponding ethyl ester and toluene is the preferred solvent for the synthesis of the *tert-*butyl ester. The reaction temperature may have a range from reflux temperature for low boiling alcohols up to reflux temperature for toluene or the reflux temperature of the corresponding toluene/alcohol azeotrope. Preferred temperatures for the preparation of beta-keto ethyl ester as well as for the beta-keto tert-butyl ester are in a range from about 70 to about 80°C. After completion of the reaction, the reaction mixture may be worked up in a usual manner, e.g., by an extractive method, and the crude product may be used directly for the next step or purified by column chromatography.

Step 3 involves preparation of compound **V** from compound **IV,** e.g., (±)-2-bromo-1-(6-fluoro-chroman-2-yl)-ethanone (compound **Va)** and (±)-2-chloro-1-(6-fluoro-chroman-2-yl)-ethanone (compound **Vb)** (Schemes 10 and 11 a-c).

The compound **IV** prepared according to step 2 can be used for the synthesis of compound **V** having a suitable leaving group (LG). Non-limiting examples of suitable leaving groups include substituted and non substituted alkyl and aryl sulfonic acid derivatives and halogen atoms. In a preferred embodiment, leaving groups are halogen atoms and the most preferred leaving groups are bromine (compound **Va)** and chlorine (compound **Vb).**

Compounds **Va** and **Vb** can be prepared via route A of Scheme 10 or route B of Schemes 11 a-c as described below.

### Route A:

The synthesis of the bromoketone (compound **Va)** by direct bromination of the methyl ketone (compound **IVa)** using bromine or NBS leads in most cases to a competitive bromination of the aromatic ring. However, after the previous conversion of the methyl ketone **IVa** to the corresponding silyl enol ether having the terminal double bond, the selective preparation of compound **Va** is possible (see Scheme 10).

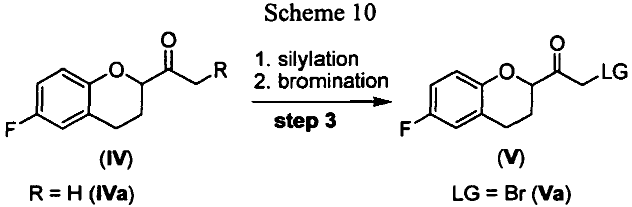

In a general procedure, the silyl enol ether can be obtained by kinetically controlled deprotonation using a strong base followed by silylation. Examples of a solvent include ethers or mixtures of ethers with such solvents, in which solutions of the strong bases are commonly available. The preferred preparation of the silyl enol ether uses lithium diisopropylamide (LDA) as the base, trimethylsilyl chloride (TMSC1) as a silylating reagent and tetrahydrofuran (THF) as the ether. The reaction starts at -78°C with the addition of the compound **IVa** to a mixture of 1 to 1.5 equivalents LDA and 1 to 2 equivalents of TMSCl. Preferred are 1.2 equivalents of LDA and 1.6 equivalents of TMSCl. After the reaction is allowed to warm to room temperature, the mixture is first worked up by an extractive method and then concentrated. The bromination may be carried out in a suitable solvent with N-bromosuccinimide (NBS), 1, 3-dibromo-5, 5-dimethylhydantoin, or pyridine hydrobromide perbromide at 0°C to room temperature. Suitable solvents include, for example, halogenated hydrocarbons, preferably, methylene chloride. After completion of the reaction, the mixture is worked up by an extractive method, and the product may be purified by column chromatography or by recrystallization. Since byproduct formation by nonselective bromination was observed, resulting in difficult purification, a more selective and efficient preparation of compound **V** was developed (see route B, Scheme 11 a).

### Route B:

Route B is an alternative preparation of the halogenated compounds Va and Vb, which gives a better selectivity than that of route A (see Scheme 11a).

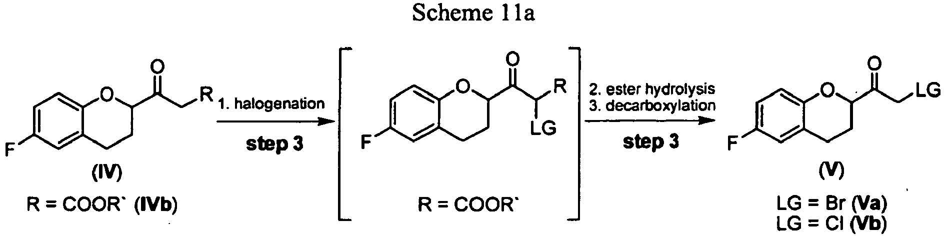

Advantageously, in route B, the reaction can be carried out at higher, more convenient temperatures by successive halogenation of the beta-keto ester **IVb**, followed by hydrolysis and decarboxylation. The halogenation can be done with a suitable halogenation reagent with or without a catalyst. Typical halogenation reagents for the preparation of the corresponding bromides or chlorides include, for example, NBS, NCS, and SO₂Cl₂. A non-limiting example of a catalyst includes Mg(ClO₄)₂. Suitable solvents for this reaction include acetonitrile, esters or halogenated hydrocarbons; acetonitrile, ethyl acetate and methylene chloride are preferred. In certain embodiments, 1.0 to 1.5 equivalents of the halogenation reagent and 0.3 equivalents of the catalyst can be used. The reaction proceeds between 0°C and room temperature to a complete conversion within 3-4 hours. Higher temperatures for the halogenation should be avoided because of possible side reactions, and lower temperature may prolong the reaction time. The following ester hydrolysis and decarboxylation to form compound **V** can be done in an aqueous or non-aqueous acid solution at higher temperatures. Mineral acids or organic acids may be used.

Compound **IVb** as the ethyl or *tert*-butyl ester may be used as the starting material, with the *tert*-butyl ester being preferred. In the case of the use of the ethyl ester, the hydrolysis and decarboxylation of the corresponding halogenated beta keto ethyl ester may be preferably carried out with an aqueous trifluoro acetic acid solution. When the *tert*-butyl ester form is used, the hydrolysis and decarboxylation of the corresponding halogenated beta keto *tert*-butyl ester is carried out preferably in a mixture of formic acid and acetic acid and preferably in the presence of water. The reaction temperature for the ester hydrolysis and decarboxylation is in a range from about 60°C to about 100°C, preferably 75-90°C. The purification of compound **V** may be done by as aforementioned for compound **Va**. Since compound **Vb** shows greater storage stability than compound **Va,** compound **Vb** is preferred.

Compound **V** having substituted or non substituted alkyl and aryl sulfonic acid derivatives as leaving groups may be prepared, e.g., by transformation of the compound **IVb'** or **IVb"** (LG = halogen) with salts of carboxylic acids to compound **IVb"'** followed first by ester hydrolysis then decarboxylation and sulfonylation using the corresponding alkyl or aryl sulfonic acid chlorides (see Scheme 11b). Alernatively, compound **Vc** may be prepared by similar transformation starting directly with the haloketones **Va** or **Vb** (see Scheme 11c)

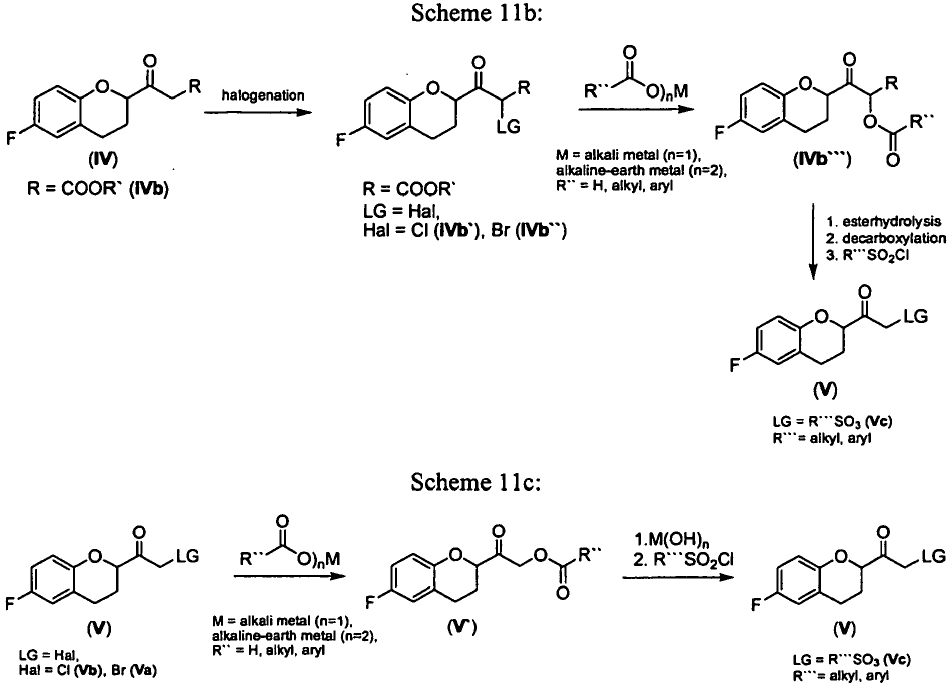

Step 4 involves preparation of compound **VI** as shown in Scheme 12 below. Non-limiting examples of compound **VI** include (±)-2-chloro-1-[6-fluoro-(2R*)-chroman-2-yl]-(1R*)-ethan-1-ol (intermediate **VIa**), (±)-2-chloro-1-[6-fluoro-(2R*)-chroman-2-yl]-(1S*)-ethan-1-ol (intermediate **VIb**), (±)-2-bromo-1-[6-fluoro-(2R*)-chroman-2-yl]-(1R*)-ethan-1-ol (intermediate **VIc),** and (±)-2-bromo-1-[6-fluoro-(2R*)-chroman-2-yl]-(1S*)-ethan-1-ol (intermediate **VId).**

A variety of reducing agents can be used for the preparation of the halomethyl alcohols (compound **VI**) from the racemic halomethyl ketones (compound **V**) (see Scheme 12). In general, two racemic diastereomers can be formed having the syn (RR/SS) or the anti (RS/SR) configuration. With regard to the Nebivolol synthetic strategy as depicted in Scheme 6(a), the reduction methods giving the syn configured (RR/SS) halomethyl alcohol in excess are preferred. Surprisingly, there are only a few investigations for the selective reduction of halomethyl ketones having an alkoxy substituted chiral center in the alpha position (e.g., Tetrahedron Letters 40 (1999), 2863-2864.) Prior to this invention, it was not appreciated to utilize the influence of an alkoxy substituted chiral center in the alpha position of halomethylketones on the formation of a new chiral center and the diastereoselectivity for such reactions is not well established).

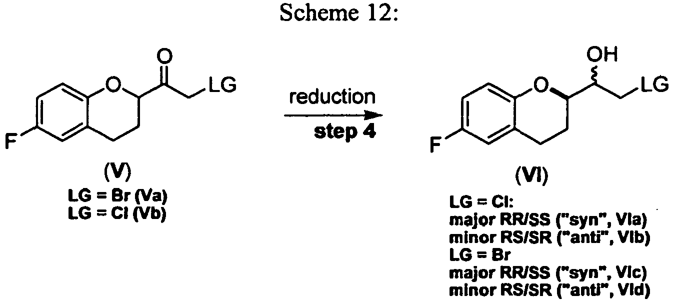

In general, there is no limitation on the use of reduction agents, e.g., borohydride or aluminiumhydride reduction reagents as well as the reagents that are useful for Meerwein Pondorf Verley reductions. Non-limiting examples of reduction agents include LiBH₄, NaBH₄, KBH₄N(nBu)₄BH₄, Zn(BH₄)₂, NaH(Oac)₃, Superhydride^{®}, Red-Al, Li-Selectride, BH₃xSMe₂ or the like. In case of catalytic hydrogenation, suitable catalysts are catalysts that do not give side reactions with halogenated compounds (e.g., the catalyst as disclosed and cited in WO 03/064357). The reduction may be carried out in the absence or in the presence of a Lewis acid, such as, for example, MgCl₂, CaCl₂, BaCl₂, ZnCl₂ Al(Oalkyl)₃, Ti(Oalkyl)₄ BF₃xOEt₂ and the like. Suitable solvents include ethers, alcohols, halogenated hydrocarbons, halogenated or alkylated aromatic solvents and the like, with the exception, that halogenated solvents are unsuited for catalytic reductions. Preferred halomethyl ketones of compound **V** bear chlorine or bromine as the substituent "LG". The reduction is conveniently carried out at temperatures between about -78°C and about room temperature, preferably between -20°C and room temperature. Table 2 shows representative results for the reduction of chloromethyl ketone **Vb** (LG = Cl).

**Table 2:**

| Reagent (eq.) | Catalyst (eq.) | Solvent | Temperature [°C] | Time [h] | Ratio RR/SS // RS/SR |
|---|---|---|---|---|---|
| LiBH₄ (1) | none | THF | -20 to -15 | 1 | 58.3 // 41.7 |
| LiBH₄ (1) | none | MeOH | -20 to -15 | 1 | 60.2 // 39.8 |
| LiBH₄ (1) | none | iPrOH | -20 to -15 | 1 | 51.0 // 49.0 |
| LiBH₄ (1) | none | CH₂Cl₂ | -20 to -15 | 3 | 42.8 // 57.2 |
| LiBH₄ (1) | none | toluene | -20 to RT | over night | 41.5 // 58.5 |
| LiBH₄ (1) | none | DME | -20 to -15 | 1 | 53.8 // 46.2 |
| LiBH₄ (1) | ZnCl₂ (2) | THF | -20 to -15 | 1 | 56.9 // 43.1 |
| LiBH₄ (1) | ZnCl₂ (2) | MeOH | -20 to -15 | 1 | 63.7 // 36.3 |
| LiBH₄ (1) | ZnCl₂ (2) | iPrOH | -20 to -15 | 1 | 60.3 // 39.7 |
| LiBH₄ (1) | ZnCl₂ (2) | CH₂Cl₂ | -20 to RT | over night | 59.3 // 40.7 |
| LiBR₄ (1) | ZnCl₂ (2) | toluene | -20 to RT | over night | 53.0 // 47.0 |
| LiBH₄ (1) | ZnCl₂ (2) | DME | -20 to -15 | 2 | 46.7 // 53.3 |
| LiBH₄ (1) | Ti(OiPr)₄ (2) | THF | -20 to -15 | 1 | 48.8 // 51.2 |
| LiBH₄ (1) | Ti(OiPr)₄ (2) | MeOH | -20 to -15 | 1 | 59.3 // 40.7 |
| LiBH₄ (1) | Ti(OiPr)₄ (2) | iPrOH | -20 to -15 | 1 | 47.3 // 52.7 |
| LiBH₄ (1) | Ti(OiPr)₄ (2) | CH₂Cl₂ | -20 to -15 | 1 | 35.6 // 64.4 |
| LiBH₄ (1) | Ti(OiPr)₄ (2) | Toluene | -20 to -15 | 1 | 38.1 // 61.9 |
| LiBH₄ (1) | Ti(OiPr)₄ (2) | DME | -20 to -15 | 1 | 43.4 // 56.6 |
| LiBH₄ (1) | MgCl₂ (2) | THF | -20 to -15 | 3 | 57.8 // 42.2 |
| LiBH₄ (1) | MgCl₂ (2) | MeOH | -20 to -15 | 1.5 | 59.5 // 40.5 |
| LiBH₄ (1) | MgCl₂ (2) | iPrOH | -20 to -15 | 1.5 | 41.1 // 58.9 |
| LiBH₄ (1) | MgCl₂ (2) | CH₂Cl₂ | -20 to -15 | 2.5 | 44.3 // 55.7 |
| LiBH₄ (1) | MgCl₂ (2) | Toluene | -20 to -15 | 2.5 | 42.9 // 57.1 |
| LiBH₄ (1) | MgCl₂ (2) | DME | -20 to -15 | 1 | 52.4 // 47.6 |
| LiBH₄ (1) | Al(OiPr)₃ (2) | THF | -20 to -15 | 1 | 54.7 // 45.3 |
| LiBH₄ (1) | Al(OiPr)₃ (2) | MeOH | -20 to -15 | 1 | 59.7 // 40.3 |
| LiBH₄ (1) | Al(OiPr)₃ (2) | iPrOH | -20 to -15 | 1 | 50.5 // 49.5 |
| LiBH₄ (1) | Al(OiPr)₃ (2) | CH₂Cl₂ | -20 to -15 | 1 | 41.9 // 58.1 |
| LiBH₄ (1) | Al(OiPr)₃ (2) | Toluene | -20 to -15 | 1 | 39.6 // 60.4 |
| LiBH₄ (1) | Al(OiPr)₃ (2) | DME | -20 to -15 | 1 | 51.1 // 48.9 |
| LiBH₄ (1) | BF₃xOEt₂ (2) | THF | -20 to RT | over night | 61.5 // 38.5* |
| LiBH₄ (1) | BF₃xOEt₂ (2) | MeOH | -20 to RT | over night | 56.3 // 43.7* |
| LiBH₄ (1) | BF₃xOEt₂ (2) | iPrOH | -20 to RT | over night | 55.4 // 44.6* |
| LiBH₄ (1) | BF₃xOEt₂ (2) | CH₂Cl₂ | -20 to -15 | 3 | 44.9 // 55.1 |
| LiBH₄ (1) | BF₃xOEt₂ (2) | Toluene | -20 to RT | over night | 45.6 // 54.6* |
| LiBH₄ (1) | BF₃xOEt₂ (2) | DME | -20 to -15 | 1 | 46.0 // 54.0 |
| NaBH₄ (1) | none | THF | -20 to -15 | 1 | 51.8 // 48.2 |
| NaBH₄ (1) | none | MeOH | -20 to -15 | 1 | 60.2 // 39.8 |
| NaBH₄ (1) | none | iPrOH | -20 to RT | 22.25 | 59.1 // 40.9 |
| NaBH₄ (1) | none | CH₂Cl₂ | -20 to RT | 21 | 50.9 // 49.1* |
| NaBH₄ (1) | none | Toluene | -20 to RT | 21 | 51.7 // 48.3* |
| NaBH₄ (1) | none | DME | -20 to -15 | 1 | 54.7 // 45.3 |
| NaBH₄ (1) | none | EtOH | -78°C to RT | 3 | 56.8 // 43.2 |
| NaBH₄ (0.5) | none | iPrOH | RT | 0.5 | 50.4 // 49.6 |
| NaBH₄ (1) | ZnCl₂ (2) | THF | -20 to -15 | 1 | 58.3 // 41.7 |
| NaBH₄ (1) | ZnCl₂ (2) | MeOH | -20 to -15 | 1 | 63.4 // 36.6 |
| NaBH₄ (1) | ZnCl₂ (2) | iPrOH | -20 to RT | 18 | 59.7 // 40.3 |
| NaBH₄ (1) | ZnCl₂ (2) | CH₂Cl₂ | -20 to RT | 21 | 63.8 // 36.2* |
| NaBH₄ (1) | ZnCl₂ (2) | Toluene | -20 to RT | 21 | 61.5 // 38.5* |
| NaBH₄ (1) | ZnCl₂ (2) | DME | -20 to -15 | 2 | 49.3 // 50.7 |
| NaBH₄ (1) | Ti(OiPr)₄ (2) | THF | -20 to -15 | 1 | 42.8 // 57.2 |
| NaBH₄ (1) | Ti(OiPr)₄ (2) | MeOH | -20 to -15 | 1 | 58.8 // 41.2 |
| NaBH₄ (1) | Ti(OiPr)₄ (2) | iPrOH | -20 to -15 | 3 | 46.4 // 63.6 |
| NaBH₄ (1) | Ti(OiPr)₄ (2) | CH₂Cl₂ | -20 to RT | 18 | 38.4 // 61.6 |
| NaBH₄ (1) | Ti(OiPr)₄ (2) | Toluene | -20 to RT | 18 | 41.4 // 58.6 |
| NaBH₄ (1) | Ti(OiPr)₄ (2) | DME | -20 to -15 | 1 | 44.9 // 55.7 |
| NaBH₄ (1) | MgCl₂ (2) | THF | -20 to RT | 18 | 44.4 // 55.6* |
| NaBH₄ (1) | MgCl₂ (2) | MeOH | -20 to -15 | 2 | 58.1 // 41.99 |
| NaBH₄ (1) | MgCl₂ (2) | iPrOH | -20 to -15 | 2 | 53.4 // 46.6 |
| NaBH₄ (1) | MgCl₂ (2) | CH₂Ch | -20 to RT | 18 | 58.2 // 41.8* |
| NaBH₄ (1) | MgCl₂ (2) | Toluene | -20 to RT | 18 | 47.9 // 52.1 |
| NaBH₄ (1) | MgCl₂ (2) | DME | -20 to -15 | 1 | 47.8 // 52.2 |
| NaBH₄ (1) | Al(OiPr)₃ (2) | THF | -20 to RT | | 46.5 // 53.5 |
| NaBH₄ (1) | Al(OiPr)₃ (2) | MeOH | -20 to -15 | 1 | 59.6 // 40.4 |
| NaBH₄ (1) | Al(OiPr)₃ (2) | iPrOH | -20 to -15 | 3 | 48.6 // 51.4 |
| NaBH₄ (1) | Al(OiPr)₃ (2) | CH₂Cl₂ | -20 to -15 | 3 | 55.0 // 45.0* |
| NaBH₄ (1) | Al(OiPr)₃ (2) | Toluene | -20 to -15 | 3 | 52.0 // 48.0* |
| NaBH₄ (1) | Al(OiPr)₃ (2) | DME | -20 to -15 | 1 | 50.4 // 49.6 |
| NaBH₄ (1) | BF₃xOEt₂ (2) | THF | -20 to RT | 20 | 52.3 // 47.7* |
| NaBH₄ (1) | BF₃xOEt₂ (2) | MeOH | -20 to RT | 20 | 57.0 // 43.0* |
| NaBH₄ (1) | BF₃xOEt₂ (2) | iPrOH | -20 to RT | 20 | 53.4 // 46.6* |
| NaBH₄ (1) | BF₃xOEt₂ (2) | CH₂Cl₂ | -20 to RT | 20 | 60.0 // 40.0* |
| NaBH₄ (1) | BF₃xOEt₂ (2) | Toluene | -20 to RT | 20 | 61.5 // 38.5* |
| NaBH₄ (1) | BF₃xOEt₂ (2) | DME | -20 to -15 | 1 | 41.7 // 58.3 |
| NaBH₄ (1) | MgCl₂ (2) | MeOH | 0 to 5 | 0.5 | 54.0 // 46.0 |
| NaBH₄ (1) | MgCl₂ (2) | MeOH | -78 to RT | 0.5 | 65.9 // 34.1 |
| NaBH₄ (1) | MgCl₂ (2) | THF | 0 to RT | 22 | 41.0 // 59.0 |
| NaBH₄ (1) | CaCl₂ (2) | MeOH | 0 to 5 | 0.5 | 47.0 // 53.0 |
| NaBH₄ (1) | BaCl₂ (2) | MeOH | 0 to 5 | 0.5 | 50.0 // 50.0 |
| NaBR₄ (11.1) | CeCl₃ (2) | MeOH | 0 to 5 | 2 | 36.9 // 63.1 |
| NaBH₄ (1) | ZnCl₂ (2) | MeOH | 0 to 5 | 0.5 | 61.0 // 39.0 |
| NaBH₄ (1) | ZnCl₂ (2) | MeOH | -78 to RT | 2 | 64.7 // 35.5 |
| NaBH₄ (1) | ZnCl₂ (2) | THF | -10 | 0.25 | 57.0 // 43.0 |
| NaBH₄ (1) | ZnCl₂ (2) | Et₂O | -10 | 0.25 | 56.0 // 44.0 |
| NaBH₄ (1) | ZnCl₂ (2) | DME | -10 | 0.25 | 50.0 // 50.0 |
| NaBH₄ (1) | ZnCl₂ (2) | EtOH | -20 to -15 | 1 | 64.0 // 36.0 |
| NaBH₄ (1) | ZnCl₂(1.5) | EtOH | -20 to -15 | 1.5 | 64.2 // 35.8 |
| NaBH₄ (1) | ZnCl₂(1.0) | EtOH | -20 to -15 | 1.5 | 64.7 // 35.3 |
| NaBH₄ (1) | ZnCl₂(0.5) | EtOH | -20 to -15 | 1.5 | 64.9 // 35.1 |
| NaBH₄ (1) | ZnCl₂(0.3) | EtOH | -20 to -15 | 1.5 | 63.8 // 36.2 |
| NaBH₄ (1) | ZnCl₂(0.2) | EtOH | -20 to -15 | 1.5 | 63.2 // 36.8 |
| NaBH₄ (1) | ZnCl₂(0.1) | EtOH | -20 to -15 | 1.5 | 63.0 // 37.0 |
| NaBH₄ (1) | none | EtOH | -20 to -15 | 1.0 | 54.7 // 45.3 |
| Bu₄NH₄ (1) | ZnCl₂ (2) | MeOH | -20 to -15 | 1 | 63.9 // 36.1 |
| Bu₄NH₄ (1) | Al(OiPr)₃ (2) | MeOH | -20 to -15 | 1 | 60.5 // 39.5 |
| Bu₄NH₄ (1) | ZnCl₂ (2) | EtOH | -20 to -15 | 1 | 62.7/37.3 |
| Zn(BH₄)₂ (1.0) | none | Et₂O | -78 to RT | 3 | 64.9 // 34.1 |
| Zn(BH₄)₂ (1.4) | none | Et₂O | -78 | 0.5 | 59.1 // 40.9 |
| Zn(BH₄)₂ (1.4) | none | THF/ Et₂O (1/1) | -78 | 0.5 | 59.4 // 40.6 |
| Zn(BH₄)₂ (1.4) | none | THF/ Et₂O (1/2) | -78 | 0.5 | 58.4 // 41.6 |
| Zn(BH₄)₂(1.4) | none | THF/ Et₂O (2/1) | -78 | 0.5 | 62.2 // 37.8 |
| Bu₄NBH₄ (1.1) | none | THF | -78 | 2 | 59.8 // 40.2 |
| Bu₄NBH₄ (1.3) | none | CH₂Ch | -78 | 2 | 39.2 // 60.8 |
| Bu₄NBH₄ (1.1) | none | THF/CH₂Cl₂(1/1) | -78 | 2 | 66.7 // 33.3 |
| Bu₄NBH₄ (1.1) | none | THF/CH₂Cl₂ (1/1) | 0 | 2 | 52.2 // 47.8 |
| Bu₄NBH₄ (1.1) | ZnCl₂ (2) | THF/CH₂Cl₂ (1/1) | -78 | 2 | 60.7 // 39.3 |
| NaBH(Oac)₃ (2) | ZnCl₂ (2) | THF/ Et₂O (2/1) | 0 to RT | 22 | 52.2 // 47.8* |
| BH₃xSMe₂ (1.1) | none | THF/CH₂Cl₂ (1/1) | -78 | 2 | 57.7 // 42.3 |
| BH₃xSMe₂ (1.1) | ZnCl₂ (2) | THF/CH₂Cl₂ (1/1) | -78 | 2 | 61.8 // 38.2 |
| Red-Al (1.3) | none | CH₂Cl₂/Toluene(1/1) | -78 | 3 | 52.9 // 48.0 |
| Red-Al (1.3) | ZnCl₂ (2) | CH₂Cl₂/Toluene(1/1) | -78 | 3 | 54.1 // 45.9* |
| Superhydride® (1.1) | none | THF/CH₂Cl₂ (1/1) | -78 | 2 | 35.5 // 64.4 |
| Superhydride® (1.1) | ZnCl₂ (2) | THF/CH₂Cl₂ (1/1) | -78 | 2 | 37.4 // 62.6 |
| DIBAH (1.1) | none | THF/CH₂Cl₂ (1/1) | -78 | 2 | 60.1 // 39.9* |
| Li(tBuO)₃AlH | none | THF/CH₂Cl₂ (1/1) | -78 | 2 | 44.4 // 55.6 |
| Li(tBuO)₃AlH | ZnCl₂ (2) | THF/CH₂Cl₂(1/1) | -78 | 2 | 56.6 // 43.4 |
| Al(OiPr)₃ (0.5) iPrOH (7.0) | CH₃SO₃H (0.5) | Toluene | 35-45 | 1.5 | 27.9 // 72.1 |
| Al(OiPr)₃ (0.5) BINAPHTHOL iPrOH (5.0) | none | Toluene | 20-25 | 16 | 25.6 // 74.4 |
| DIBAH (2.0) Acetone (2.0) | none | THF | 20-25 | 16 | 27.6 // 72.4 |
| DIBAH (2.0) Acetone (2.0) | none | THF/Toluene (1/1) | 20-25 | 16 | 27.6 // 72.4 |
| Al(OtBu)₃ (0.5) D/L-Phenethyl-alkohol (2.0) | none | Toluene | 20-25 | 2 | 28.0 // 72.0 |
| Al(OtBu)₃ (0.1) D/L-Phenethyl-alkohol (2.0) | none | Toluene | 20-25 | 6 | 28.0 // 72.0 |
| Al(OsBu)₃ (0.5) sBuOH (7.0) | CH₃SO₃H (0.5) | Toluene | 20-25 | 1 | 25.7 // 74.3 |
| Al(OiPr)₃ (0.5) Cyclohexanol (7.0) | CH₃SO₃H (0.5) | Toluene | 20-25 | 5 | 19.6 // 80.4 |
| Al(OtBu)₃ (0.5) Cyclohexanol (7.0) | CH₃SO₃H (0.5) | Toluene | 20-25 | 3 | 18.4 // 81.6 |
| Al(OtBu)₃ (0.5) Cyclohexanol (7.0) | CH₃SO₃H (0.5) | Toluene | 0-5 | 6 | 14.5 // 85.5 |
| Al(OtBu)₃ (0.5) 3-Pentanol(7.0) | CH₃SO₃H (0.5) | Toluene | 0-5 | 3 | 18.0 // 82.0 |
| Al(OtBu)₃ (0.5) 9-Hydroxyfluoren (3.4) | CH₃SO₃H (0.5) | Toluene | 0-5 | 4.5 | 27.1 // 72.9 |
| Al(OtBu)₃ (0.5) Diphenylcarbinol (7.0) | CH₃SO₃H (0.5) | Toluene | 0-RT | 31 | 11.7 // 88.3 |

| | | | | | |
|---|---|---|---|---|---|
| In Table 2, incomplete conversion is marked with the symbol "*." | | | | | |

The ratio of diastereomeric configurations RR/SS to RS/SR is from about 0.3 to about 2, preferably 1.1 to 2, and most preferably 1.2 to 2.

It was observed that diastereoselective reductions at temperatures above -20°C give the best ratio for the diastereomer (compound VIa, syn configuration RR/SS) when the reaction is carried out e.g. with NaBH₄ in MeOH or EtOH in the presence of a catalyst e.g., ZnCl 2 (0.1-2.0 equivalents). Formation of the diastereomer having the anti configuration (compound VIb, RS/SR) is favored by using the Meerwein Pondorf Verley reduction. In this case, the ratio of RS/SR to RR/SS is up to 9.

After the almost complete conversion, the reaction can be worked up in a manner known in the art, by concentrating the reaction mixture and dissolving the residue in a water immiscible solvent, preferably toluene or MTBE, followed by successive washing with an aqueous acid solution, preferably 2N HCl solution followed by washing with water and/or an alkaline solution, preferably NaHCO₃ solution. The diastereomeric product mixture may be purified by column chromatography or used directly for the next step.

It was observed that in contrast to the reduction of compound **Vb**, the reduction of compound **Va** proceeds with partial cyclization to the corresponding epoxides (compounds **VIIa** and **VIIb,** see below).

Step 5 involves preparation of compound **VII** from compound **VI** as shown in Scheme 13. Non-limiting examples of compound **VII** include ±)-6-fluoro-[(2R*)-oxiran-2-yl]-(2S*)-chromane (compound **VIIa)** and (±)-6-fluoro-[(2R*)-oxiran-2-yl]-(2R*)-chromane (compound **VIIb).**

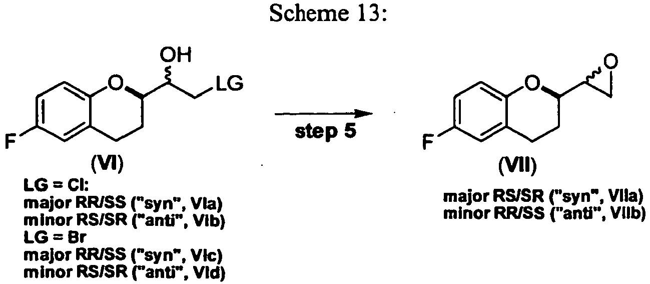

Formation of the epoxides of compound **VII** from the halomethyl alcohols of compound **VI** is conveniently carried out in solvents such as, for example, ethers or alcohols with a base such as, for example, alkali or alkaline earth metal hydroxide, alkali or alkaline earth metal alkoxides, alkali or alkaline earth metal carbonates, tertiary amines or alkali hydrides. The use of alkali alkoxides in alcohols as the solvent is preferred, and most preferred is the use of sodium methoxide in methanol as the solvent. The temperature for this reaction may range between about 0°C and about 40°C, preferably between 15°C and 25°C. About 1.0 to about 2.0 equivalents of the base may be used and 1.1 equivalents are preferred. Upon completion of the reaction, the excess base is typically neutralized by addition of an acid, preferably acetic acid. The mixture is then concentrated, and the residue is dissolved in a suitable solvent, e.g., an ether or a halogenated hydrocarbon. Washing this solution off with a half saturated aqueous sodium chloride solution and concentration of the organic layer gives epoxides of compound **VII.** If the reaction is carried out using a mixture of diastereomeric halomethyl alcohols of formula **VI,** then the corresponding diastereomeric epoxide mixture of formula **VII** will be formed. The diastereomeric mixture of epoxides may be used directly for the next step or separated by column chromatography. In a preferred embodiment of the process, the mixture is used for the next step without separation of the diastereomers.

Step 6 involves preparation of compound **VIII** from compound **VII** as shown in Scheme 14 and separation of the diastereomers. Non-limiting examples of compound **VIII** include (±)-2-Benzylamino-1-[6-fluoro-(2R*)-chroman-2-yl]-(1S*)-ethan-1-ol (compound **VIIIa**) and (±)-2-Benzylamino-1-[6-fluoro-(2R*)-chroman-2-yl]-(1R*)-ethan-1-ol (compound **VIIIb**).

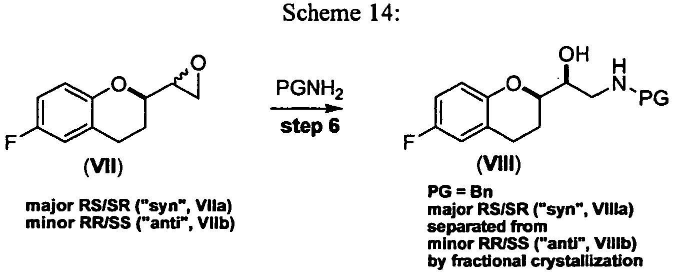

Examples of suitable protective groups (PG) include hydrogen or a suitable amine protecting group. Preferred are protecting groups which permit the subsequent alkylation step (step 7) and can be easily removed in the final step by a simple deprotection method. Therefore, preferred PG is an allyl group, a substituted or unsubstituted arylmethyl group. If PG is a benzyl group, then the preparation of compounds **VIII** may be carried out in the same manner as described in EP 0145067 but without any prior separation of the diastereomeric mixture of compounds **VIIa** and **VIIb**. Advantageously, inventors have discovered that a chromatographic separation of the oily compounds **VIIa** and **VIIb** is not necessary in this method, and that the diastereomeric mixture can be separated by fractional crystallization after conversion to VIIIa and VIIIb. Therefore, in a preferred embodiment of the procedure, a mixture of **VIIa** and **VIIb** is reacted with benzylamine to give the corresponding diastereomeric mixture of the compounds **VIIIa** and **VIIIb**, which is further separated by fractional crystallization. Since compounds **VIIIa** and **VIIIb** have basic properties, the fractional crystallization may be carried out not only with the free amine but also with an appropriate salt. Compound **VIIIa** as well as compound **VIIIb** are useful intermediates for the preparation ofNebivolol and therefore this method can be used for the selective preparation of both isomers in a commercial scale process.

With regard to the present strategy for the preparation of Nebivolol, the selective preparation and isolation of compound **VIIIa** is most preferred.

In a typical preparation, an equimolar or an enriched diastereomeric mixture of compounds **VHa** (syn) and **VHb** (anti) at ratio syn/anti of more or equal 1, produced according to steps 4 and 5, is treated with an excess benzylamine (≥ 3 equivalents) in a C₁-C₃- alcohol as the solvent at temperatures ranging from about room temperature to about 50°C. In a preferred embodiment, the reaction is carried out at 40°C with 3 equivalents of benzylamine in 2-propanol.

After complete conversion, the reaction mixture is cooled to initiate the crystallization. It was found that the preferred reaction solvent is suitable for the fractional crystallization. Additional crops may be obtained by further crystallization of the concentrated mother liquors from the same alcohol or mixture of this alcohol with ethers, preferably diisopropyl ether. Further enrichment of the diastereomeric ratio with regard to compound **VIIIa** can be obtained by recrystallization or by slurry in C₁-C₃- alcohols, ethers, toluene, acetonitrile or mixtures thereof.

Beginning with a diastereomeric mixture of compounds **VIIa** and **VIIb** at a ratio of about 57/43, the compound **VIIIa** could be obtained according to the aforementioned procedure containing 5% or less of compound **VIIIb.**

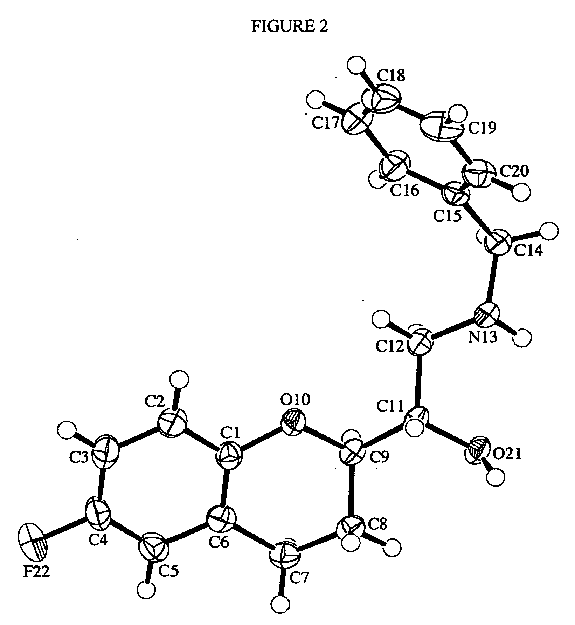

The relative configuration of the preferred compound **VIIIa** (see Figure 2) was confirmed by single X-ray measurement as shown in Table 3 below.

**Table 3: Crystallographic data of compound VIIIa**

| | |
|---|---|
| Crystallized from | diisopropyl ether / CH₂Cl₂ |
| Empirical formula | C₁₈H₂₀FNO₂ |
| Formula weight [g mol⁻¹] | 301.36 |
| Crystal colour, habit | colorless, prism |
| Crystal dimensions [mm] | 0.15 x 0.20 x 0.25 |
| Temperature [K] | 160(1) |
| Crystal system | monoclinic |
| Space group | P2₁/*c* (#14) |
| Z | 4 |
| Reflections for cell determination | 4489 |
| 2*θ*range for cell determination [°] | 4-60 |
| Unit cell parameters *a* [Å] | 4.5882(1) |
| *b* [Å] | 26.3162(5) |
| *c* [Å] | 12.4357(3) |
| *α* [°] | 90 |
| *β* [°] | 92.288(1) |
| *γ* [°] | 90 |
| *V* [Å³] | 1500.34(6) |
| *F*(000) | 640 |
| *Dₓ* [g cm⁻³] | 1.334 |
| µ(Mo *K*α) [mm⁻¹] | 0.0947 |
| Scan type | *φ* and *ω* |
| 2θ(ₘₐₓ) [°] | 60 |
| Total reflections measured | 35736 |
| Symmetry independent reflections | 4388 |
| *R*ᵢₙₜ | 0.061 |
| Reflections with *I*> 2σ(*I*) | 3005 |
| Reflections used in refinement | 4386 |
| Parameters refined | 208 |
| Final *R*(*F*) [*I* > 2σ(*I*) reflections] | 0.0480 |
| *wR*(*F*²) (all data) | 0.1162 |
| Weights: | *w* = [σ²(*F*ₒ²) + (0.0419*P*)² + 0.2604*P*]⁻¹ where P = (*F*ₒ² + 2*F*_{c}²)/3 |
| Goodness of fit | 1.036 |
| Secondary extinction coefficient | 0.012(2) |
| Final *Δₘₐₓ*/*σ* | 0.001 |
| *Δρ*(max; min) [e Å⁻³] | 0.24; -0.19 |
| *σ*(*d*_{(*C-C*)}) [A] | 0.002 |

Step 7 involves preparation of compound **IX** from compounds **VIII** and **V** and separation of diastereomers of compound **IX.** Non-limiting examples of compound **IX** include (±)-2-{Benzyl-[2-(6-fluoro-(2R*)-chroman-2-yl)-(2S*)-hydroxy-ethyl]-amino}-1-(6-fluoro-(2S*)-chroman-2-yl)-ethanone (compound **IXa**) (Scheme 15) and (±)-2-{Benzyl-[2-(6-fluoro-(2R*)-chroman-2-yl)-(2S*)-hydroxy-ethyl]-amino}-1-(6-fluoro-(2R*)-chroman-2-yl)-ethanone (compound **IXb**) (see Scheme 17).

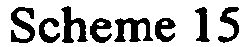

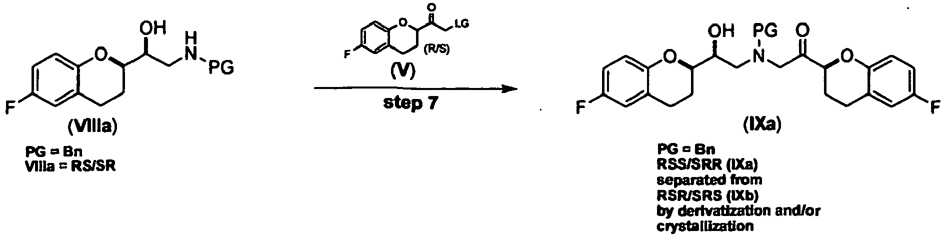

Reaction of the preferred racemic diastereomer of compuond **VIIIa** with the racemic compound **V**, having an appropriate reactive leaving group (LG), gives a diastereomeric mixture of the new compounds **IXa** and **IXb**. This mixture may be separated by column chromatography or by fractional crystallization to obtain the desired compound **IXa** as a suitable intermediate for the synthesis of racemic Nebivolol. Since the compounds **IXa** and **IXb** have basic properties, the fractional crystallization may be carried out using the free amine or an appropriate salt.

As described in Step 3 above, suitable leaving groups (LG) of compound **V** include halogen, alkylsulfonyloxy groups, substituted or unsubstituted arylsulfonyloxy groups or the like. Preferred leaving groups are halogen and the most preferred leaving groups are bromine (compound **Va**) and chlorine (compound **Vb**). Protecting groups (PG) are described in Step 6; a benzyl group is the most preferred protecting group.

The alkylation reactions are conveniently carried out in a suitable inert organic solvent or mixture of such solvents and in the presence of a base and a suitable amount of a catalyst. Exemplary solvents include substituted or halogenated hydrocarbons such as methylene chloride, dichloroethane etc.; ether, e.g., THF, dioxane, dimethoxyethane and polar aprotic solvents, e.g., DMF, DMA, NMP, DMSO and the like. Preferred solvents are polar aprotic solvents. 1.0-1.5 equivalents of compound V may be used, and 1.1 equivalents are preferred. Exemplary bases include tertiary amines, e.g., triethylamine, pyridine, alkali metal carbonate, alkali metal hydrogen carbonate or sodium hydride. Preferred bases are alkali metal hydrogen carbonates and most preferred is sodium hydrogen carbonate. 1.5-2.5 equivalents of the base may be used, and 2.0 equivalents are preferred. Exemplary catalysts for acceleration of the reaction include alkali metal halides or tetraalkylammonium halides. If the leaving group is chloride, then the corresponding bromides or iodides are preferred and most preferably, sodium bromide or sodium iodide. At least 0.1 equivalents of the catalyst may be used and 0.15 equivalents are preferred. In a preferred embodiment, the reaction may be carried out at temperatures between about room temperature and about 80°C. Lower temperatures may prolong the reaction time and higher temperatures may cause side reactions. After the reaction is completed, the mixture can be worked up by an extractive method as known in the art. Evaporation of the solvent after the extraction and crystallization of the diastereomeric mixture by using a suitable anti-solvent delivers the diastereomeric compounds **IXa** and **IXb,** which may be separated by column chromatography or by fractional crystallization from a suitable solvent. Since the compounds **IXa** and **IXb** have basic properties, the fractional crystallization may be carried out with the free amine mixture or with appropriate salts. Advantageously, the separation of a mixture of compounds **IXa** and **IXb** could be effected by fractional crystallization of the free amines from acetonitrile as solvent.

Suitable derivatization of compounds **IXa** and **IXb** may also be useful for separation of the diastereomeric mixture. Surprisingly, the inventors have discovered that the diastereomer **IXb** can be selectively silylated in the presence of the diastereomer **IXa**. Because of the higher solubility of the silylated compound **IXb** compared with the non-silylated compounds, the efficiency of the fractional crystallization can be significantly increased. In general, the silylation may be carried out in organic solvents or mixture of solvents such as ethers, esters, halogenated hydrocarbons, aromatic solvents (e.g., toluene, chlorobenzene, etc.), polar aprotic solvents (e.g., DMF, DMSO) with a silylating reagent, if necessary in the presence of a base. Preferred organic solvents include acetonitrile, THF and MTBE and mixtures thereof. If bases are necessary, then amines, e.g., triethylamine, pyridine, imidazole, etc., alkali metal carbonate or alkali metal hydrogen carbonate may be used. Amines are the preferred organic solvents and the most preferred is imidazole. 1.0-2.0 equivalents of the base may be used, and 1.5 equivalents are preferred. As a silylating reagent, TMSC1, HMDS, BSU, etc. may be used, but TMSC1 is preferred. For a successful separation of the diastereomers, it is important to use the silylating reagent in molar ratios of 0.40/n to 0.60/n with regard to the total amount of both diastereomers, wherein n is the possible amount of transferred silyl groups per silylating reagent. Fewer equivalents may give an insufficient separation and more equivalents may result in the loss of a yield. The reaction is typically carried out in a temperature range between about 0°C and about room temperature. Lower temperature may prolong the reaction time and higher temperatures may cause an insufficient selectivity.

With regard to the present strategy for the preparation of Nebivolol, the isolation of compound **IXa** from a mixture consisting of **IXa** and **IXb** by fractional crystallization or by selective derivatization of **IXb** followed by crystallization of **IXa** is preferred. The term "substantially pure diastereomers" as used herein means at least 50% pure, preferably 80 %pure and more preferably 95%pure.

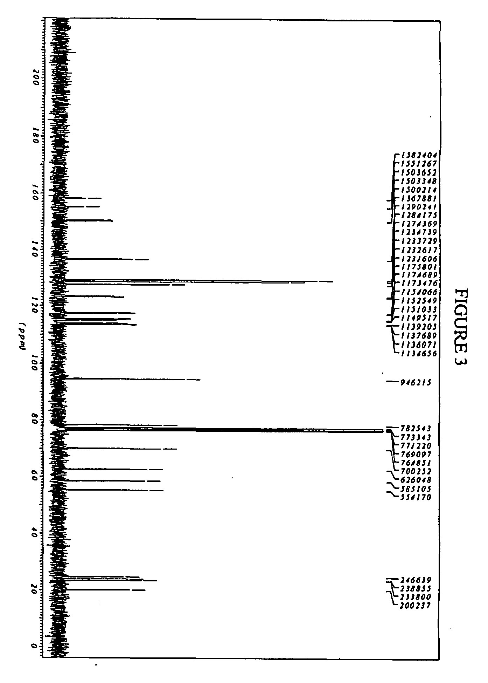

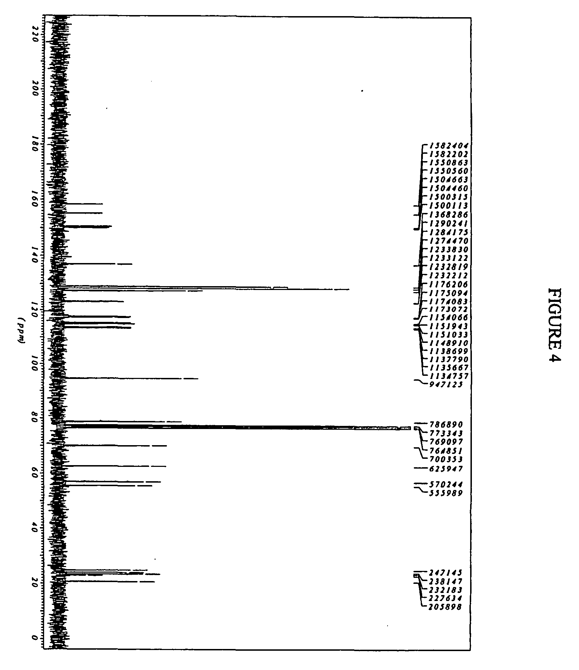

NMR measurements of the separated diastereomers have indicated that both isomers cyclize to a semi-ketal form. ¹³C-NMR spectra showed that instead of the carbonyl peaks, new peaks at 94.616 and 94.707 respectively were present, which indicated a carbon atom of the cyclic semi-ketal form (see Schemes 16 and 17 and Figures 3 and 4).

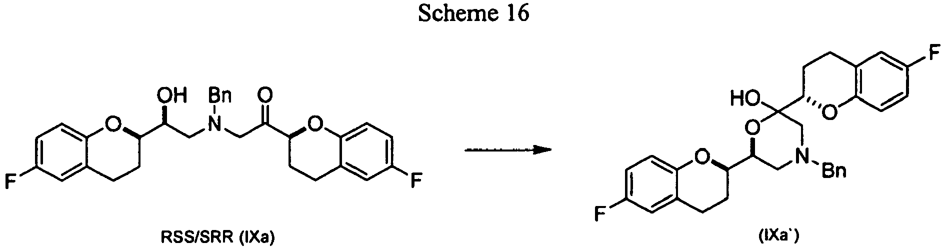

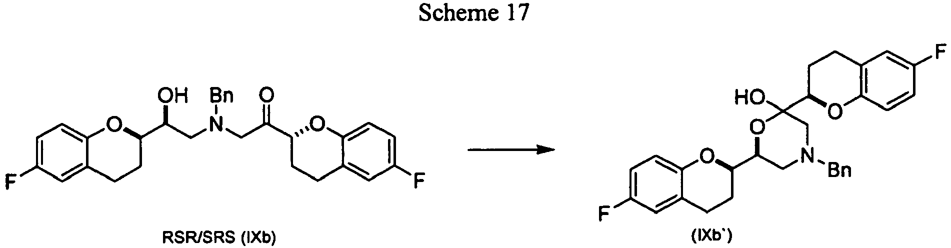

The relative configuration of the compound **IXb'** was confirmed by single **X-ray** measurement as shown below:

**Table 4: Crystallographic data of compound IXb'**

| | |
|---|---|
| Crystallized from | MeCN |
| Empirical formula | C₂₉H₂₉F₂NO₄ |
| Formula weight [g mol⁻¹] | 493.55 |
| Crystal colour, habit | colorless, prism |
| Crystal dimensions [mm] | 0.25 × 0.25 × 0.28 |
| Temperature [K] | 160(1) |
| Crystal system | monoclinic |
| Space group | *P*2₁/*n* (#14) |
| Z | 4 |
| Reflections for cell determination | 5882 |
| 2*θ*range for cell determination [°] | 4-55 |
| Unit cell parameters *a* [Å] | 14.0502(3) |
| *b* [Å] | 11.3937(3) |
| *c* [Å] | 15.5302(3) |
| *α* [°] | 90 |
| *β* [°] | 100.145(1) |
| *γ* [°] | 90 |
| *V* [Å³] | 2447.3(1) |
| *F*(000) | 1040 |
| *Dₓ* [g cm⁻³] | 1.339 |
| µ(Mo Kα) [mm⁻¹] | 0.0986 |
| Scan type | φ and ω |
| 2θ(max) [°] | 55 |
| Total reflections measured | 54789 |
| Symmetry independent reflections | 5601 |
| *R*ᵢₙₜ | 0.057 |
| Reflections with *I* > 2σ(*I*) | 4092 |
| Reflections used in refinement | 5598 |
| Parameters refined | 330 |
| Final *R(F)* [*I*> 2σ(*I*) reflections] | 0.0468 |
| *wR(F²)* (all data) | 0.1252 |
| Weights: | *w* = [σ²(*F*ₒ²) + (0.0616*P*)² + 0.3697*P*]⁻¹ where *P* = (*F*ₒ² + 2*F*_{c}²)/3 |
| Goodness of fit | 1.052 |
| Secondary extinction coefficient | 0.012(2) |
| Final Δₘₐₓ/σ | 0.001 |
| *Δρ*(max; min) [e Å⁻³] | 0.27; -0.21 |
| *σ*(*d*_{(C-C)}) [Å] | 0.002 |

Step 8 involves preparation of compound **X** as shown in Scheme 18. Non-limiting examples of compound **X** include (±)-[2R*[R*[R*(S*)]]]-α,α'-[[(phenylmethyl)imino]bis(methylene)]-bis[6-fluoro-chroman-2-methanol] (intermediate **Xa**) and (±)-[2R*[S*[R*(S*)]]]-α,α'-[[(phenylmethyl)imino]bis(methylene)]bis[6-fluoro-chroman-2-methanol] (compound **Xb**). A variety of reducing agents may be used for reduction of compound **IXa,** whereupon two racemic diastereomers can be formed having the RSSS/SRRR configuration (compound **Xa**) or the RSRS configuration (compound **Xb**). With regard to the Nebivolol synthetic strategy as depicted in Schemes 6a and 18, reduction methods giving the compound **Xa** in excess are preferred. There are few investigations of the selective reductions of chiral 1-hydroxy-5-keto compounds controlled by stereogenic centres at remote positions of the chain (distance of four or more atom centers; Tetrahedron Letters 35 (1994) 4891-4894, Tetrahedron Letters 40 (1999) 593-596, J. Org. Chem. 63 (1998) 7964-7981). In contrast to these investigations, compound **IXa** contains three asymmetric centres especially in 1-2, as well as in 1-5 and 1-6 position, which may control the diastereoselectivity in the reduction of the keto group.

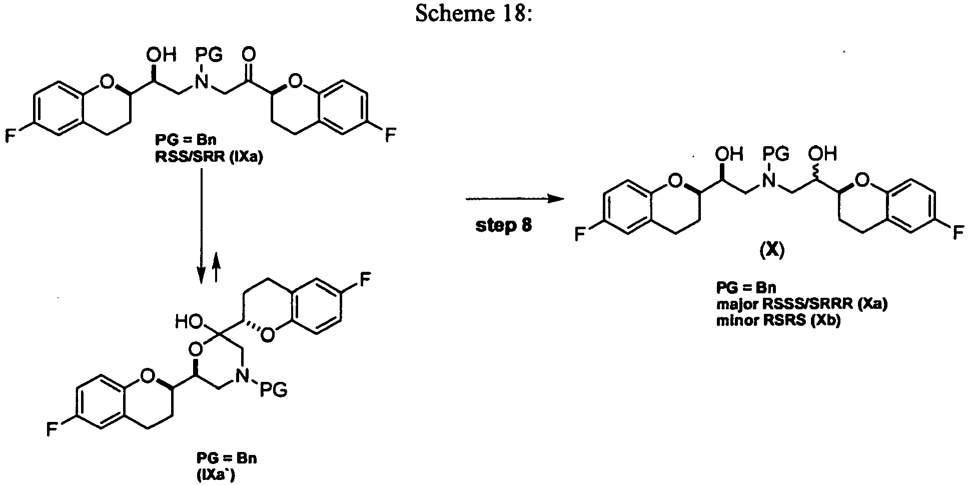

PG may be hydrogen or an appropriate amine protecting group as defined above. Preferred are the same protecting groups as already described for Steps 6a-c and 7.

There is in principal no limitation in the use of borohydride or aluminiumhydride reduction reagents which may be selected from, e.g., LiBH₄, NaBH₄, KBH₄ N(nBu)₄BH₄, Zn(BH₄)₂, NaH(Oac)₃, Superhydride^{®}, Red-Al, Li-Selectride, BH₃xSMe₂ or the like, or such reagents which are useful for Meerwein Pondorf Verley reductions. However, it must be considered that Meerwein Pondorf Verley reductions are reversible (Oppenauer Oxidation). Since compound **Xa** contains two secondary alcohol groups which may be in an oxididation/reduction equilibrium with the reduction reagent, a mixture of three diastereomers (benzylated Nebivolol (RSSS/SRRR) and the two meso forms thereof (RSRS and RRSS)) may be formed.

To avoid such side reactions, the hydroxyl group of compound **IXa** may be protected before the Meerwein Ponndorf Verley reduction is carried out. Another possibility is the continuous distillation of the ketone (e.g. acetone if isopropanol is used as hydride donor).

Catalytic hydrogenation of compound **IXa** may be a further option but if PG is a reduction labile protective group (e.g. benzyl) then deprotection must be taken into account.

The reductions may be carried out in absence or in presence of a Lewis acid selected from MgCl₂, CaCl₂, BaCl₂, ZnCl₂ Al(Oalkyl)₃, Ti(Oalkyl)₄ BF₃xOEt₂ or the like. Suitable solvents are ether, alcohols, halogenated hydrocarbons, or the like, with the exception that halogenated solvents are unsuited for catalytic reductions. The reduction is conveniently carried out at -20°C to room temperature. Even though lower temperatures may increase the selectivity, the reaction time will be extended, and higher temperature may cause a lost of selectivity. Table 5 shows typical results for the selective reduction of compound **IXa**.

**Table 5:**

| Reagent (eq.) | Catalyst (eq.) | Solvent | Temperature [°C] | Time [h] | Ratio **Xa//Xb** RSSS/SRRR // RSRS |
|---|---|---|---|---|---|
| NaBH₄ (1) | none | THF/EtOH/H₂O | RT | 3 | 50 // 50 |
| LiBH₄ (1) | none | THF | 0-5 | 5 | 70 // 30 |
| LiBH₄ (1) | Ti(OiPr)₄ (2) | THF | -20 to -RT | 3 | 78 // 22 |
| LiBH₄ (1) | Ti(OiPr)₄ (2) | THF | 0-5 | 2 | 82 // 18 |
| Zn(BHa)₂ (1) | none | Et₂O/THF | RT | 3 | 77 // 23 * |
| KBH₄ (1) KBH₄ (1) | ZnCl₂ (2) | MeOH/THF | 0-5 | 2 | 83 // 17* |
| K-Selectride (2) | none | THF | 0-5 | 3 | 84 // 16 |
| K-Selectride (4) | Ti(OiPr)₄ (2) | DCM/THF | 0-5 | 3 | 83 // 17* |
| KBH₄ (1) | LiCl | THF | 0-5 | 18 | 70 // 30 |
| Bu₄BH₄ (1) | none | THF | 0-RT | 24 | 94.4 // 5.6 |
| KBH₄ (1) | Ti(OiPr)₄ (2) Diglyme (3) | THF | 0-RT | 26 | 94.5 // 4.5 |
| KBH₄ (1) | Ti(OiPr)₄ (2) | DME | 0-RT | 26 | 94.5 // 4.5 |

| | | | | | |
|---|---|---|---|---|---|
| In Table 2, incomplete conversion is marked with the symbol "*." | | | | | |

The ratio of diastereomeric configurations RSSS/SRRR to RSRS is from about 1 to about 20, preferably 2 to 20, and most preferably 4 to 20.

After a complete conversion, the work up procedure can be done in a normal manner. The diastereomeric product mixture may be separated by column chromatography or by fractional crystallization. Since the compounds **Xa** and **Xb** have basic properties, salt formation prior to the fractional crystallization is a further option. The diastereomeric product mixture may be also used as crude product without further purification for the next step.

Step 9 involves preparation of (±)-[2R*[R* *[R*(S*)]]]-α,α'-[iminobis(methylene)]bis[6-fluoro-chroman-2-methanol] hydrochloride (compound **I**) and separation from the byproduct (±)-[2R*[S*[R*(S*)]]]-α,α'-[iminobis-(methylene)]bis[6-fluoro-chroman-2-methanol] hydrochloride as shown in Scheme 19.

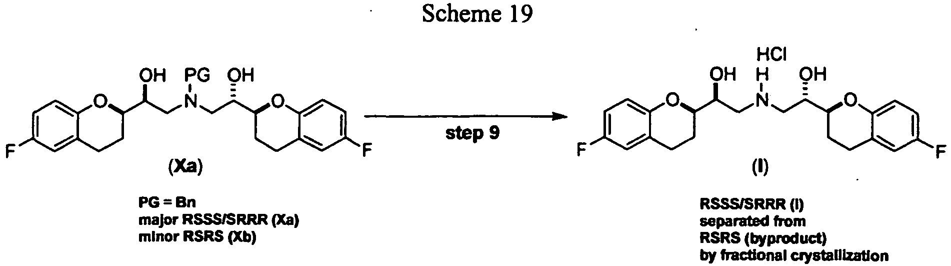

The final steps for the preparation of racemic Nebivolol hydrochloride include deprotection, salt formation and purification by fractional crystallization to remove the byproducts, mainly the undesired diastereomer having the RSRS configuration.

PG may have the same meaning as already described above, and if PG is other than hydrogen, the deprotection may be carried out by known procedures. Since benzyl groups are most preferred, the deprotection can be carried out by catalytic hydrogenation. If compound **Xa** contains compound **Xb** as a byproduct, then the purification may be done by fractional crystallization. Since compounds **Xa** and **Xb** have basic properties after deprotection, the fractional crystallization may be done after an appropriate salt formation. It was found that a mixture consisting of Nebivolol and its RSRS diastereomer can be readily separated by fractional crystallization after formation of the HCl salt or any other pharmaceutically acceptable salt.

Compound **I** may be converted to its pharmaceutically acceptable non-toxic acid addition salt formed by treatment with appropriate acids, such as, for example, inorganic acids, such as hydrohalic acid, e.g. hydrochloric, hydrobromic and the like, and sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propane-tricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids.

The salt formation using HCl is preferred for the fractional crystallization to provide readily pharmaceutically acceptable HCl salt of Nebivolol. The fractional crystallization may be generally done in suitable solvents in which Nebivolol is less soluble than its RSRS diastereomer. Alcohols as solvents for the fractional crystallization are preferred and methanol is the most preferred solvent.

### STEPS FOR RECYCLING OF DIASTEREOMERS

This invention also incudes steps for the diastereomer recycling formed during the process.

### I. Recycling options for the intermediates formed in Step 5 and Step 6

A non-limiting example of recycling is illustrated in Scheme 20 using Cl as a leaving group.

The recycling step can be conducted using e.g. a Mitsunobu reaction for inversion of the secondary alcohol group for recycling of the undesired diastereomer **VIII(b)** formed in Step 6. However, a suitable protection (PG') of the nitrogen may be required. As a person skilled in the art would appreciate, examples of suitable protective groups PG' include formation of corresponding carbamates by using e.g., alkyl chloroformates or formation of corresponding amides by using carboxylic acid chlorides or anhydrides. The protective group can be introduced after separation of the diastereomeric mixture by fractional crystallization followed by isolation of the undesired diastereomer from the mother liquors.

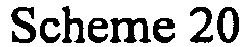

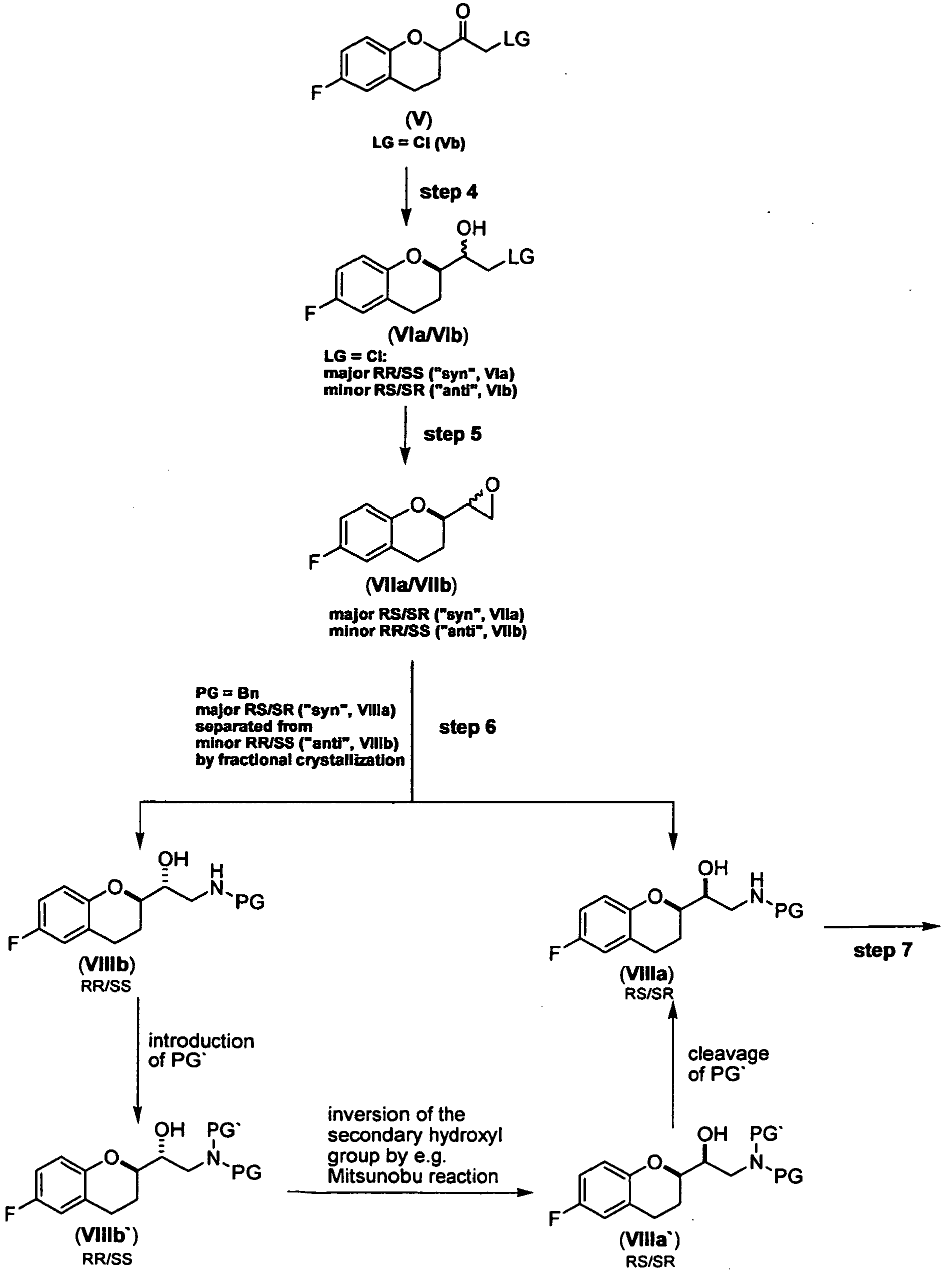

### II. Recycling option for compounds formed in Step 7

In Step 7, a mixture of two diastereomers is formed (compounds **IXa** and **IXb)** which is separated by fractional crystallization. There are two options for a recycling of the undesired diastereomer **IXb** (Scheme 21, Tables 6, 7, 8). The first one is an epimerization of the undesired diastereomer **IXb** to give again a mixture consisting of **IXa** and **IXb** which can be separated by the above described method.

**Table 6: Epimerization studies in various solvents containing 10% 1,8-Diazabicyclo[5.4.0]undec-7-ene (DBU), starting from IXa/IXb ratio of about 23/77.**

| Solvent | Temperature [°C] | Ratio of compounds **IXa/IXb** | Ratio of compounds **IXa+IXb/VIIIa** |
|---|---|---|---|
| MeOH | RT | 69/31 | 20/80 |
| DMF | RT | 53/47 | 85/15 |
| Acn | RT | 53/47 | 71/29 |
| THF | 40 | 53/47 | 61/39 |
| AcOEt | 40 | 54/46 | 74/26 |
| Toluene | 40 | 55/45 | 81/19 |

The mixtures were analyzed after 40 h by HPLC. The results in Table 6 show that the cleavage of the compounds **IXa/IXb** in methanol to the compound **VIIIa** is faster than the epimerization. Epimerization in all other solvents gave after 40 h an almost 1/1 mixture of the diastereomers **IXa** and **IXb,** whereas the tendency for cleavage to compound **VIIIa** is accurately suppressed especially in DMF and toluene as solvent. Further investigations of epimerization in DMF and toluene are shown in Tables 7 and 8.

**Table 7: Epimerization studies in DMF containing 5% 1,8-Diazabicyclo[5.4.0]undec-7-ene (DBU) at 30°C, starting from IXa/IXb ratio of about 23/77. The mixtures were analyzed by HPLC**

| Time [h] | Ratio of compounds **IXa/IXb** | Ratio of compounds **IXa+IXb/VIIIa** |
|---|---|---|
| 1 | 28/72 | 98.3/1.7 |
| 2.5 | 31/69 | 97.4/2.6 |
| 5.5 | 37/63 | 95.6/4.4 |
| 22.5 | 50/50 | 91.3/8.7 |
| 45.5 | 54/46 | 87.8/12.2 |

**Table 8: Epimerization studies in toluene containing 5% 1,8-Diazabicyclo[5.4.0]undec-7-ene (DBU) at 30°C, starting from IXa/IXb ratio of about 23/77. The mixtures were analyzed by HPLC**

| Time [h] | Ratio of compounds **IXa/IXb** | Ratio of compounds **IXa+IXb/VIIIa** |
|---|---|---|
| 1 | 24/76 | 99.7/0.3 |
| 5.5 | 27/73 | 99.4/0.6 |
| 22.5 | 33/67 | 98.3/1.7 |
| 45.5 | 43/58 | 96.0/4.0 |

The second recycling option includes the cleavage of the undesired diastereomer **IXb** to give a mixture of compound **VIIIa** and some byproducts. The cleavage may be done by a tautoterism of the aminoketone into the enamine form followed by hydrolysis using methods known in the art. The compound **VIIIa** could then be isolated and reintroduced into the process again as shown in Scheme 21.

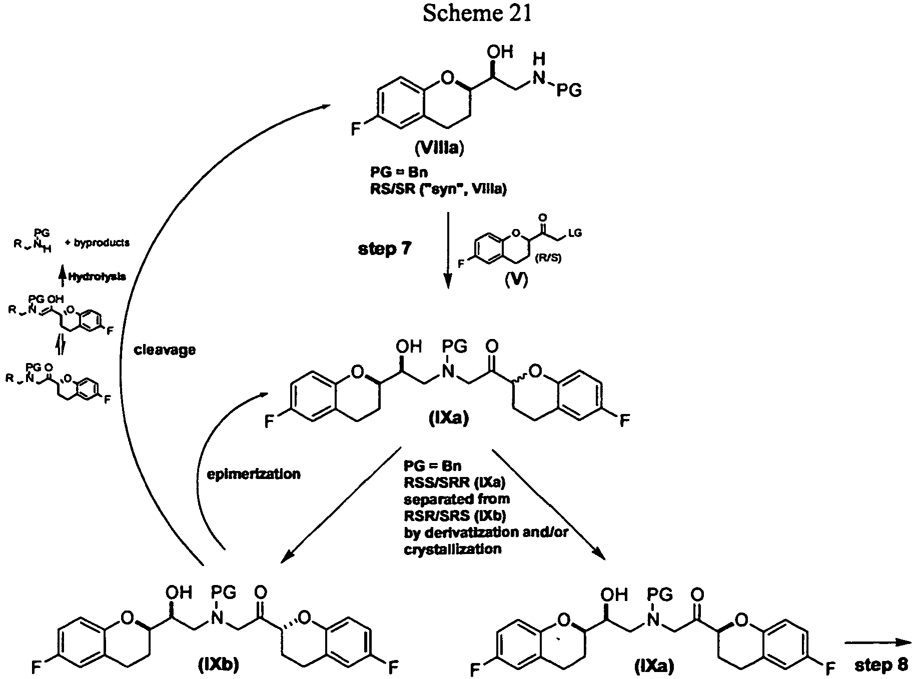

### III. Alternative synthesis of Nebivolol using the diastereomer VIIIb

Although, in accordance with the above described synthetic strategy producing the diastereomer **VIIIa** as an intermediate is preferred, the undesired diastereomer **VIIIb** can also be used as intermediate for the preparation of racemic Nebivolol as shown in Scheme 22.

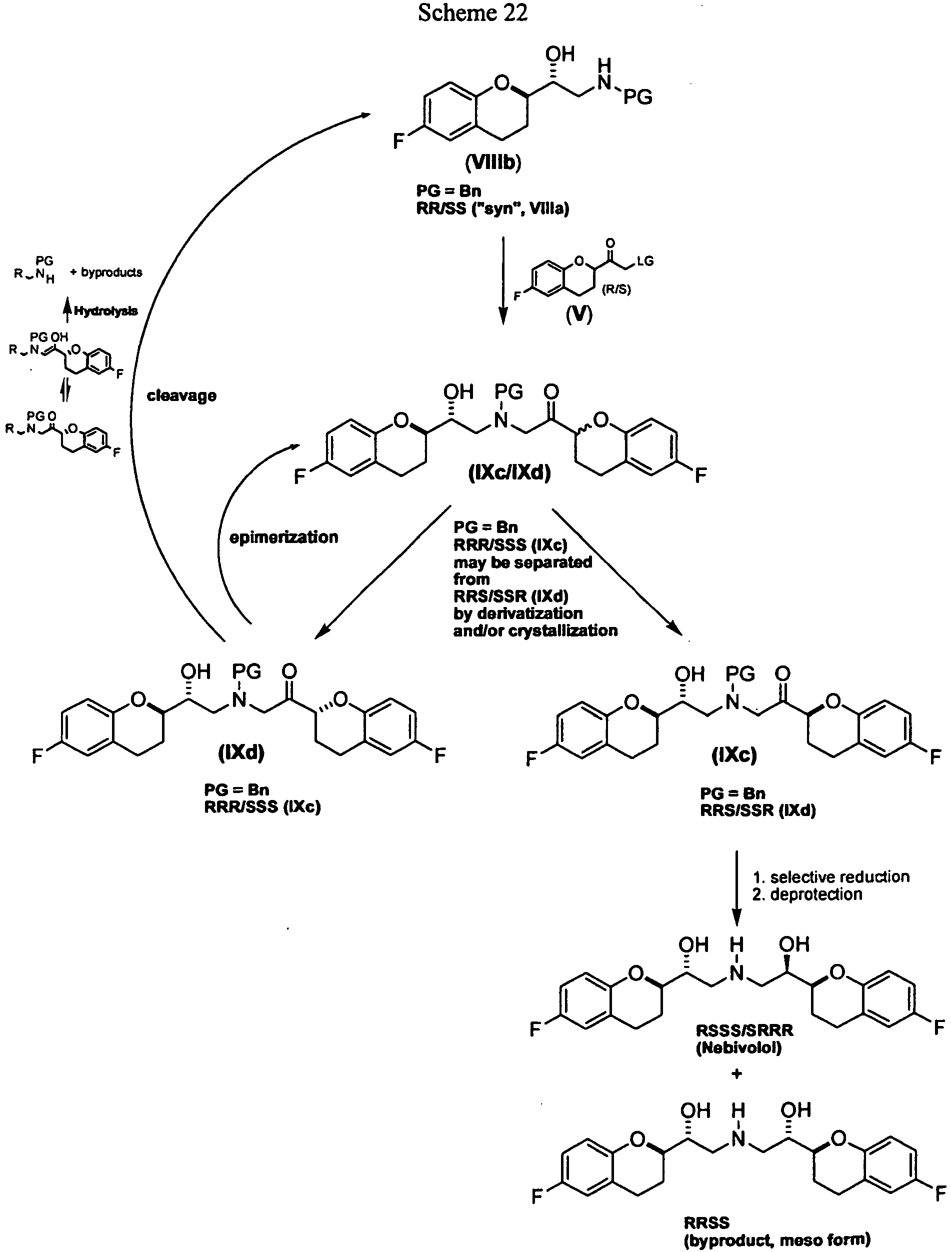

Other similar recycling methods are also possible. For example, in contrast to the route using the preferred compound **VIIIa,** the use of compound **VIIIb** will deliver after the alkylation step a diastereomeric mixture of compounds **IXc** and **IXd.** Reduction of compound **IXd** will give after deprotection Nebivolol and the second meso form having the RRSS configuration (in contrast, the preferred route produces the meso form having RSRS configuration). As mentioned above, the meso form having RSRS configuration is more soluble than the second meso form (RRSS), and therefore, Nebivolol contaminated with the RSRS diastereomer (according to the preferred route) can be easily purified by recrystallization. In case of RRSS contamination of Nebovolol, purification could be also carried out by recrystallization, but due to the similar solubility of the RRSS diastereomer compared with the solubility of Nebivolol, the purification is more difficult and loss of yield has to be taken into account.

### IV. Recycling of the Nebivolol meso forms (RSRS and RRSS)

Both Nebivolol meso forms obtained according to the above described processes may be directly converted to Nebivolol after a suitable protection (e.g. cyclic carbamate, cyclic silyl group etc.), followed by inversion of the secondary alcohol group (e.g. by Mitsunobu reaction) and deprotection as shown in Scheme 23.

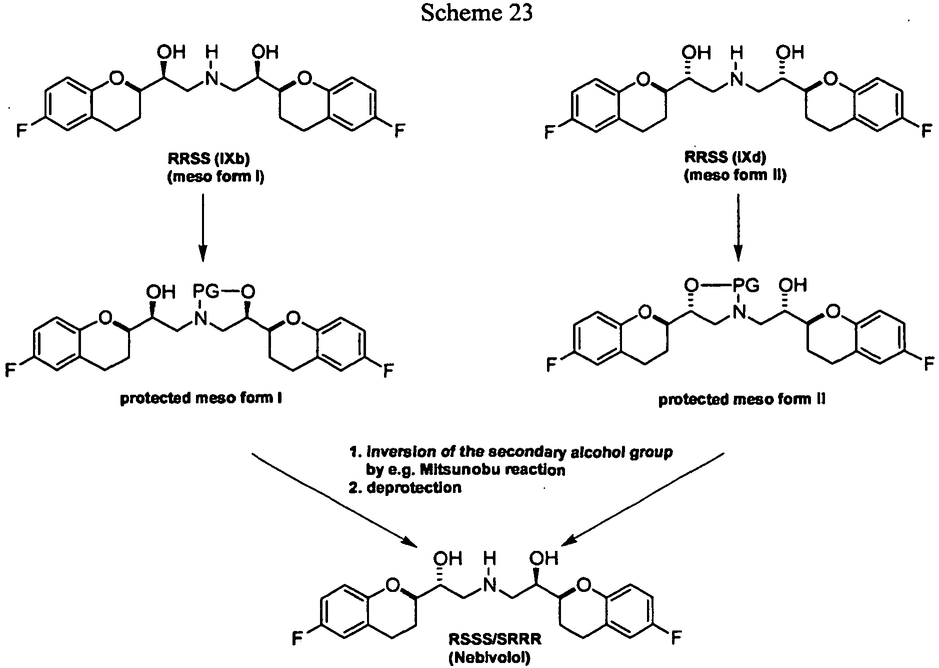

Reactions for protecting, deprotecting and inversion can be performed by methods known in art.

Although these methods decrease the costs and enhance the efficiency by recycling of the undesired diastereomer, they still require additional steps for recycling and isolating of the desired compounds. To reduce the costs and the environmental impact, a further improvement for the preparation of the compound **IXa/IXa'** is achieved as described below.

It was further discovered that under certain conditions, a further enrichment favouring the formation of **IXa** can be achieved by epimerization of the equimolar mixture of **IXa** and **IXb,** which results in a more efficient process than the recycling methods described above. Such increase of the ratio of desired diastereomer to undesired diastereomer in the mixture results in the improvement of the yield and volume yield and facilitates the isolation of IXa by fractional crystallization. Finally, waste and production time is also reduced by this method.

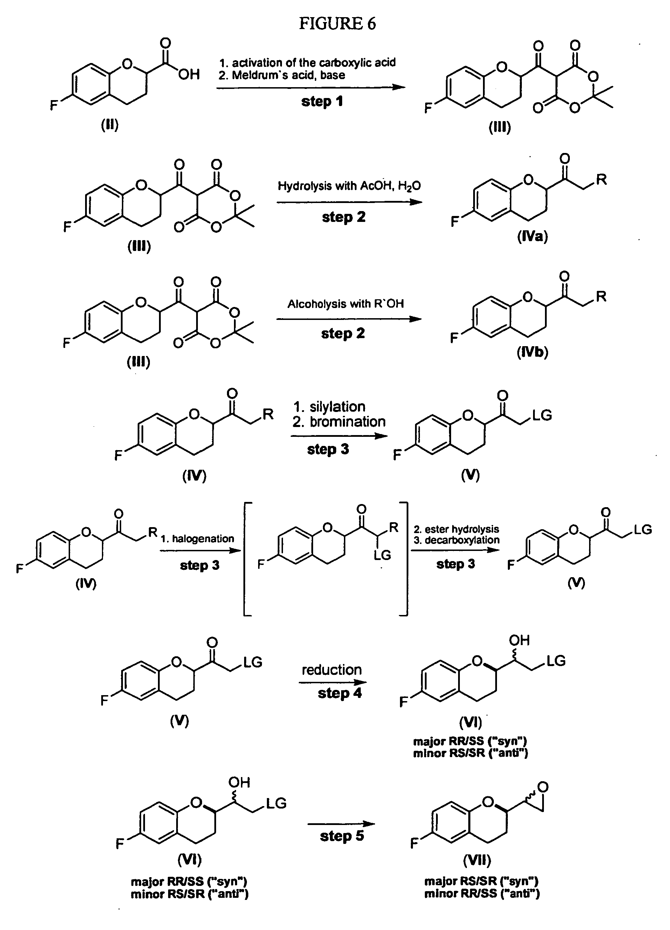

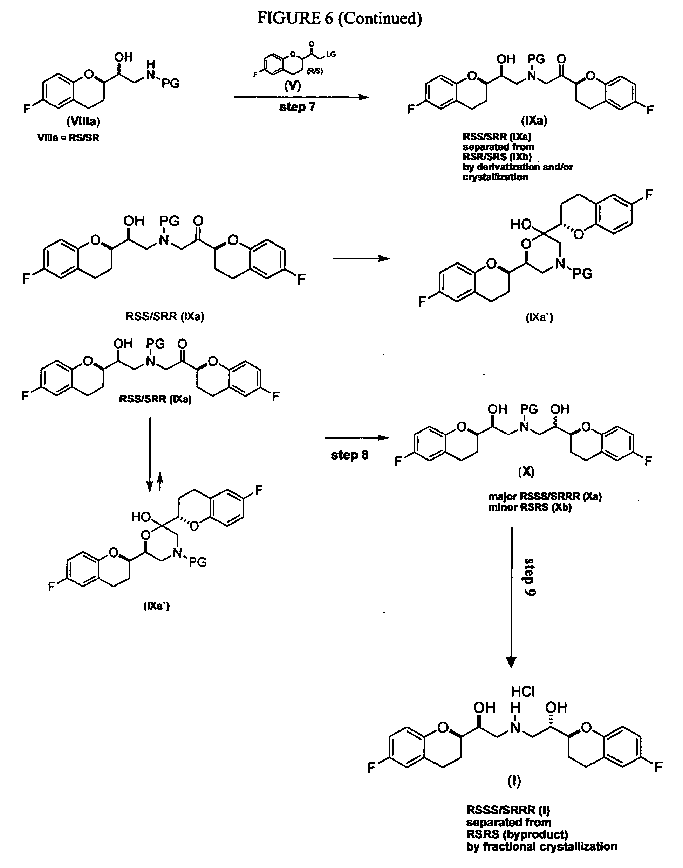

Therefore, this invention provides new methods and conditions to increase the diastereomeric ratio **of IXa** and **IXb** in step 7 (Figure 6) from about 1:1 up to 9:1 before the isolation **of IXa** is carried out (see Figures 7-9).

The increase of the diastereomeric ratio can be obtained using a simultaneous epimerization-crystallization procedure comprising an epimerization of a mixture consisting of compounds **IXa** and **IXb** in a ratio of about 1:1, in the presence of a suitable base and in an organic solvent wherein the solubility of compound **IXa** is slightly worse than the solubility of compound **IXb.**

Figure 7 shows the simultaneous epimerization-crystallization procedure which is a dynamic process for enriching the diastereomeric ratio and obtaining the desired diastereomer (compounds **IX** are illustrated in the acyclic forms).

A protective group (PG) may be any appropriate amine protecting group. PG is preferably selected from an allyl group or a substituted or unsubstituted arylmethyl group; the most preferred PG is a benzyl group (Bn). Suitable bases for the epimerization can be selected from alkoxides, amidines, guanidines or phosphazenes, preferred bases are amidines such as, for example, diazabicycloundecene (DBU), diazabicyclononene (DBN) and the most preferred base is DBU. The base may be used in at least 0.05 equivalents and 0.25 equivalents are preferred. The epimerization is preferably carried out in a temperature range between 20°C and 70°C and most preferably between 40°C and 70°C. Lower temperatures can also be used but they prolong the reaction time and higher temperatures may cause a higher degradation rate. The epimerization can be carried out under isothermal conditions but slow cooling of a mixture within the described temperature range is preferred. The organic solvent can be selected from solvents in which a suspension can be formed within the described temperature range to carry out the simultaneous crystallization-epimerization procedure of the invention. Suitable solvents are generally selected from aprotic solvents e.g., aromatic solvents, ethers, esters, amides or nitriles or from protic solvents, such as, for example, alcohols. Acidic solvents, which inactivate the base by salt formation, are preferably excluded. Preferred are aprotic solvents and the most preferred solvent is acetonitrile. Since the presence of water can cause degradation of the compounds **IXa** and **IXb,** it is further preferred to use solvents, wherein the amount of water is less than 1.0% and most preferably less than 0.1%.

The reaction is conveniently carried out by slow cooling of a suspension containing equimolar amounts of compounds **IXa** and **IXb** in the presence of a base. Compound **IXa** wherein the amount of the undesired diastereomer is ≤1% can be isolated in yields up to 74% and in only one step without the need of several recrystallizations, selective derivatizations or additional recycling steps as described above.

Thus, the simultaneous epimerization-crystallization procedure comprises simultaneous (a) an epimerization of a mixture consisting of compounds **IXa** and **IXb** in the presence of a suitable base and in an organic solvent and (b) favoured crystallization of IXa from the mixture. It is necessary that during the epimerization, the compound IXa is removed from the equilibrium by simultaneous crystallization. The epimerization studies were done in solution and not in suspension.

The simultaneous epimerization-crystallization procedure for synthesis of compound **IXa** is superior to the above described crystallization and recycling methods because the preparation of **IXa** according to the selective derivatization process yielded **IXa** only in a yield of 41 % as compared to the yield of 74% according to the simultaneous epimerization-crystallization process. To obtain the same yield with the former method, it is therefore necessary to carry out several recycling steps what requires more time and chemicals and produces more waste. Furthermore, the small amounts of compounds **IXa/IXb** lost in the new process during the isolation of **IXa** can be easily recovered from the mother liquor and can be re-used for the next epimerization run. Recovering lost material and using it in the next epimerization procedure further increases the efficiency of the simultaneous epimerization-crystallization process.

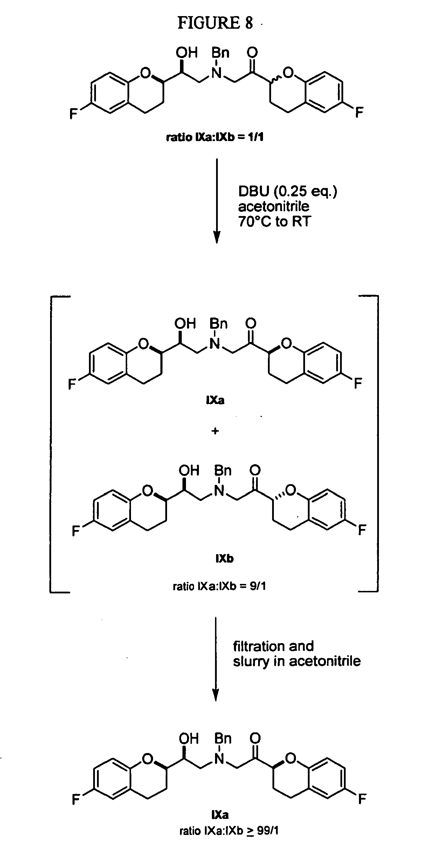

The simultaneous epimerization-crystallization procedure will now be described using Figure 8 as an example. Figure 8 shows the epimerization of a mixture of compounds **IXa** and **IXb,** wherein PG is a benzyl group (Bn). The **IXa** and **IXb** compounds are illustrated in the open-chain (acyclic) form. In solution, equilibrium of the acyclic forms **IXa** and **IXb** with the corresponding cyclic forms **IXa'** and **IXb'** is present. For the epimerization, the acyclic form is required, which has an acidic proton at the chiral centre in alpha position to the carbonyl group.

A slurry of a mixture consisting of an almost equimolar ratio of compounds **IXa** and **IXb** in acetonitrile is heated to an internal temperature of 70°C. After cooling to 60°C, DBU (0.25 eq.) is added, and the epimerization is carried out by cooling the mixture to 40°C using the temperature gradient described in Table 9.

**Table 9**

| Time [h] | Internal temperature [°C] |
|---|---|
| 0 | 60 |
| 0.5 | 55 |
| 1.5 | 50 |
| 5.5 | 50 |
| 5.75 | 45 |
| 8.5 | 45 |
| 8.75 | 40 |
| 13.5 | 40 |

The reaction is stopped by adding acetic acid (0.25 eq.) and the mixture is cooled to 25°C. The crude product is isolated by filtration and further purified by forming a slurry in acetonitrile. The filtration is done after finishing of simultaneous epimerization-crystallization process to isolate IXa. The following slurry in acetonitrile is only an additional purification step for removing remaining impurities and small amounts of the undesired diastereomer. Table 10 shows the success of the epimerization monitored by regular in-process controls (IPC) (determined by HPLC).

**Table 10**

| IPC | Time [h] | Temperature [°C] | Ratio IXa/IXb |
|---|---|---|---|
| 1 | 0 | - | 50.8 / 49.2 (starting material) |
| 2 | 0.5 | 55.5 | 56.1 / 43.9 |
| 3 | 1.5 | 50 | 62.9 / 37.1 |
| 4 | 2.5 | 50 | 66.3 / 33.7 |
| 5 | 3.5 | 50 | 71.0 / 29.0 |
| 6 | 4.5 | 50 | 75.1 / 24.9 |
| 7 | 5.5 | 50 | 78.7 / 21.3 |
| 8 | 7.5 | 45 | 85.0 / I5.0 |
| 9 | 8.5 | 45 | 87.3 / 12.7 |
| 10 | 9.5 | 40 | 82.0 / 18.0 |
| 11 | 10.5 | 40 | 85.2 / 14.8 |
| 12 | 11.5 | 40 | 89.7 / 10.3 |
| 13 | 13.5 | 40 | 89.2/10.8 |

Since the decomposition of **IXa/IXb** during the epimerization is assisted by H₂O, it is preferred that the starting material as well as the acetonitrile is almost anhydrous (Table 11). A mixture **IXa/IXb** was incubated at 50°C in the presence of 0.25 eq. DBU and various amounts of H₂O. The amount of remaining **IXa/IXb** was determined periodically by HPLC. The results are demonstrated in Table 11.

**Table 11:**

| % H₂O in ACN (v/v) | 2 h | 7 h | 22 h |
|---|---|---|---|
| 0.01 | 91% | 86% | 73% |
| 0.2 | 91% | 86% | 75% |
| 1.0 | 89% | 81% | 66% |

Temperatures above 70°C for more then 30 min should be avoided since **IXa/IXb** are not completely stable above 70°C. Decomposition and epimerization were monitored when stirring a slurry of **IXa** in ACN for 21 h at the appropriate temperature as shown in Table 12.

**Table 12**

| Temperature [°C] | Decomposition [%] | Epimerization [%] ¹⁾ (IXa -> IXb) |
|---|---|---|
| 75-78 | 6.8 | 5.6 |
| 65 | 0.8 | 0.2 |
| 50 | not detected | not detected |

| | | |
|---|---|---|
| ¹⁾ Since the rate of decomposition of **IXa** and **IXb** may be different, it is still unknown whether the change of ratio is rather caused by a faster decomposition of **IXa** than by epimerization. | | |

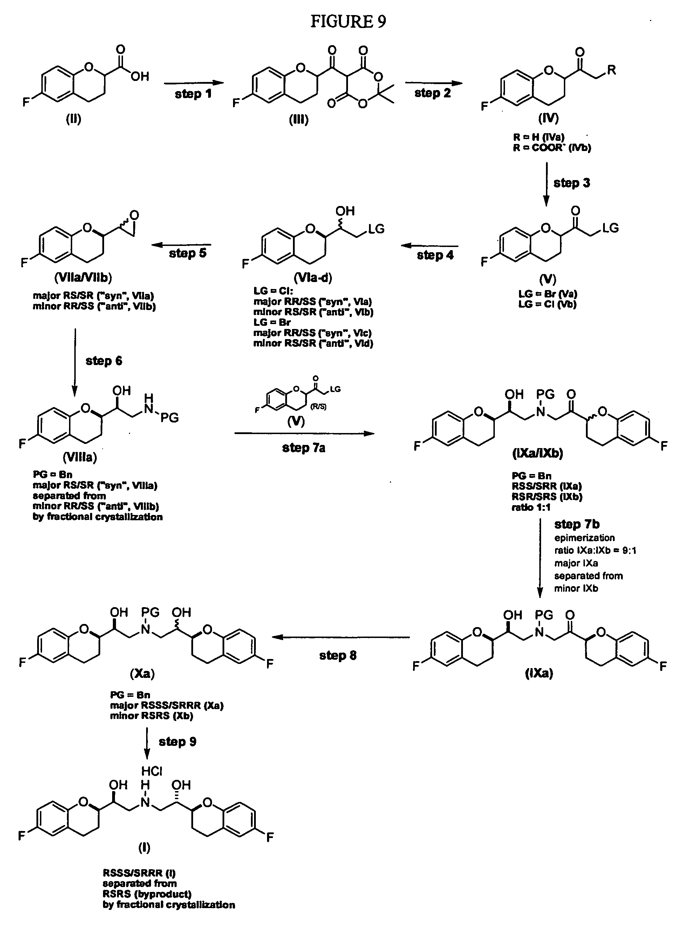

Figure 9 demonstrates a process of making racemic Nebivolol and its pharmaceutically acceptable salts wherein step 7b depicts the simultaneous epimerization-crystallization step of the invention.

Parameters for the simultaneous epimerization-crystallization step of the invention were selected based on realization that the solubility of compound IXa is less than the solubility of compound IXb. Thus, it was discovered that **IXa** can be crystallized in favour and therewith removed from the equilibrium while in solution the equilibrium of both diastereomers can be regenerated by epimerization of the undesired diastereomer **IXb** in presence of a base. Equilibrium was investigated at different temperatures with a certain base in a certain solvent and cooling rates. The cooling is necessary to complete the crystallization and to optimize the yield. Since the cooling reduces the solubility of both diastereomers, conditions for further crystallization in favour of the desired diastereomer were obtained. Because the epimerization is slower at lower temperatures, the adjustment of the equilibrium is also slower. A balanced ratio between rate of epimerization to adjust the equilibrium in solution and cooling of the mixture to favour the crystallization of IXa was obtained. Therefore, a temperature gradient (as described in Table 9 and in the Examples 19 and 20 below) is necessary to maintain a balanced ratio between epimerization rate and crystallization stages. Since higher temperatures can also cause degradation of the compounds IXa and IXb, which is still favoured in the presence of water, a condition for the epimerization-crystallization process has to be found wherein the degradation is reduced to a minimum to avoid lost of yield.

The invention will be illustrated in more detail with reference to the following Examples, but it should be understood that the present invention is not deemed to be limited thereto.

### EXAMPLES

### Example 1:

### Step 1: Preparation of (±)-5-[6-fluorochroman-2-carbonyl]-2,2-dimethyl[1,3]dioxane-4,6-dione (compound III).

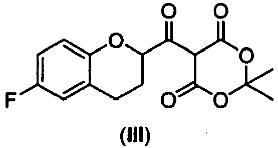

Thionylchloride (109.21 g, 918 mmol) was added under nitrogen atmosphere at 20-25°C to a suspension of 6-fluorochroman-2-carboxylic acid (90.00 g, 459 mmol) and DMF (1.68 g, 23 mmol) in toluene (635 ml). Afterwards, the suspension was heated to an internal temperature of 60-70°C, whereupon a clear yellow solution was obtained under simultaneous evolution of a gas. The reaction was completed within 70 min at this temperature, and the mixture was then concentrated in vacuum (bath temperature 45-50°C, pressure ≤35 mbar) to yield the chroman-2-carboxylic acid chloride as a yellow oil (112.65 g). The crude product was dissolved in methylene chloride (65 ml) and added slowly under nitrogen atmosphere to a solution of Meldrum's acid (70.90 g, 482 mmol) and pyridine (72.62 g, 918 mmol) in methylene chloride (261 ml) at an internal temperature of 0-10°C. The reaction mixture was allowed to warm to 20-25°C within 50 min. and stirred at this temperature for additional 30 min. Methylene chloride (325 ml) and water (325 ml) were then added to the formed brown suspension. The two phase mixture was stirred for 5 min, separated, and the organic layer was subsequently extracted twice with water (200 ml each), then with 2N aqueous HCl solution (250 ml) and finally with water (250 ml). After drying over Na₂SO₄, the organic layer was filtrated and concentrated in vacuo (≤50 mbar) to give a brown, viscous oil (170.76 g), which crystallized after 10 min at room temperature. The solid was slurried at 20-25°C in diisopropyl ether (500 ml) for 2h. After filtration of the suspension, the wet product was washed with diisopropyl ether (70 ml) and dried in vacuo (13 h at 40°C) to give a yellow-ocher colored solid (yield: 114.71 g, HPLC-purity: 96.98%).

### Example 2:

### Step 2, route A: Preparation of (±)-1-(6-fluoro-chroman-2-yl)-ethanone (compound IVa)

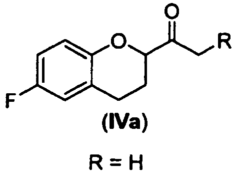

Example 1 was reproduced using 16 g of 6-fluorochroman-2-carboxylic acid, and the residue obtained after work up and evaporation of methylene chloride was used directly for Step 2, route A. A mixture of thus obtained crude product (compound **III**) with water (40 ml) and acetic acid (40 ml) was heated for 70 min to reflux and then cooled to room temperature. The reaction mixture was extracted with methylene chloride (40 ml), and the organic layer was twice washed with 1 N aqueous NaOH solution (each 20 ml). After drying over MgSO₄, the organic layer was filtrated and evaporated. The residue was purified by column chromatography over silica gel using ethylacetate/cyclohexane (1/3 by volume) as eluent. Collection of the second fraction and evaporation of the solvent gave the product as yellow oil (yield: 11.89 g, HPLC-purity: 98.76%).

### Example 3:

### Step 2, route B: Preparation of (±)-3-(6-fluorochroman-2-yl)-3-oxo-propionic acid ethyl ester (compound IVb as ethylester).

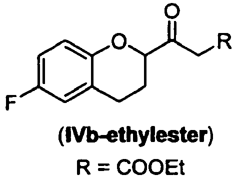

Example 1 was reproduced using 16 g of 6-fluorochroman-2-carboxylic acid and the residue obtained after work up and evaporation of methylene chloride was used directly for Step 2, route B. A suspension of this crude product (compound **III**) in ethanol (150 ml) was heated to reflux for 75 min, whereupon a clear solution was obtained. After cooling of the solution to room temperature and evaporation of the solvent, the residue was portioned between methylene chloride (80 ml) and water (80 ml). The phases were separated, and the organic layer extracted with 1 N aqueous NaOH solution (40 ml). The methylene chloride solution was dried over MgSO₄, filtrated and evaporated. The residue was purified by column chromatography over silica gel using ethylacetate/cyclohexane (1/4 by volume) as eluent. Collection of the first fraction and evaporation of the solvent gave the product as yellow-brown oil (yield: 11.89 g, HPLC-purity: 92.45%).

### Example 4:

### Step 2, route B: Preparation of (±)-3-(6-fluorochroman-2-yl)-3-oxo-propionic acid tert-butyl ester (compound IVb as tert-butyl ester).

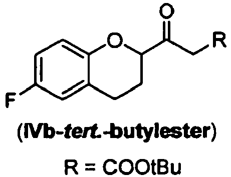

Tert-butanol (83.90 g) was added at room temperature to a suspension of compound **III** (94.00 g) (obtained by the process as described in Example 1) in toluene (280 ml). The suspension was heated to the internal temperature of 70-80°C, whereupon a clear solution was obtained under simultaneous evolution of a gas. The reaction was completed within 80 min. The mixture was cooled to room temperature and extracted successively with saturated NaHCO₃ solution (235 ml) and saturated NaCl solution. The organic layer was dried over Na₂SO₄, filtrated and concentrated in vacuo to give the product as an orange-brown oil (yield: 95.79 g, HPLC-purity: 97.20%; the crude product contained small amounts of toluene). The crude product was used for the next step without further purification.

### Example 5:

### Step 3, route A: Preparation of (±)-2-bromo-1-(6-fluoro-chroman-2-yl)-ethanone (compound Va).

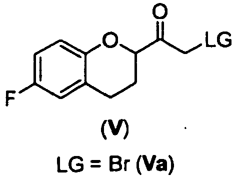

TMSCl (3.2 ml) was added to a solution of 2 M LDA (9.0 ml) in 20 ml THF at -78°C within 10 min. A solution of compound **IVa** (3.0 g) (obtained by the process as described in Example 2) in THF (3 ml) was then added and after 10 min, the reaction mixture was allowed to warm to room temperature within 40 min. A white solid precipitated and the suspension were portioned between cyclohexane (100 ml) and cold 10% NaHCO₃ solution (60 ml). The aqueous layer was diluted with water (20 ml) and separated. The organic layer was extracted twice with 10% NaHCO₃ solution (each 30 ml), dried over Na₂SO₄ filtrated and concentrated. The residue was dissolved in methylene chloride (15 ml) and cooled to an internal temperature of 0-5°C. A suspension of NBS (2.94 g) in methylene chloride (10 ml) was added to this mixture. After stirring for 1.5-2 h at this temperature, the reaction mixture was poured into 10% NaHCO₃ solution (15 ml), the organic layer was separated and concentrated. The residue was purified by column chromatography over silica gel using ethylacetate/cyclohexane (1/5 by volume) as eluent. Collection of the first fraction and evaporation of the solvent gave a product mixture consisting of compound **Va** (78.1 % by HPLC) and the corresponding byproduct (**VaBP1**), 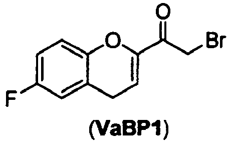 which was formed by non selective bromination followed by elimination as a yellow-brown oil (yield: 2.17 g, since compound **Va** seems to be less stable than compound **Vb**, it should be stored preferably under light exclusion at -20°C).

### Example 6:

### Step 3, route B: Preparation of (±)-2-bromo-1-(6-fluoro-chroman-2-yl)-ethanone (compound Va).

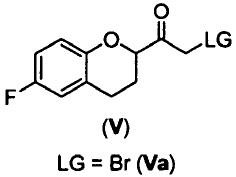

N-Bromo succinimide (NBS) (5.04 g) was added at 5-10°C in portions to a solution of compound **IVb**-*tert*-butyl ester (10.0 g) (obtained by the process as described in Example 4) and Mg(ClO₄)₂ (2.32 g) in ethyl acetate (100 ml). The reaction was subsequently monitored by HPLC. After the addition, the mixture was stirred at 5-10°C for 45min. Since 16% of the adduct remained, additional NBS (1.0 g) was added. The mixture was stirred for 20 min at 5-10°C, then allowed to warm to room temperature and stirred for 20 min at this temperature. The precipitate was filtered off, and the mother liquor concentrated in vacuo to give 2-bromo-3-(6-fluoro-chroman-2-yl)-3-oxo-propionic acid *tert*-butyl ester as a red oil (15.3 g). To carry out the hydrolysis and decarboxylation, the red oil was taken up in acetic acid (42 ml) and formic acid (49 ml), and the mixture was heated to an internal temperature of 80-85°C, whereupon an evolution of a gas was observed. After 60 min, the reaction was completed, and the mixture was concentrated in vacuo to give a brown oil. The oil was then dissolved in ethyl acetate (50 ml) and n-hexane (50 ml), and the solution was extracted successively twice with semi saturated NaCl solution (each 20 ml) and with saturated NaHCO₃ solution (20 ml). The organic layer was dried over MgSO₄ filtrated and concentrated in vacuo to give an amber oil, which was taken up in cyclohexane. The crystallization was initiated at room temperature by seeding. After 45 min, the suspension was filtered to give compound **Va** (2.98 g, beige solid) after drying. Additional amounts of compound **Va** (1.9 g) was obtained from the mother liquor after stirring at 6-7°C for 1.5 h, filtrating and drying (overall yield: 4.88 g, HPLC-purity: 98.5%; since compound **Va** seems to be less stable than compound **Vb**, it should be stored preferably under light exclusion at -20°C).

### Example 7:

### Step 3, route B: Preparation of (±)-2-chloro-1-(6-fluoro-chroman-2-yl)-ethanone (compound Vb).

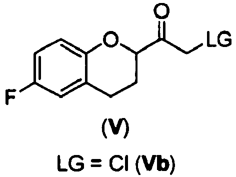

Mg (ClO₄)₂ (21.40 g) was slowly added at room temperature to a solution of compound **IVb**-*tert*-butyl ester (105.59 g) (obtained by the process as described in Example 4) in ethyl acetate (800 ml). Afterwards, NCS (41.80 g) was added in portions at 20-25°C within 3.5-4 h, and the reaction was subsequently monitored by HPLC. After complete addition, the yellow suspension was stirred for 30 min at 20-25°C and then filtrated. The filter cake was washed with ethyl acetate (100 ml), and the combined filtrates were extracted successively with saturated NaCl solution (150 ml) and water (150 ml) and afterwards concentrated in vacuo (60 mbar) to give a brown oil (116.82 g). To carry out the hydrolysis and decarboxylation, the brown oil was taken up in a mixture of acetic acid (420 ml), formic acid (390 ml) and water (80 ml). The solution was heated to an internal temperature of 80-90°C, whereupon an evolution of a gas was observed. After completion of the reaction (within 2 h), the solution was concentrated in vacuo (≤30 mbar) to give a dark orange oil (83.25 g), which was dissolved in ethyl acetate (400 ml). This solution was successively extracted with semi saturated NaCl solution (300 ml), saturated NaHCO₃ (300 ml) solution and water (100 ml). After drying over Na₂SO₄, the suspension was filtrated and concentrated to give initially a red oil (80.00 g), which slowly crystallized at room temperature. For further purification, the crude product (77.0 g) was dissolved in isopropanol (240 ml) at an internal temperature of 45-50°C. The solution was seeded to initiate the crystallization and cooled to 0-5°C. After 75 min stirring at 0-5°C, the suspension was filtrated, and the filter cake was washed with cold isopropanol (40 ml). The wet product was dried in vacuo at 35-40°C to give a yellowish solid (57.13 g, HPLC-purity: 99.00%).

### Example 8:

### Steps 1-3: Preparation of (±)-2-chloro-1-(6-fluoro-chroman-2-yl)-ethanone (compound Vb) from 6-fluorochroman-2-carboxylic acid (II)

A mixture of 6-fluorochroman-2-carboxylic acid (114.4 g, assay = 99%, 577 mmol), thionylchloride (83.15 g, 692 mmol) and DMF (2.18 g, 30 mmol) in toluene (471 g) was slowly heated under nitrogen atmosphere to an internal temperature of 70-80°C (at an internal temperature of 57°C an evolution of a gas started). When the raction was complete (within 40 min at 78°C, HPLC analysis showed 98.6% of the corresponding acid chloride), an amount of 208g solvent was distilled off in vacuum (pressure: 220 (start)-155 (end) mbar, internal temperature: 73 (start)-69 (end)°C, steam temperature: 39 (start)-63 (end)°C). A second flask was charged with Meldrum's acid (89.1 g, 606 mmol), pyridine (89 ml, 1.11 mol) and methylenechloride (375 ml). After this mixture was cooled to an internal temperature of 0-5°C, the above prepared solution of 6-fluorochroman-2-carboxylic acid chloride in toluene was added slowly at an internal temperature of 0-5°C. The reaction mixture was then allowed to warm to 20°C within 80 minutes (an in process HPLC analysis showed 92.6% product). *Tert*-butanol (81.0 g, 1.08 mol) was added and the mixture was slowly (within 4 h) heated to an internal temperature of 70-80 °C under simultaneous distillation of the solvent and evolution of a gas. During the heating up (after 75 min and at an internal temperature of 56 °C) additional *tert-*butanol (75 g, 1.00 mol) was added. The distillation and the evolution of the gas stopped when the internal temperature had reached 75-80°C (at this time 370 g solvent were distilled off). When an in process HPLC anlysis showed the completion of the reaction, the mixture was cooled to 20°C and a solution of sulfuric acid (41.8 g) in water (200 ml) was added. The organic layer was separated, extracted twice with saturated NaHCO₃ solution (each 200 ml), then concentrated in vacuo to approximately 60% of the volume (pressure: 370-150 mbar; note: during distillation the water should be removed completely) and diluted at room temperature with ethylacetate (450 ml). After addition of Na₃PO₄ (91.5 g), sulfuryl chloride (53 ml) was added slowly (within 3 h) at an internal temperature of 10-20°C and stirring was continued until an in process HPLC analysis showed the completion of the reaction (approx. 1h). The mixture was extracted twice with water (each 150 ml) and distilled in vacuo (pressure: 150-170 mbar) until 305 g distillate was obtained. Afterwards, acetic acid (400 ml) was added and the mixture was distilled in vacuo again (pressure: 30-40 mbar) until additional 292 g distillate were obtained. Concentrated hydrochloric acid (84 ml) was added and the mixture was stirred at an internal temperature of 40-50°C until the reaction (hydrolysis and decarboxylation) was complete (4 h, monitored by HPLC). After 100 g solvent were distilled off in vacuo (pressure: 200-40 mbar; removal of remaining toluene and *tert*.-butanol), the emulsion was diluted at an internal temperature of 20°C with acetic acid (70 ml) to give a solution. Then, water (20 ml) and seeding crystalls were added to initiate the crystallization. When the crystallization started, additional water (230 ml) was added slowly. The suspension was stirred at room temperature (15 h), then filtrated and the filter cake was washed with a mixture of acetic acid and water (v/v =1/1,100 ml). The wet product was dried in vacuo at 40°C to give a ocher solid (overall yield: 101.84 g, HPLC-purity: 98.9%).

### Example 9:

### Step 4: Preparation of(±)-2-chloro-1-(6-fluoro-(2R*)-chroman-2-yl)-(1R*)-ethan-1-ol (compound VIa) and (±)-2-chloro-1-(6-fluoro-(2S*)-chroman-2-yl)-(1R*)-ethan-1-ol (compound VIb)

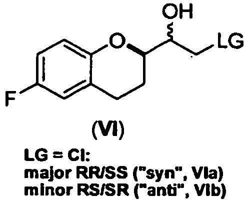

Compound **Vb** (33.74 g) was added at 0-5°C to a solution of ZnCl₂ (40.3 g) in methanol (470 ml), and the mixture was stirred until all solid was dissolved (1 h). The solution was cooled to -10°C, and NaBH₄ was added in portions within 35 min. After completion of the reaction monitored by HPLC, the mixture was concentrated to a volume of about 150 ml and then diluted with toluene (400 ml). The organic solution was successively extracted twice with 1.0 N HCl solution (each 200 ml) and with saturated NaHCO₃ solution (100 ml). After drying over MgSO₄, the suspension was filtrated, and the solvent evaporated in vacuo to give a brownish oil (35.28 g, ratio **VIa/VIb** = 61/39; the crude product mixture contains small amounts of toluene). The crude product was used for the next step without further purification.

### Example 10:

### Step 5: Preparation of (±) - 6-fluoro-[(2R*)-oxiran-2-yl]-(2S*)-chromane (compound VIIa) and (±)-6-fluoro-[(2R*)-oxiran-2-yl]-(2R*)-chromane (compound VIIb)

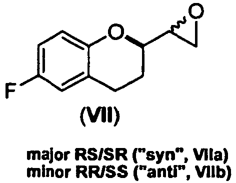

A methanolic NaOMe solution (30%, 30.9 g) was added at 20-25°C to a solution of a mixture of compounds **VIa** and **VIb** (37.9 g, ratio **VIa/VIb** = 61/39) in methanol (380 ml). The reaction was monitored by HPLC and after stirring for 3.5 h at 20-25°C, additional methanolic NaOMe solution (30%, 1.4 g) was added. After the reaction was completed (within 3.5 h), the mixture was neutralized by addition of acetic acid and then concentrated in vacuo. The residue was portioned between methylene chloride (300 ml) and a semi saturated NaCl solution (200 ml). The phases were separated, and the organic layer was dried over MgSO₄. After filtration, the filtrate was concentrated to give a brownish oil (32.1 g, ratio **VIIa/VIIb** = 61/39). The crude product was used directly for the next step.

### Example 11:

### Step 6: Preparation of (±)-2-Benzylamino-1-[6-fluoro-(2R*)-chroman-2-yl]-(1 1S*)-ethan-1-ol (compound VIIIa) and (±)-2-Benzylamino-1-[6-fluoro-(2R*)-chroman-2-yl]-(1R*)-ethan-1-ol (compound VIIIb) and separation of the diastereomers VIIIa and VIIIb

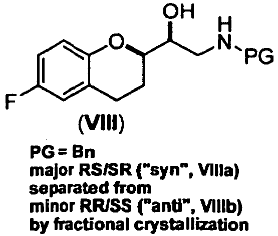

A mixture of compounds **VIIa** and **VIIb** (ratio: 57/43) was slowly added (within 1.5 h) at an internal temperature of 40°C to a solution of benzylamine (5.4 g) in 2-propanol (30 ml) . After completion of the reaction monitored by HPLC, the solution was cooled to room temperature and seeding material was added. Next, the diastereomers **VIIIa** and **VIIIb** were separated by fractional crystallization. The suspension was stirred at room temperature for 1 h and filtrated to give a colorless solid after drying in vacuo (1.01 g). The mother liquor was concentrated until 25 g residue was obtained. Afterwards, the concentrated mixture was heated to 60°C and cooled within 3 h to 0-5°C. Additional product was obtained after filtration and drying of the wet product in vacuo (0.3 g). The mother liquor was concentrated until 15 g residue was obtained, and diisopropyl ether (15 g) was added. A third fraction was obtained after filtration and drying of the wet product in vacuo (0.33 g). The second and third crop were recrystallized from 2-propanol (3.7 g), and after the filtration, the wet product (0.6 g) was dissolved with the first crop in 2-propanol (10 g) at reflux. The mixture was cooled to 0-5°C and then filtrated. The wet product was dried in vacuo to give a colorless solid (yield: 1.1 g, ratio **VIIIa/VIIIb** = 96/4).

Diastereomer **VIIIb** could be e.g., obtained from the mother liquor after concentration to dryness followed by an extractive work up and crystallization.

### Example 12:

### Steps 4-6: Preparation of (±)-2-Benzylamino-1-[6-fluoro-(2R*)-chroman-2-yl]-(1S*)-ethan-1-ol (compound VIIIa) and (±)-2-Benzylamino-1-[6-fluoro-(2R*)-chroman-2-yl]-(1R*)-ethan-1-ol (compound VIIIb) from (±)-2-chloro-1-(6-fluoro-chroman-2-yl)-ethanone (compound Vb) and separation of the diastereomers VIIIa and VIIIb

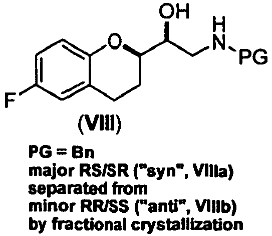

Compound **Vb** (76.31 g, 324 mmol) and ZnCl₂ (22.53 g, 162 mmol) was dissolved in ethanol (648 ml) at an internal temperature of 20-30°C. This solution was then cooled to an internal temperature of -15 to -20°C and a solution of NaBH₄ (12.77 g, 324 mmol) and NaOMe (4 ml of a 30% solution in MeOH) in MeOH (136 ml) was added slowly. During the addition the internal temperature was kept between -20 and -10°C and the reaction was monitored by HPLC. After completion of the reaction the mixture was allowed to warm to 0-5°C and hydrochloric acid was added (160 ml 2N HCl solution). The mixture was allowed to warm to 20-25°C and stirred at this temperature for 30 minutes. The solvent was almost removed completely in vacuo to give a brown suspension (191.3 g). This residue was portioned between hydrochloric acid (160 ml 2N HCl solution) and MTBE (450 ml). The organic layer was separated, extracted with hydrochloric acid (30 ml 2N aqueous HCl solution) then twice with water (each 250 ml) and concentrated in vacuo to give a brown oil (79.77 g, ratio **VIa/VIb** = 63.5/36.5). After dissolving of the oil in 2-propanol, a solution of NaOMe in MeOH (64.18 g, concentration: 30%) was added at an internal temperature of 20-25°C. The reaction was monitored by HPLC. After completion of the reaction, the mixture was cooled to 0-5°C and neutralized by addition of acetic acid (1.9 ml). The suspension was filtered over Celite and the filter cake washed with 2-propanol (25 ml). The filtrate was concentrated in vacuo to give a semiconcentrated turbid brownish solution (115.97 g). This mixture was filtered again and the filter cake washed with 2-propanol (25 ml) to give a clear brown solution which was then slowly added (within 3h) to a solution of benzylamine (105.2 g, 972 mmol) in 2-propanol (352 ml) at an internal temperature of 33-38°C. The reaction was monitored by HPLC and seeded to initiate the crystallization of the product during the reaction. After completion of the addition, the mixture was stirred for 3.5 h at 25-30°C, then cooled to 0-5°C and stirred at this temperature for 1.5 h. The suspension was filtered and the filter cake washed with precooled (0-5°C) 2-propanol (46 ml). The wet product was dried in vacuo at 50-55°C to give a slightly beige colored solid (42.23 g, ratio **VIIIa/VIIIb** = 92/8). The crude product was dissolved in acetonitrile (294 ml) by heating to reflux. The solution was slowly cooled to 0-5°C (6-7h), filtrated and the filter cake washed with acetonitrile (38 ml). The wet product was dried in vacuo at 50-55°C to give a white solid (overall yield: 38.2 g, ratio **VIIIIa/VIIIb** = 98.8/1.2, HPLC-purity of **VIIIa:** 98.62%).

### Example 13:

### Step 7: Preparation of (±)-2-{Benzyl-[2-(6-fluoro-(2R*)-chroman-2-yl)-(2S*)-hydroxyethyl]-amino}-1-(6-fluoro-(2S*)-chroman-2-yl)-ethanone (compound IXa), (±)-4-Benzyl-2-[6-fluoro-(2R*)-chroman-2-yl]-(6S*)-[6-fluoro-(2S*)-chroman-2-yl]-morpholin-2-ol (compound IXa'), (±)-2-{Benzyl-[2-(6-fluoro-(2R*)-chroman-2-yl)-(2S*)-hydroxy-ethyl]-amino}-1-(6-fluoro-(2R*)-chroman-2-yl)-ethanone (compound IXb), and (±)-4-Benzyl-(6S*)-2,6-bis-[6-fluoro-(2R*)-chroman-2-yl]-morpholin-(2S*)-ol (compound IXb') and separation of the diastereomers

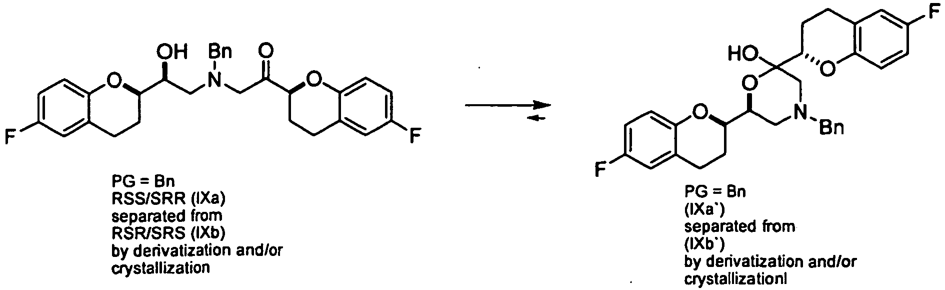

Compound **V** (14.62 g) was added at an internal temperature of 40°C to a suspension of compound **VIIIa** (17.5 g), NaHCO₃ (9.6 g) and NaBr (0.9 g) in DMF (70 ml). When the reaction was completed (within 3-3.5 h, monitored by HPLC), the suspension was cooled to room temperature and diluted with MTBE (400 ml) and water (200 ml). Afterwards, the phases were separated, the organic layer was extracted with water (100 ml), and the combined water layers were reextracted with MTBE (100 ml). After evaporation of the combined organic layers, the remaining amber colored oil (35.0 g) was taken up in diisopropyl ether (400 ml) and seeded. The suspension was initially stirred at room temperature for 1.5 h and then at 0-5°C for 0.5 h. After filtration, the wet product was dried in vacuo to give a light yellow solid (yield: 23.95 g, HPLC-purity 97.5%, ratio of **IXa/IXa'** to **IXb/IXb'** = 52/48).

The mother liquor was concentrated to an amount of 56 g, then cooled to 0-5°C and seeded. A second crop was obtained after filtration and drying (yield: 2.62 g, HPLC-purity 92.6%, ratio of **IXa/IXa'** to **IXb/IXb'** = 43/57

Separation of the diastereomers was conducted by fractional crystallization from acetonitrile.

The diastereomeric mixture of compounds **IXa/IXa'** and **IXb/IXb'** (ratio: 55/45,2.31 g) was dissolved in acetonitrile at an internal temperature of 70°C. The light yellow solution was seeded, cooled to room temperature (within 2-3 h) and stirred at this temperature for 1.5-2 h. Filtration of the suspension and drying of the wet product gave a first crop (yield: 0.26 g, ratio of **IXa/IXa** to **IXb/IXb'** = 95/5).

The mother liquor was concentrated to an amount of 30 g and stirred at room temperature after seeding. Filtration of the suspension gave a wet product (1.12 g), which was recrystallized from acetonitrile (11.2 g). Drying of the wet product in vacuo gave a second crop (0.50 g, ratio of **IXa/IXa'** to **IXb/IXb'** = 62/38). This crop was recrystallized from acetonitrile (10 ml) to give a wet product (0.57 g), which was dissolved again in acetonitrile (8ml) by heating. The solution was cooled to room temperature and seeded. After filtration of the suspension and drying of the wet product gave a third crop (yield: 0.16 g, ratio of **IXa/IXa'** to **IXb/IXb'** = 98/2).

Separation of the diastereomers was also conducted by selective silylation and fractional crystallization.

### Procedure A:

Imidazole (0.417 g) was added at 0-5°C to a suspension of compounds **IX** (2.0 g, prepared according to the above described procedure, ratio of **IXa/Xa'** to **IXb/IXb'** = 52/48) in a mixture of acetonitrile (13.5 ml) and THF (1.5 ml). Afterwards, TMSC1 (0.228 mg) was slowly added at this temperature within 3.5-4 h and under monitoring by HPLC. After the addition was complete, the mixture was concentrated in vacuo to an amount of 8-10 ml, and then acetonitrile (5 ml) was added. Stirring the suspension at 0-5°C for 1-1.5 h followed by filtration gave a white wet product (1.31 g), which was dried in vacuo (yield: 0.82 g, ratio of **IXa/IXa'** to **IXb/IXb'** > 98/2).

### Procedure B:

Imidazole (0.21 g) was added at 0-5 °C to a suspension of compounds **IX** (1.0 g, prepared according to the above described procedure, ratio of **IXa/IXa'** to **IXa/IXb'** = 52/48) in MTBE (10 ml). Afterwards, TMSC1 (0.115 mg) was slowly added at this temperature within 3.5-4 h and under monitoring by HPLC. The reaction was completed by addition of 4 drops of TMSC1. Afterwards, the suspension was filtrated, and the wet product (0.87 g) was dried in vacuo to give a white crude product (0.51 g, ratio of **IXa/IXa'** to **IXb/IXb'** = 98/2, the product contains imidazole hydrochloride). To remove the imidazole hydrochloride, the crude product was slurried at room temperature in a mixture of acetonitrile and water (3.0 ml, 4/1 by volume) for 2.5-3 h. Filtration of the suspension and drying the wet product (0.65 g) in vacuo gave a white solid (yield: 0.31 g, ratio of **IXa/IXa'** to **IXb/IXb'** = 98/2).

### Example 14:

### Step 7: Preparation of(±)-2-{Benzyl-[2-(6-fluoro-(2R*)-chroman-2-yl)-(2S*)-hydroxyethyl]-amino}-1-(6-fluoro-(2S*)-chroman-2-yl)-ethanone (compound IXa), (±)-4-Benzyl-2-[6-fluoro-(2R*)-chroman-2-yl]-(6S*)-[6-fluoro-(2S*)-chroman-2-yl]-morpholin-2-ol (compound IXa'), (±)-2-{Benzyl-[2-(6-fluoro-(2R*)-chroman-2-yl)-(2S*)-hydroxy-ethyl]-amino}-1-(6-fluoro-(2R*)-chroman-2-yl)-ethanone (compound IXb), and (±)-4-Benzyl-(6S*)-2,6-bis-[6-fluoro-(2R*)-chroman-2-yl]-morpholin-(2S*)-ol (compound IXb') and separation of the diastereomers

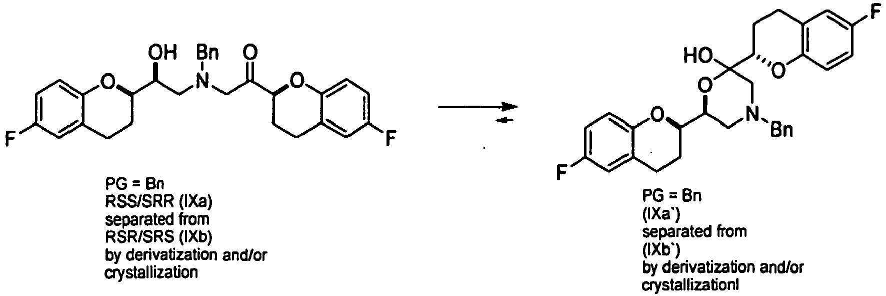

A mixture of compound **VIIIa** (49.0 g, 162.6 mmol, prepared according to example 12), compound Vb (42.5 g, 178.9 mmol, prepared according to example 8), NaBr (1.68 g, 16.3 mmol) and NaHCO₃ (20.5 g, 243.9 mmol) in DMF (200 ml) was heated to an internal temperature of 60-65°C until an in process HPLC analysis showed an almost complete conversion (approx. 1h). Afterwards the suspension was filtered and the filter cake washed with DMF (70 ml). To the filtrate was added at 50°C water (15 ml) and seeding crystals to initiate the crystallization. Then the product was careful precipitated by slow addition of water (within 4 h) at 50°C. Finally the precipitation was completed by addition of water (25 ml) at 50°C. The suspension was cooled to 20-25°C and filtrated. The wet product was washed with 2-propanol (100 ml) and dried in vacuo at 50°C to give a white solid (yield: 70.15 g, HPLC-purity: 99.1 %, ratio of **IXa/IXa'** to **IXb/IXb'** = 52/48). To a suspension of this solid (70.0 g) and imidazole (14.6 g, 214 mmol) in acetonitrile (385 ml) was added slowly (1.75 ml/h) TMSC1 (7.56 g, 68.2 mmol) at an internal temperature of -10 to -15 °C. After that the suspension was stirred for 2 h at -5 to 0°C under monitoring by HPLC. The reaction was completed by addition of TMSC1 (1.34 g, 12.3 mmol). The suspension was filtered and the wet product dried in vacuo at 40°C to give a white solid (66.45 g, ratio of **IXa/IXa'** to **IXb/IXb'** = 92/8). This product was slurried in a mixture of cyclohexane (285 ml) and MTBE (95 ml) at an internal temperature of 60°C for 10 minutes. After the suspension was cooled to 25°C and filtrated, the wet product was washed with cyclohexane (50 ml) and suspended again in cyclohexane (350 ml). The suspension was stirred at 60-65°C for 20 minutes, then cooled to 25°C and filtered. The wet product was washed with cyclohexane (50 ml) and dried in vacuo at 40°C to give a white solid (overall yield: 28.83 g, , ratio of **IXa/IXa'** to **IXb/IXb'** = 98.6/1.4).

### Example 15:

### Step 8: Preparation of (±)-[2R*[R*[R*(S*)]]]-α,α'-[[(Phenylmethyl)imino]bis-(methylene)]bis[6-fluoro-chroman-2-methanol] (compound Xa) and (±)-[2R*[S*[R*(S*)]]]-α,α'-[[(phenylmethyl)imino]bis(methylene)]bis[6-fluoro-chroman-2-methanol] (compound Xb)

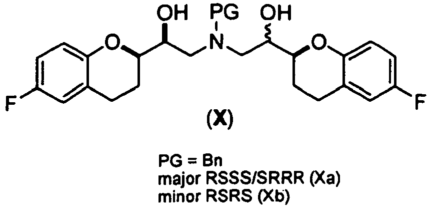

A solution of compound **IXa/IXa'** (0.40 g, containing 2% of compound **IXb)** in THF (8.0 ml) was cooled to an internal temperature of -10 to -15°C. To this solution, Ti(OiPr)₄ (0.485 mg) was added followed by LiBH₄ (18 mg). After stirring at -10 to -15°C for 1 h and at 0-5°C for 1.5-2h, the reaction mixture was poured into a mixture of methylene chloride (10 ml) and saturated NaHCO₃ solution (10 ml). The suspension was filtered over Celite, and the phases were separated. After drying over MgSO₄, the organic layer was concentrated to give a colorless foam (418 mg, ratio of **Xa/Xb** = 78/22). The crude product was used for the next step without any further purification.

### Example 16:

### Step 8: Preparation of (±)-[2R*[R*[R*(S*)]]]-α,α'-[[(Phenylmethyl)imino]bis-(methylene)]bis[6-fluoro-chroman-2-methanol] (compound Xa)

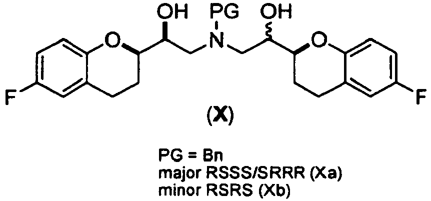

KBH₄ (3.15 g, 56.73 mmol) was added to a solution of compound **IXa/IXa'** (28.0 g, prepared according to example 14) and Ti(OiPr)₄ (32.9 g, 113.5 mmol) in DME (280 ml) at an internal temperature of 0 °C. After stirring at this temperature for 21 h (monitored by HPLC), the mixture was allowed to warm to room temperature and hydrochloric acid (280 ml, 10% aqueous solution) was added slowly. The suspension was stirred for 2.5 h. The suspension was filtered and the wet product washed first with a mixture of DME (25 ml) and hydrochloric acid (25 ml, 2N aqueous solution), then with hydrochloric acid (50 ml, 2N aqueous solution) and twice with water (each 50 ml). The wet product was suspended in ethanol (120 ml) and heated to 50°C. Afterwards, an aqueous solution of NaOH (8.3 g, 30%) was added to give initially a clear solution and the mixture was heated to 60°C. After the crystallization started, water was added (33ml) and the suspension was cooled to room temperature. The suspension was filtered and the wet product washed with a mixture of EtOH/water (20 ml, v/v = 3/1). Next, the wet product was dissolved in EtOH (160 ml) by heating to 70-75°C and then cooled to 65°C. Water (40 ml) and seeding crystalls were added and the mixture was cooled to room temperature and stirred at this temperature over night. After filtration, the wet product was washed with a mixture of EtOH/water (30 ml, v/v = 3/1) and dried in vacuo at 50°C to give a white solid (yield: 21.66 g, HPLC-purity: 99.85%).

### Example 17:

### Step 9: Preparation of (±)-[2R*[R*[R*(S*)]]]-α,α'-[iminobis(methylene)]bis[6-fluoro-chroman-2-methanol] hydrochloride (compound I) and separation from the byproduct (±)-[2R*[S*[R*(S*)]]]-α,α'-[iminobis-(methylene)]bis[6-fluoro-chroman-2-methanol] hydrochloride

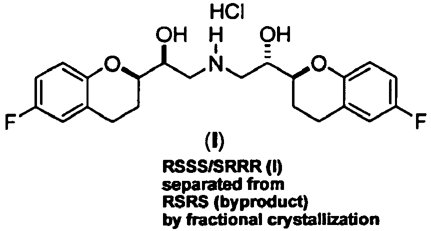

The compounds **Xa** and **Xb** (418 mg, ratio of **Xa/Xb** = 78/22, prepared according to Example 11) were dissolved in a mixture of EtOH containing 14% HCL (0.665 g) and MeOH (10 ml). This mixture was hydrogenated at normal pressure and at room temperature with palladium-on-charcoal catalyst 5% (100 mg). After completion of the reaction (within 3 h), the mixture was diluted with MeOH (25 ml), heated to 40°C and then filtrated over Celite. The filter cake was washed with hot MeOH (40°C, 30 ml), and the combined filtrates were concentrated in vacuo to an amount of 7-8 g. The resulting suspension was filtrated to give a colorless solid after drying of the wet product (yield: 0.17 g, ratio of compound I/byproduct = 95.5/4.5). The mother liquor was concentrated, and the residue taken up in 2.0 ml MeOH. The suspension was stirred at room temperature for 0.5 h and then filtrated to give an additional crop (yield: 28 mg) after drying the wet product in vacuo. Both crops were recrystallized from MeOH (2.0 ml) to give a colorless solid (yield: 0.161 g, ratio of compound I/byproduct = 98/2) after drying.

### Example 18:

### Step 9: Preparation of (±)-[2R*[R*[R*(S*)]]]-α,α'-[iminobis(methylene)]bis[6-fluoro-chroman-2-methanol] hydrochloride (compound I)

A mixture of compound **Xa** (21.0 g, 42.3 mmol, prepared according to example 16) and palladium-on-charcoal catalyst 5% (1.35 g) in acetic acid (150 ml) was hydrogenated at normal pressure and at an internal temperature of 40°C. After an in process HPLC analysis showed complete deprotection, the suspension was filtered over Celite. The filtrate was cooled to 20°C and concentrated aqueous hydrochloric acid was added (4.59 g, 46.5 mmol). After filtration, the wet product was washed first with acetic acid (10 ml) then with ethanol (20 ml) and dried in vacuo to give a white solid (yield: 18.05 g, HPLC-purity: 99.7 %).

### Example 19:

### Preparation of (±)-2-{Benzyl-[2-(6-fluoro-(2R*)-chroman-2-yl)-(2S*)-hydroxy-ethyl]-amino}-1-(6-fluoro-(2S*)-chroman-2-yl)-ethanone (compound IXa) ⇔ (±)-4-Benzyl-2-[6-fluoro-(2R*)-chroman-2-yl]-(6S*)-[6-fluoro-(2S*)-chroman-2-yl]-morpholin-2-ol (compound IXa')

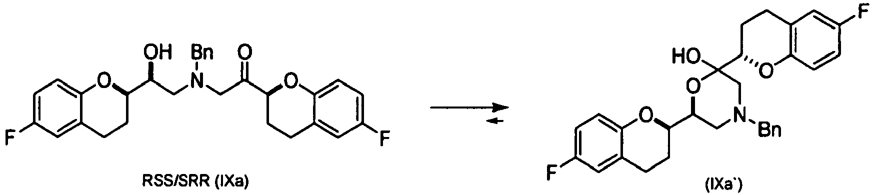

A 1.01 double jacket glass reactor was charged with a mixture of compounds **IXa/IXb** (100 g, 202.6 mmol, ratio **IXa/IXb** = 50.8/49.2) and acetonitrile (311 g, water 0.01% KF). The colourless slurry was heated to an internal temperature (IT) = 70°C and stirred at this temperature for 13 minutes. After slowly cooling the mixture to 60°C (30 minutes), DBU (7.79 g, 50.7 mmol) was added and the reaction mixture was cooled with the following gradient:

| Time [h] | IT [°C] |
|---|---|
| 0 | 60 |
| 0.5 | 55 |
| 1.5 | 50 |

Subsequently, the slurry was stirred 1 h at 50°C and then the ratio of **IXa/IXb** was controlled by HPLC (result: **IXa/IXb** = 66.3/33.7). Stirring was continued at 50°C until the ratio of **IXa/IXb** ≥75/25 (monitored by HPLC). In this case, the slurry was stirred for 4 h at 50°C (ratio of **IXa/IXb** = 75.1/24.9. The mixture was then cooled to 45°C and stirred at this temperature until the ratio of **IXa/IXb** >84/16 (monitored by HPLC). The slurry was stirred for 2 h at 45°C (ratio of **IXa/IXb** = 85/15). Afterwards, the mixture was cooled to 40°C and stirred at this temperature until the ratio of **IXa/IXb** ≥89/11 (monitored by HPLC) but not longer than 8h (in such case the reaction should be worked up in the same manner as following described). In this case, the slurry was stirred for 5 h at 40°C (ratio of **IXa/IXb** = 89.2/10.8). The reaction mixture was neutralized with acetic acid (3.051 g, 50.7 mmol) and then cooled to 25°C. After stirring for 2 h at 25°C, the suspension was filtered off and the filter cake washed four times with acetonitrile (each 31 g) (ratio of **IXa/IXb** in the filter cake after washing = 99.06/0.94). The amber mother liquor was further processed (see below, recovery, ML1). The wet product was suspended in acetonitrile (249 g) and the slurry wa heated to 70°C. After 1-10 minutes of slurrying at this temperature, the mixture was cooled to 20°C (within 2 h 45 min) and stirred at this temperature for 1.5 h. After filtration, the wet product was washed twice with acetonitrile (each 31 g) and dried in vacuo at 50°C to give a colourless solid (yield: 74.09 g, 73.4%, HPLC-purity 99.33%, ratio of **IXa/IXb** = 99.37/0.63, assay = 99.1%). The mother liquor from the slurry (ML2) was used for the recovery step (see below).

### Example 20:

### Recovery of starting material from the mother liquors obtained from Example 19

Water (117 g) was added to the mother liquor (ML1, 350.02 g) at IT = 24°C, and the resulting mixture was seeded with **IXa/IXb** (0.05 g). The crystallization started a few minutes later. After stirring 1.5 h at 20°C - 25°C, the beige slurry was cooled to 0°C - 5°C and stirred at this temperature for 3.5 h. The slurry was filtered off and the wet product was washed with a mixture of acetonitrile (31 g) and water (10 g). The amber mother liquor was disposed and the wet product was suspended at 20°C - 25°C in the second mother liquor (ML2, 279 g, see above). Then water was added (93 g) and the mixture was stirred at 0°C -5°C for 1.5 h. After filtration of the slurry, the wet product was washed with a mixture of acetonitrile (31 g) and water (10 g) and dried in vacuo at 50°C to give an off-white solid (yield: 7.74 g, 7.6%, HPLC-purity 99.49% (sum of both diastereomers), ratio of **IXa/IXb** = 39.1/60.9, assay = 98.2% (sum of both diastereomers)).

## Claims

1. A process for preparing racemic [25* [R* [R* [R*]]]] and [2R* [S* [S* [S*]]]]-(±)α,α'-[iminobis(methylene)]bis[6-fluoro-3,4-dihydro-2H-1-benzopyran-2-methanol] and pharmaceutically acceptable salts thereof, the process comprising:
(a) providing a compound of formula (IX) 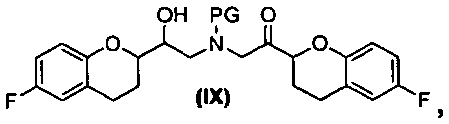 a compound of formula (IX') which is a cyclic semi-ketal form of the compound of formula (IX) 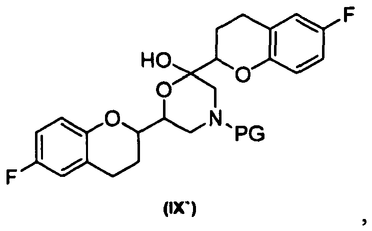 or a mixture thereof, wherein the compound of formula (IX) and the compound of formula (IX') are mixtures of diastereomers, wherein PG is hydrogen or an amine protecting group, wherein the amine protecting group is at least one of an allyl group or an aryl-C₁ alkyl group;
(b) separating diastereomers of the compound of formula (IX) or the compound of formula (IX') to obtain substantially pure diastereomers of formula (IX) or formula (IX') having at least 50% of a RSS/SRR or RRS/SSR configuration in a simultaneous epimerization-crystallization step, wherein the epimerization-crystallization step comprises:
(1) epimerizing an RSR/SRS configuration of the compound of formula (IX) or (IX') to give a mixture of the RSS/SRR configuration and the RSR/SRS configuration of diastereomers of formula (IX) or formula (IX') or
epimerizing an RRR/SSS configuration of the compound of formula (IX) or (IX') to give a mixture of the RRS/SSR configuration and the RRR/SSS configuration of diastereomers of formula (IX) or formula (IX'), provided that said epimerizing is conducted in a presence of a base and an organic solvent wherein the mixture is optionally cooled using a temperature gradient and wherein the RSS/SRR configuration or the RRS/SSR configuration in the mixture are obtained in at least two fold excess relative to the RSR/SRS configuration and the RRR/SSS configuration; and
(2) crystallizing substantially pure diastereomers of formula (IX) or formula (IX') having the RSS/SRR configuration or the RRS/SSR configuration in at least two fold excess relative to the RSR/SRS configuration and the RRR/SSS configuration;
separating the mixture by fractional crystallization optionally after salt formation or after derivatization to obtain substantially pure diastereomers of formula (IX) or formula (IX') having the RSS/SRR or RRS/SSR configuration;
(c) reducing substantially pure diastereomers of formula (IX) or formula (IX') having a RSS/SRR or RRS/SSR configuration to give a compound of formula (X) 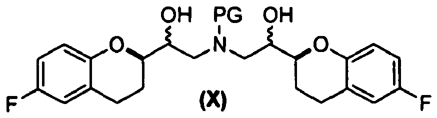 as a RSSS/SRRR diastereomeric mixture having a ratio of a RSSS/SRRR diastereomeric configuration to a SRSR or RRSS diastereomeric configuration, wherein said ratio is at least 1;
(d) deprotecting the compound of formula (X), provided that PG is not H and if PG is H then omitting said deprotection, to obtain a compound of formula (I) 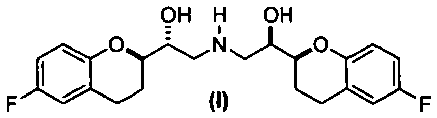 or pharmaceutically acceptable salts thereof; and
(e) removing a RSRS or RRSS diastereomeric configuration of the compound of formula (I) or pharmaceutically acceptable salts thereof if present by recrystallization or by a slurry to give racemic [2S*[R*[R*[R*]]]] and [2R*[S*[S*[S*]]]]-(±)α,α'-[iminobis(methylene)]bis[6-fluoro-3,4-dihydro-2H-1-benzopyran-2-methanol] or pharmaceutically acceptable salts thereof.

2. The process according to claim 1, wherein step (b) is carried out for the RSR/SRS configuration of the compound of formula (IX) or (IX').

3. The process according to claim 2, wherein the RSS/SRR configuration in the mixture is obtained in at about nine fold excess of the RSR/SRS.

4. The process according to claim 1, wherein in step (b) the mixture is cooled using the temperature gradient from about 70°C to about 20°C.

5. The process according to claim 4, wherein the temperature gradient from 70°C to 40°C.

6. The process according to claim 1, wherein providing the compound of formula (IX) 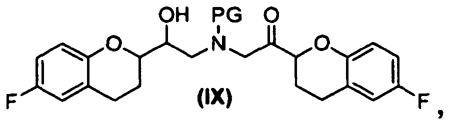 the compound of formula (IX') which is a cyclic semi-ketal form of the compound of formula (IX) 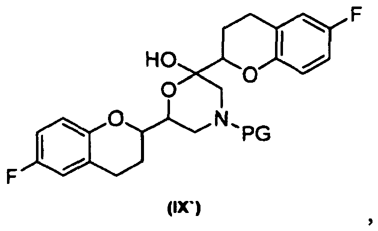 or the mixture thereof, wherein the compound of formula (IX) and the compound of formula (IX') are mixtures of diastereomers in step (a) comprises:
(i) providing a compound of formula (VIII) 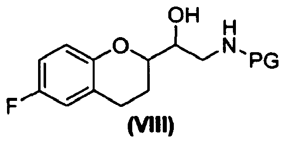 as a diastereomerically pure compound comprising at least 95% of RS/SR configuration or RR/SS configuration, wherein PG is hydrogen or an amine protecting group, wherein the amine protecting group is at least one of an allyl group or an aryl-C₁ alkyl group;
(ii) providing a racemic compound of formula (V) 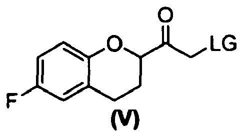 wherein LG is a member selected from the group consisting of chloro, bromo, iodo, alkylsulfonyloxy and arylsufonyloxy;
(iii) N-alkylating the compound of formula (VIII) with the compound of formula (V), wherein said N-alkylating is carried out in an inert organic solvent in a presence of a base and optionally in the presence of a catalyst.

7. The process according to claim 6, wherein the protecting group is a benzyl group.

8. The process according to claim 6, wherein the leaving group is chloro or bromo.

9. The process according to claim 6, wherein in step (iii) the organic solvent is a polar aprotic solvent selected from the group consisting of DMF, DMA and NMP.

10. The process according to claim 6, wherein in step (iii), the base is at least one of tertiary amines, alkali metal carbonate or alkali metal hydrogen carbonate.

11. The process according to claim 6, wherein the base is sodium hydrogen carbonate.

12. The process according to claim 11, wherein about 1.5 to about 2.5 equivalents of the base are used.

13. The process according to claim 6, wherein in step (iii) the catalyst is at least one of alkali metal bromides, alkali metal iodides, tetraalkylammonium bromides or tetraalkylammonium iodides.

14. The process according to claim 13, wherein the catalyst is sodium bromide.

15. The process according to claim 14, wherein about 0.05 to about 0.25 equivalents of the catalyst are used.

16. The process according to claim 15, wherein 0.15 equivalents of the catalysts are used.

17. The process according to claim 6, wherein in step (iii) said N-alkylation is carried out at a temperature between about room temperature and about 80°C.

18. The process according to claim 6, wherein in step (ii) the compound of formula (V) is provided in an amount of about 1.0 to about 1.5 equivalents.

19. The process according to claim 6, wherein step (b) is carried out for the RSR/SRS configuration of the compound of formula (IX) or (IX').

20. The process according to claim 19, wherein the RSS/SRR configuration in the mixture is obtained in at about nine fold excess of the RSR/SRS.

21. The process according to claim 6, wherein in step (b) the mixture is cooled using the temperature gradient from about 70°C to about 20°C.

22. The process according to claim 21, wherein the temperature gradient from 70°C to 40°C.

23. The process according to claim 6, wherein in step (b), the organic solvent is acetonitrile.

24. The process according to claim 6, wherein in step (b), said epimerizing is carried out in the presence of at least 0.1 equivalents of the base.

25. The process according to claim 6, wherein said epimerizing is carried out in the presence of at least 0.25 equivalents of the base.

26. The process according to claim 6, wherein in step (b), the base is a member selected from the group consisting of an alkoxide, an amidine, a guanidine and a phosphazene.

27. The process according to claim 26, wherein the base is an amidine.

28. The process according to claim 27, wherein the base is diazabicycloundecene.

29. The process according to claim 6, wherein in step (b) water if present cannot exceed 1.0%.

30. The process according to claim 6, wherein in step (b) water if present cannot exceed 0.1 %.

31. The process according to claim 6, wherein in step (c) said reducing is carried out for the RSS/SRR configuration the compound of formula (IX) or the compound of formula in a solvent with alkali borohydride, tetrabutylammonium borohydride, alkali tri(sec-butyl) borohydride or zinc borohydride, optionally in a presence of a Lewis acid.

32. The process according to claim 31, wherein the Lewis acid is at least one of Ti(OAlkyl)₄, ZnCl₂ alkali halide or alkaline earth halide.

33. The process according to claim 32, wherein the solvent is at least one of an ether, an alcohol or a halogenated hydrocarbon.

34. The process according to claim 33, wherein said reducing is carried out at temperatures between about -20°C and about room temperature.

35. The process according to claim 6, wherein in step (d) said deprotecting is carried out by catalytic hydrogenation.

36. The process according to claim 6, wherein in step (e), said purifying the compound of formula (I) is done by a slurry of its hydrochloride salt in a solvent.

37. The process according to claim 36, wherein the slurry is carried out in methanol as the solvent.

## Patentansprüche

1. Verfahren zur Herstellung von racemischem [2S*[R*[R*[R*]]]]- und [2R*[S*[S*[S*]]]]-(±)α,α'-[Iminobis(methylen)]bis[6-fluor-3,4-dihydro-2H-1-benzopyran-2-methanol] und pharmazeutisch annehmbarer Salze davon, wobei das Verfahren umfasst:
(a) Bereitstellen einer Verbindung der Formel (IX) 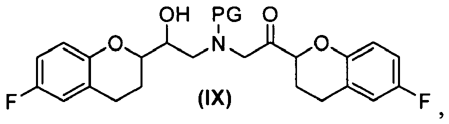 einer Verbindung der Formel (IX'), welche eine cyclische Semiketal-Form der Verbindung der Formel (IX) ist 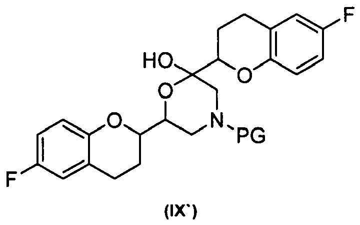 oder einer Mischung davon, wobei die Verbindung der Formel (IX) und die Verbindung der Formel (IX') Mischungen von Diastereomeren sind, wobei PG Wasserstoff oder eine amin-schützende Gruppe ist, und wobei die amin-schützende Gruppe mindestens eine von einer Allylgruppe oder einer Aryl-C₁-Alkylgruppe ist;
(b) Trennen von Diastereomeren der Verbindung der Formel (IX) oder der Verbindung der Formel (IX'), um im Wesentlichen reine Diastereomere der Formel (IX) oder der Formel (IX') mit mindestens 50% einer RSS/SRR- oder RRS/SSR-Konfiguration in einem gleichzeitigen Epimerisierungs-Kristallisations-Schritt erhalten, wobei der Epimerisierungs-Kristallisations-Schritt umfasst:
(1) Epimerisieren einer RSR/SRS-Konfiguration der Verbindung der Formel (IX) oder (IX'), um eine Mischung der RSS/SRR-Konfiguration und der RSR/SRS-Konfiguration von Diastereomeren der Formel (IX) oder der Formel (IX') zu ergeben, oder Eperimerisieren einer RRR/SSS-Konfiguration der Verbindung der Formel (IX) oder (IX'), um eine Mischung der RRS/SSR-Konfiguration und der RRR/SSS-Konfiguration von Diastereomeren der Formel (IX) oder der Formel (IX') zu ergeben, mit der Maßgabe, dass das Epimerisieren in Gegenwart einer Base und eines organischen Lösungsmittels durchgeführt wird, wobei die Mischung gegebenenfalls unter Einsatz eines Temperaturgradienten gekühlt wird, und wobei die RSS/SRR-Konfiguration oder die RRS/SSR-Konfiguration in der Mischung in mindestens zweifachem Überschuss, bezogen auf die RSR/SRS-Konfiguration und die RRR/SSS-Konfiguration, erhalten werden; und
(2) Kristallisieren von im Wesentlichen reinen Diastereomeren der Formel (IX) oder der Formel (IX') mit der RSS/SRR-Konfiguration oder der RRS/SSR-Konfiguration in mindestens zweifachem Überschuss, bezogen auf die RSR/SRS-Konfiguration und die RRR/SSS-Konfiguration;
Auftrennen der Mischung durch fraktionierte Kristallisation, gegebenenfalls nach Salzbildung oder nach Derivatisierung, um im Wesentlichen reine Diastereomere der Formel (IX) oder der Formel (IX') mit der RSS/SRR- oder der RRS/SSR-Konfiguration zu erhalten;
(c) Reduzieren von im Wesentlichen reinen Diastereomeren der Formel (IX) oder der Formel (IX') mit einer RSS/SRR- oder RRS/SSR-Konfiguration, um eine Verbindung der Formel (X) 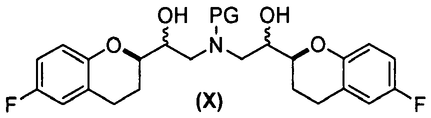 als eine RSSS/SRRR-Diastereomeren-Mischung mit einem Verhältnis einer RSSS/SRRR-Diastereomeren-Konfiguration zu einer SRSR/RRSS-Diastereomeren-Konfiguration, wobei das Verhältnis mindestens 1 beträgt, zu ergeben;
(d) Befreien der Verbindung der Formel (X) von der Schutzgruppe, falls PG nicht H ist, und Unterlassen des Befreiens von der Schutzgruppe, falls PG H ist, um eine Verbindung der Formel (I) 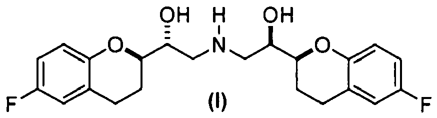 oder pharmazeutisch annehmbare Salze davon zu erhalten; und
(e) Entfernen einer RSRS- oder RRSS-Diastereomeren-Konfiguration der Verbindung der Formel (I) oder pharmazeutisch annehmbarer Salze davon, falls vorhanden, durch Umkristallisation oder durch eine Aufschlämmung, um racemisches [2S*[R*[R*[R*]]]]- und [2R*[S*[S*[S*]]]]-(+)α,α'-[Iminobis(methylen)]bis[6-fluor-3,4-dihydro-2H-1-benzopyran-2-methanol] oder pharmazeutisch annehmbare Salze davon zu erhalten.

2. Verfahren nach Anspruch 1, wobei Schritt (b) für die RSR/SRS-Konfiguration der Verbindung der Formel (IX) oder (IX') durchgeführt wird.

3. Verfahren nach Anspruch 2, wobei die RSS/SRR-Konfiguration in der Mischung in einem etwa neunfachen Überschuss der RSR/SRS-Konfiguration erhalten wird.

4. Verfahren nach Anspruch 1, wobei in Schritt (b) die Mischung unter Einsatz eines Temperaturgradienten von etwa 70°C bis etwa 20°C gekühlt wird.

5. Verfahren nach Anspruch 4, wobei der Temperaturgradient von 70°C bis 40°C reicht.

6. Verfahren nach Anspruch 1, wobei das Bereitstellen der Verbindung der Formel (IX) 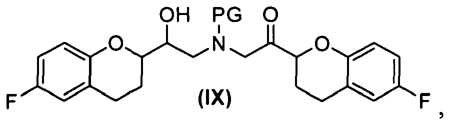 der Verbindung der Formel (IX'), welche eine cyclische Semi-ketal-Form der Verbindung der Formel (IX) ist, 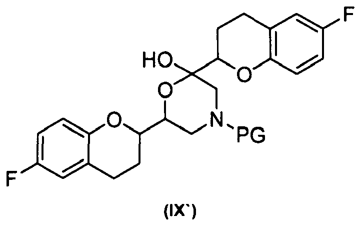 oder der Mischung davon, wobei die Verbindung der Formel (IX) und die Verbindung der Formel (IX') Mischungen von Diastereomeren sind, in Schritt (a) umfasst:
(i) Bereitstellen einer Verbindung der Formel (VIII) 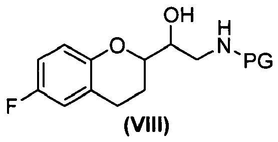 als eine diastereomerreine Verbindung, die mindestens 95% der RS/SR-Konfiguration oder der RR/SS-Konfiguration umfasst, wobei PG Wasserstoff oder eine amin-schützende Gruppe ist, wobei die amin-schützende Gruppe mindestens eine von einer Allylgruppe oder einer Aryl-C₁-Alkylgruppe ist;
(ii) Bereitstellen einer racemischen Verbindung der Formel (V) 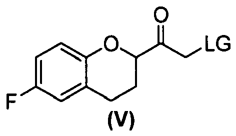 worin LG ein aus der Gruppe, welche aus Chlor, Brom, Iod, Alkylsulfonyloxy und Arylsulfonyloxy besteht, ausgewähltes Element ist;
(iii) N-Alkylieren der Verbindung der Formel (VIII) mit der Verbindung der Formel (V), wobei die N-Alkylierung in einem inerten organischen Lösungsmittel in Gegenwart einer Base und optional in Gegenwart eines Katalysators durchgeführt wird.

7. Verfahren nach Anspruch 6, wobei die Schutzgruppe eine Benzylgruppe ist.

8. Verfahren nach Anspruch 6, wobei die Austrittsgruppe Chlor oder Brom ist.

9. Verfahren nach Anspruch 6, wobei in Schritt (iii) das organische Lösungsmittel ein polares aprotisches Lösungsmittel ist, ausgewählt aus der Gruppe, die aus DMF, DMA und NMP besteht.

10. Verfahren nach Anspruch 6, wobei in Schritt (iii) die Base mindestens eines von tertiären Aminen, Alkalimetallcarbonat oder Alkalimetallhydrogencarbonat ist.

11. Verfahren nach Anspruch 6, wobei die Base Natriumhydrogencarbonat ist.

12. Verfahren nach Anspruch 11, wobei etwa 1,5 bis etwa 2,5 Äquivalente der Base verwendet werden.

13. Verfahren nach Anspruch 6, wobei in Schritt (iii) der Katalysator mindestens eines von Alkalimetallbromiden, Alkalimetalliodiden, Tetraalkylammoniumbromiden oder Tetraalkylammoniumiodiden ist.

14. Verfahren nach Anspruch 13, wobei der Katalysator Natriumbromid ist.

15. Verfahren nach Anspruch 14, wobei etwa 0,05 bis etwa 0,25 Äquivalente des Katalysators verwendet werden.

16. Verfahren nach Anspruch 15, wobei 0,15 Äquivalente des Katalysators verwendet werden.

17. Verfahren nach Anspruch 6, wobei in Schritt (iii) die N-Alkylierung bei einer Temperatur zwischen etwa Raumtemperatur und etwa 80°C durchgeführt wird.

18. Verfahren nach Anspruch 6, wobei in Schritt (ii) die Verbindung der Formel (V) in einer Menge von etwa 1,0 bis etwa 1,5 Äquivalenten bereitgestellt wird.

19. Verfahren nach Anspruch 6, wobei Schritt (b) für die RSR/SRS-Konfiguration der Verbindung der Formel (IX) oder (IX') durchgeführt wird.

20. Verfahren nach Anspruch 19, wobei die RSS/SRR-Konfiguration in der Mischung in einem etwa neunfachen Überschuss der RSR/SRS-Konfiguration erhalten wird.

21. Verfahren nach Anspruch 6, wobei in Schritt (b) die Mischung unter Einsatz eines Temperaturgradienten von etwa 70°C bis etwa 20°C gekühlt wird.

22. Verfahren nach Anspruch 21, wobei der Temperaturgradient von 70°C bis 40°C reicht.

23. Verfahren nach Anspruch 6, wobei in Schritt (b) das organische Lösungsmittel Acetonitril ist.

24. Verfahren nach Anspruch 6, wobei in Schritt (b) das Epimerisieren in Gegenwart von mindestens 0,1 Äquivalenten der Base durchgeführt wird.

25. Verfahren nach Anspruch 6, wobei das Epimerisieren in Gegenwart von mindestens 0,25 Äquivalenten der Base durchgeführt wird.

26. Verfahren nach Anspruch 6, wobei in Schritt (b) die Base ein aus der Gruppe, welche aus einem Alkoxid, einem Amidin, einem Guanidin und einem Phosphazen besteht, ausgewähltes Element ist.

27. Verfahren nach Anspruch 26, wobei die Base ein Amidin ist.

28. Verfahren nach Anspruch 27, wobei die Base Diazabicycloundecen ist.

29. Verfahren nach Anspruch 6, wobei in Schritt (b) Wasser, falls vorhanden, nicht 1,0 % überschreiten kann.

30. Verfahren nach Anspruch 6, wobei in Schritt (b) Wasser, falls vorhanden, nicht 0,1 % überschreiten kann.

31. Verfahren nach Anspruch 6, wobei in Schritt (c) das Reduzieren für die RSS/SRR-Konfiguration der Verbindung der Formel (IX) oder der Verbindung der Formel (IX') in einem Lösungsmittel mit Alkaliborhydrid, Tetrabutylammoniumborhydrid, Alkalitri(sec-butyl)borhydrid oder Zinkborhydrid, gegebenenfalls in Gegenwart einer Lewissäure, durchgeführt wird.

32. Verfahren nach Anspruch 31, wobei die Lewissäure mindestens eines von Ti(OAlkyl)₄, ZnCl₂, Alkalihalogenid oder Erdalkalihalogenid ist.

33. Verfahren nach Anspruch 32, wobei das Lösungsmittel mindestens eines von einem Ether, einem Alkohol oder einem halogenierten Kohlenwasserstoff ist.

34. Verfahren nach Anspruch 33, wobei das Reduzieren bei Temperaturen zwischen etwa -20°C und etwa Raumtemperatur durchgeführt wird.

35. Verfahren nach Anspruch 6, wobei in Schritt (d) die Befreiung von der Schutzgruppe durch katalytische Hydrierung durchgeführt wird.

36. Verfahren nach Anspruch 6, wobei im Schritt (e) das Reinigen der Verbindung der Formel (I) durch eine Aufschlämmung ihres Hydrochlorid-Salzes in einem Lösungsmittel erfolgt.

37. Verfahren nach Anspruch 36, wobei die Aufschlämmung in Methanol als Lösungsmittel erfolgt.

## Revendications

1. Procédé de préparation du [2S*[R*[R*[R*]]]] et du [2R*[S*[S*[S*]]]]-(±)α,α'-[iminobis(méthylène)]bis[6-fluoro-3,4-dihydro-2H-1-benzopyrane-2-méthanol] racémiques et de leurs sels pharmaceutiquement acceptables, ledit procédé comprenant les étapes consistant à :
(a) fournir un composé de formule (IX) 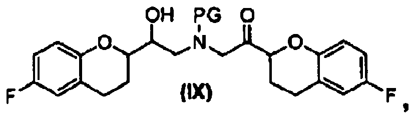 un composé de formule (IX') qui est une forme hémi-cétale cyclique du composé de formule (IX) 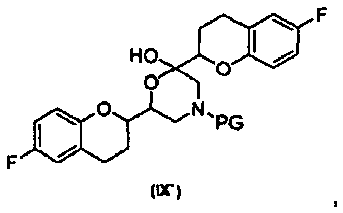 ou un mélange de ceux-ci, dans lequel le composé de formule (IX) et le composé de formule (IX') sont des mélanges de diastéréomères, dans lequel PG est un atome d'hydrogène ou un groupe amine protecteur, dans lequel le groupe amine protecteur est au moins un élément parmi un groupe allyle ou un groupe aryl-alkyle en C₁ ;
(b) séparer les diastéréomères du composé de formule (IX) ou du composé de formule (IX') pour obtenir des diastéréomères essentiellement purs de formule (IX) ou de formule (IX') ayant au moins 50 % d'une configuration RSS/SRR ou RRS/SSR dans une étape d'épimérisation - cristallisation simultanée, où l'étape d'épimérisation - cristallisation consiste à :
(1) épimériser une configuration RSR/SRS du composé de formule (IX) ou (IX') pour donner un mélange de configuration RSS/SRR et de configuration RSR/SRS de diastéréomères de formule (IX) ou de formule (IX') ou
épimériser une configuration RRR/SSS du composé de formule (IX) ou (IX') pour donner un mélange de configuration RRS/SSR et de configuration RRR/SSS de diastéréomères de formule (IX) ou de formule (IX') à la condition que ladite épimérisation soit réalisée en présence d'une base et d'un solvant organique où le mélange est éventuellement refroidi en utilisant un gradient de température et où la configuration RSS/SRR ou la configuration RRS/SSR dans le mélange sont obtenues en un excédent d'au moins deux fois par rapport à la configuration RSR/SRS et la configuration RRR/SSS ; et
(2) cristalliser les diastéréomères essentiellement purs de formule (IX) ou de formule (IX') ayant la configuration RSS/SRR ou la configuration RRS/SSR en un excédent d'au moins deux fois par rapport à la configuration RSR/SRS et la configuration RRR/SSS ;
séparer le mélange par cristallisation fractionnaire éventuellement après la formation d'un sel ou après dérivatisation pour obtenir des diastéréomères essentiellement purs de formule (IX) ou de formule (IX') ayant la configuration RSS/SRR ou RRS/SSR ;
(c) réduire les diastéréomères essentiellement purs de formule (IX) ou de formule (IX') ayant la configuration RSS/SRR ou RRS/SSR pour donner un composé de formule (X) 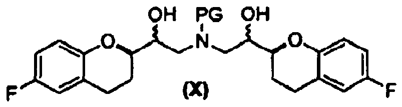 sous la forme d'un mélange diastéréomérique RSSS/SRRR ayant un rapport entre une configuration diastéréomérique RSSS/SRRR et une configuration diastéréomérique SRSR ou RRSS, qui est d'au moins 1 ;
(d) déprotéger le composé de formule (X) à la condition que PG ne soit pas H et si PG est H alors omettre ladite déprotection pour obtenir un composé de formule (I) 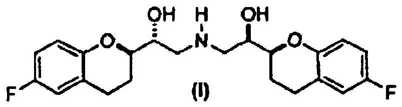 ou un sel pharmaceutiquement acceptable de celui-ci ; et
(e) enlever une configuration diastéréomérique RSRS ou RRSS du composé de formule (I) ou de ses sels pharmaceutiquement acceptables, si elle est présente, par recristallisation ou par une suspension pour donner les [2S*[R*[R*[R*]]]] et [2R*[S*[S*[S*]]]]-(±),α'α-[iminobis(méthylène)]bis[6-fluoro-3,4-dihydro-2H-1-benzopyrane-2-méthanol] racémiques ou leurs sels pharmaceutiquement acceptables.

2. Procédé selon la revendication 1, dans lequel l'étape (b) est réalisée pour la configuration RSR/SRS du composé de formule (IX) ou (IX').

3. Procédé selon la revendication 2, dans lequel la configuration RSS/SRR dans le mélange est obtenue en un excédent d'environ neuf fois par rapport à RSR/SRS.

4. Procédé selon la revendication 1, dans lequel, à l'étape (b), le mélange est refroidi en utilisant le gradient de température allant d'environ 70 °C à environ 20 °C.

5. Procédé selon la revendication 4, dans lequel le gradient de température va de 70 °C à 40 °C.

6. Procédé selon la revendication 1, dans lequel l'étape consistant à fournir le composé de formule (IX) 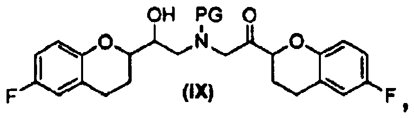 le composé de formule (IX') qui est une forme hémi-cétale cyclique du composé de formule (IX) 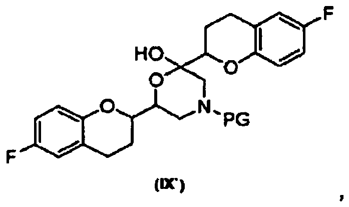 ou un mélange de ceux-ci, dans lequel le composé de formule (IX) et le composé de formule (IX') sont des mélanges de diastéréomères, à l'étape (a), consiste à :
(i) fournir un composé de formule (VIII) 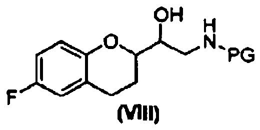 sous la forme d'un composé diastéréomériquement pur comprenant au moins 95 % de configuration RS/SR ou de configuration RR/SS, dans lequel PG est un atome d'hydrogène ou un groupe amine protecteur, dans lequel le groupe amine protecteur est au moins un élément parmi un groupe allyle ou un groupe aryl-alkyle en C₁ ;
(ii) fournir un composé racémique de formule (V) 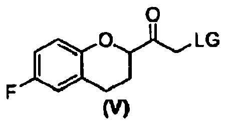 dans lequel LG est un élément choisi dans le groupe constitué par le chloro, le bromo, l'iodo, l'alkylsulfonyloxy et l'arylsulfonyloxy ;
(iii) N-alkyler le composé de formule (VIII) avec le composé de formule (V), dans lequel ladite N-alkylation est réalisée dans un solvant organique inerte en présence d'une base et éventuellement en présence d'un catalyseur.

7. Procédé selon la revendication 6, dans lequel le groupe protecteur est un groupe benzyle.

8. Procédé selon la revendication 6, dans lequel le groupe sortant est le chloro ou le bromo.

9. Procédé selon la revendication 6, dans lequel, à l'étape (iii), le solvant organique est un solvant aprotique polaire choisi dans le groupe constitué par le DMF, le DMA et la NMP.

10. Procédé selon la revendication 6, dans lequel, à l'étape (iii), la base est au moins un élément parmi les amines tertiaires, le carbonate de métaux alcalins ou l'hydrogénocarbonate de métaux alcalins.

11. Procédé selon la revendication 6, dans lequel la base est l'hydrogénocarbonate de sodium.

12. Procédé selon la revendication 11, dans lequel environ 1,5 à environ 2,5 équivalents de la base sont utilisés.

13. Procédé selon la revendication 6, dans lequel, à l'étape (iii), le catalyseur est au moins un élément parmi les bromures de métaux alcalins, les iodures de métaux alcalins, les bromures de tétraalkylammonium ou les iodures de tétraalkylammonium.

14. Procédé selon la revendication 13, dans lequel le catalyseur est le bromure de sodium.

15. Procédé selon la revendication 14, dans lequel environ 0,05 à environ 0,25 équivalent du catalyseur est utilisé.

16. Procédé selon la revendication 15, dans lequel 0,15 équivalent de catalyseurs est utilisé.

17. Procédé selon la revendication 6, dans lequel, à l'étape (iii), ladite N-alkylation est réalisée à une température située entre environ la température ambiante et environ 80 °C.

18. Procédé selon la revendication 6, dans lequel, à l'étape (ii), le composé de formule (V) est fourni en une quantité d'environ 1,0 à environ 1,5 équivalents.

19. Procédé selon la revendication 6, dans lequel l'étape (b) est réalisée pour la configuration RSR/SRS du composé de formule (IX) ou (IX').

20. Procédé selon la revendication 19, dans lequel la configuration RSS/SRR dans le mélange est obtenue en un excédent d'environ neuf fois de RSR/SRS.

21. Procédé selon la revendication 6, dans lequel, à l'étape (b), le mélange est refroidi en utilisant le gradient de température d'environ 70 °C à environ 20 °C.

22. Procédé selon la revendication 21, dans lequel le gradient de température va de 70 °C à 40 °C.

23. Procédé selon la revendication 6, dans lequel, à l'étape (b), le solvant organique est l'acétonitrile.

24. Procédé selon la revendication 6, dans lequel, à l'étape (b), ladite épimérisation est réalisée en présence d'au moins 0,1 équivalent de la base.

25. Procédé selon la revendication 6, dans lequel ladite épimérisation est réalisée en présence d'au moins 0,25 équivalent de la base.

26. Procédé selon la revendication 6, dans lequel, à l'étape (b), la base est un élément choisi dans le groupe constitué par un alcoxyde, une amidine, une guanidine et un phosphazène.

27. Procédé selon la revendication 26, dans lequel la base est une amidine.

28. Procédé selon la revendication 27, dans lequel la base est le diazabicycloundécène.

29. Procédé selon la revendication 6, dans lequel, à l'étape (b), l'eau, si elle est présente, ne peut excéder 1,0 %.

30. Procédé selon la revendication 6, dans lequel, à l'étape (b), l'eau, si elle est présente, ne peut excéder 0,1 %.

31. Procédé selon la revendication 6, dans lequel, à l'étape (c), ladite réduction est réalisée pour la configuration RSS/SRR du composé de formule (IX) ou du composé de formule dans un solvant avec du borohydrure alcalin, du borohydrure de tétrabutylammonium, du borohydrure de tri(sec-butyle) alcalin ou du borohydrure de zinc, éventuellement en présence d'un acide de Lewis.

32. Procédé selon la revendication 31, dans lequel l'acide de Lewis est au moins un élément parmi le Ti(Oalkyl)₄, le ZnCl₂, l'halogénure d'alcalin ou l'halogénure d'alcalino-terreux.

33. Procédé selon la revendication 32, dans lequel le solvant est au moins un élément parmi un éther, un alcool ou un hydrocarbure halogéné.

34. Procédé selon la revendication 33, dans lequel ladite réduction est réalisée à des températures situées entre environ -20 °C et environ la température ambiante.

35. Procédé selon la revendication 6, dans lequel, à l'étape (d), ladite déprotection est réalisée par hydrogénation catalytique.

36. Procédé selon la revendication 6, dans lequel, à l'étape (e), ladite purification du composé de formule (I) est effectuée par une suspension de son sel de chlorhydrate dans un solvant.

37. Procédé selon la revendication 36, dans lequel la suspension est réalisée dans du méthanol comme solvant.
